# EUROPEAN PATENT SPECIFICATION

(11) **EP 1 268 480 B1**
(45) Date of publication and mention of the grant of the patent: **05.11.2003**
(21) Application number: 01940294.0
(22) Date of filing: 05.04.2001
(51) Int. Cl.: C07D 473/06, C07D 239/54, A61K 31/52, A61P 15/00

(54) **8-QUINOLINXANTHINE AND 8-ISOQUINOLINXANTHINE DERIVATIVES AS PDE 5 INHIBITORS**
8-CHINOLINXANTHIN AND 8-ISOCHINOLINXANTHIN DERIVATE ALS PDE 5 INHIBITOREN
DERIVES 8-QUINOLINXANTHINE ET 8-ISOQUINOLINXANTHINE COMME INHIBITEURS PDE 5

(30) Priority: 07.04.2000 GB 0008694
(43) Date of publication of application: 02.01.2003
(73) Proprietor: Novartis AG, 4002 Basel (CH); Novartis Pharma GmbH, 1230 Wien (AT)
(72) Inventor: BHALAY, Gurdip, Horsham, West Sussex RH13 7XG (GB); COLLINGWOOD, Stephen Paul, Crawley, West Sussex RH70 7UN (GB); FAIRHURST, Robin Alec, Ashington, West Sussex RH10 7UN (GB); GOMEZ, Sylvie, Felicite, Redcliff, Surrey RH1 2HR (GB); NAEF, Reto, CH-4310 Rheinfelden (CH); SANDHAM, David, Andrew, Horsham, West Sussex RH12 4BP (GB)
(74) Representative: Gros, Florent
(86) International application number: EP0103909
(87) International publication number: WO01077110

(56) References cited:
- WO-A-00/37061
- WO-A-94/28902
- WO-A-99/62905
- US-A- 4 599 338

## Description

This invention relates to organic compounds, their preparation and their use as pharmaceuticals.

In one aspect, the invention provides compounds of formula 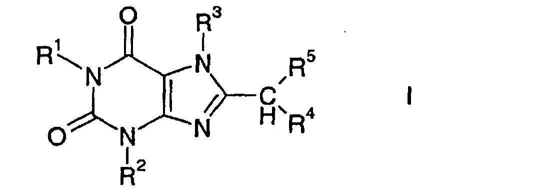 in free or salt form, where
R¹ is hydrogen or alkyl optionally substituted by hydroxy, alkoxy, or alkylthio,
R² is hydrogen, alkyl, hydroxyalkyl, alkylcarbonyloxyalkyl, alkoxyalkyl, alkylthioalkyl, alkenyl, cycloalkylalkyl, heterocyclylalkyl, aralkyl in which the aryl ring thereof is optionally fused to a 5-membered heterocyclic group or is optionally substituted by one or more substituents selected from alkoxy, amino, alkylamino, dialkylamino, acylamino, halogen, hydroxy, aminosulfonyl, alkylaminosulfonyl, dialkylaminosulfonyl, alkylsulfonylamino or dialkylaminosulfonylamino,
R³ is hydrogen or alkyl optionally substituted by hydroxy, alkoxy, or alkylthio,
R⁴ is hydrogen or alkyl,
R⁵ is a quinolinyl, isoquinolinyl or oxodihydroisoquinolinyl group optionally fused to a 5-membered heterocyclic group and optionally substituted by one or more substituents selected from halogen, cyano, hydroxy, alkyl, hydroxyalkyl, alkoxyalkyl, alkylthioalkyl, alkoxy, alkylthio, alkenyl, alkoxycarbonyl, alkynyl, carboxyl, acyl, a group of formula -N(R⁶)R⁷, aryl optionally substituted by one or more substituents selected from halogen or alkoxy, or heteroaryl having 5 or 6 ring atoms, attached through a ring carbon atom to the indicated carbon atom, and
R⁶ and R⁷ are each independently hydrogen or alkyl optionally substituted by hydroxy or alkoxy or one of R⁶ and R⁷ is hydrogen and the other is acyl, or R⁶ and R⁷ together with the nitrogen atom to which they are attached denote a 5- or 6- membered heterocyclyl group.

"Alkyl" as used herein denotes straight chain or branched alkyl, which may be, for example, C₁-C₁₀-alkyl such as methyl, ethyl, n-propyl, isopropyl, n-butyl, isobutyl, sec-butyl, tert-butyl, straight or branched pentyl, straight or branched hexyl, straight or branched heptyl, straight or branched octyl, straight or branched nonyl or straight or branched decyl. Preferably alkyl is C₁-C₈-alkyl.

"Alkoxy" as used herein denotes straight chain or branched alkoxy which may be, for example, C₁-C₁₀-alkoxy such as methoxy, ethoxy, n-propoxy, isopropoxy, n-butoxy, isobutoxy, sec-butoxy, tert-butoxy, straight or branched pentoxy, straight or branched hexyloxy, straight or branched heptyloxy, straight or branched octyloxy, straight or branched nonyloxy or straight or branched decyloxy. Preferably, alkoxy is C₁-C₄-alkoxy.

"Alkylthio" as used herein may be C₁ to C₁₀-alkylthio such as methylthio, ethylthio, n-propylthio, isopropylthio, n-butylthio, sec-butylthio, isobutylthio, tert-butylthio, pentylthio, hexylthio, heptylthio, octylthio, nonylthio or decylthio. Preferably alkylthio is C₁ to C₄-alkylthio.

"Alkenyl" as used herein means straight chain or branched alkenyl, which may be, for example, C₂ to C₁₀-alkenyl such as vinyl, 1-propenyl, 2-propenyl, 1-butenyl, isobutenyl, or straight or branched pentenyl, hexenyl, heptenyl, octenyl, nonenyl or decenyl. Preferred alkenyl is C₂ to C₄-alkenyl.

"Cycloalkylalkyl" as used herein denotes alkyl, for example C₁ to C₁₀-alkyl such as one of the C₁ to C₁₀-alkyl groups hereinbefore mentioned, substituted by a C₃ to C₈ cycloalkyl group such as cyclopropyl, methylcyclopropyl, cyclobutyl, cyclopentyl, cyclohexyl, methylcyclohexyl, dimethylcyclohexyl, cycloheptyl or cyclooctyl. Preferably, cycloalkylalkyl is C₃-C₆-cycloalkyl-C₁-C₄-alkyl.

"Heterocyclylalkyl" as used herein denotes alkyl, for example C₁ to C₁₀-alkyl such as one of the C₁ to C₁₀-alkyl groups hereinbefore mentioned, substituted by a 5- or 6- membered heterocyclyl group having one or two hetero atoms selected from nitrogen, oxygen and sulfur in the ring, such as pyrrolyl, pyrrolidinyl, furyl, thienyl, pyridyl, piperidyl, imidazolyl, imidazolidinyl, pyrazolidinyl, piperazinyl, morpholinyl, oxazolyl, or furazanyl. Preferably, heterocyclylalkyl is C₁-C₄-alkyl substituted by a 5- or 6- membered heterocyclyl group having one or two nitrogen or oxygen atoms or one nitrogen atom and one oxygen atom in the ring.

"Aralkyl" as used herein means C₆-C₁₀-aryl-C₁-C₁₀ alkyl and may be, for example, one of the C₁-C₁₀-alkyl groups mentioned hereinbefore, particularly one of the C₁-C₄-alkyl groups, substituted by phenyl, tolyl, xylyl or naphthyl. Preferably, aralkyl is phenyl-C₁-C₄-alkyl, particularly benzyl or 2-phenylethyl.

"Acyl" as used herein denotes alkylcarbonyl, for example C₁-C₁₀-alkylcarbonyl where C₁-C₁₀-alkyl may be one of the C₁-C₁₀-alkyl groups hereinbefore mentioned, optionally substituted by one or more halogen atoms; cycloalkylcarbonyl, for example C₃-C₈-cycloalkylcarbonyl where C₃-C₈-cycloalkyl may be, for example, cyclopropyl, cyclobutyl, cyclopentyl, cyclohexyl, cycloheptyl or cyclooctyl; 5- or 6- membered heterocyclylcarbonyl having one or two hetero atoms selected from nitrogen, oxygen and sulfur in the ring, such as furylcarbonyl or pyridylcarbonyl; arylcarbonyl, for example C₆-C₁₀-arylcarbonyl such as benzoyl; or aralkylcarbonyl, for example C₆ to C₁₀-aryl-C₁-C₄-alkylcarbonyl such as benzylcarbonyl or phenylethylcarbonyl. Preferably acyl is C₁-C₄-alkylcarbonyl.

"Alkynyl" as used herein denotes straight or branched alkynyl, for example C₂ to C₆-alkynyl such as ethynyl, propargyl, 2-butynyl, pentynyl or hexynyl. Preferably alkynyl is C₂-C₄-alkynyl.

"Aryl" as used herein denotes a monovalent carbocylic aromatic group, for example C₆-C₁₀-aryl such as phenyl, phenyl substituted by one or more, e.g. one, two or three, C₁-C₄-alkyl groups, or naphthyl. Preferably aryl is phenyl.

"Heteroaryl having 5 or 6 ring atoms" as used herein denotes a monovalent aromatic heterocyclic group having 5 or 6 ring atoms of which one, two or three are selected from nitrogen, oxygen and sulfur, such as pyrrolyl, furyl, thienyl, pyridyl, pyrazolyl, imidazolyl, triazolyl, oxazolyl, isoxazolyl, thiazolyl, isothiazolyl, dithiazolyl, trithiazolyl, furazanyl, pyrazinyl, pyrimidinyl or triazinyl.

In alkylamino, dialkylamino, acylamino, dialkylaminosulfonylamino, alkylcarbonyl, alkylcarbonyloxy, alkoxycarbonyl, hydroxyalkyl, alkylthioalkyl and alkoxyalkyl, the alkyl, acyl or alkoxy groups as appropriate have the meanings hereinbefore described.

"Halogen" as used herein may be fluorine, chlorine, bromine or iodine; preferably it is fluorine, chlorine or bromine.

The 5- membered heterocyclic ring to which R⁵ as a quinolinyl, isoquinolinyl or oxodihydroisoquinolinyl group is optionally fused may be, for example, a 5- membered heterocyclic ring having one or two hetero atoms in the ring, said hetero atoms being selected from oxygen, nitrogen and sulfur. Examples of such heterocyclic rings include pyrrole, pyrroline, pyrrolidine, furan, dihydrofuran, tetrahydrofuran, thiophene, dihydrothiophene, tetrahydrothiophene, imidazole, imidazoline, imidazolidine, pyrazole, pyrazoline, pyrazolidine, dioxolane, oxazole, isoxazole, thiazole and isothiazole rings. Preferably the 5- membered heterocyclic ring is a saturated ring having two hetero atoms, preferably two oxygen or two nitrogen atoms, especially two oxygen atoms.

R⁵ as a quinolinyl group may be a 2-quinolinyl, 3-quinolinyl, 4-quinolinyl, 5-quinolinyl, 6-quinolinyl, 7-quinolinyl or 8-quinolinyl group, preferably a 4-quinolinyl, 5-quinolinyl or 8-quinolinyl group. R⁵ as an isoquinolinyl group may be a 1-isoquinolinyl, 3-isoquinolinyl, 4-isoquinolinyl, 5-isoquinolinyl, 6-isoquinolinyl, 7-isoquinolinyl, or 8-isoquinolinyl group, preferably a 1-isoquinolinyl or 4-isoquinolinyl group. In most of the especially preferred embodiments of the invention, R⁵ is a 4-isoquinolinyl group.

R⁵ as a substituted quinolinyl or isoquinolinyl group is preferably substituted by one, two, three or four of the abovementioned substituents, especially one, two or three of those substituents. The preferred substituted 4-isoquinolinyl group is preferably substituted in the 1- and/or 6- and/or 7- and/or 8- position of the isoquinoline ring system.

In especially preferred embodiments of the invention, R⁵ is a quinolinyl group of formula 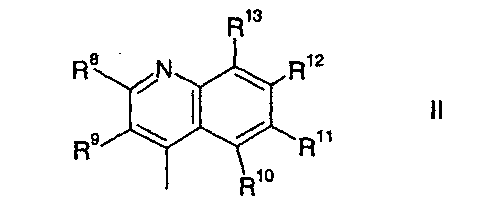 or an isoquinolinyl group of formula 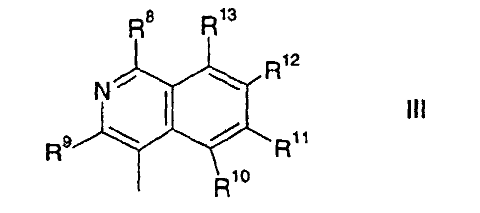 where R⁸, R⁹, R¹⁰, R¹¹, R¹² and R¹³ are each independently hydrogen or a substituent selected from halogen, cyano, hydroxy, alkyl, hydroxyalkyl, alkoxyalkyl, alkylthioalkyl, alkoxy, alkylthio, alkenyl, alkoxycarbonyl, alkynyl, carboxyl, acyl, a group of formula -N(R⁶)R⁷, aryl optionally substituted by one or more substituents selected from halogen or alkoxy, or heteroaryl having 5 or 6 ring atoms, or R¹¹ and R¹² together with the carbon atoms to which they are attached denote a 5- membered heterocyclic group having two oxygen or nitrogen atoms in the ring, and R⁶ and R⁷ are as hereinbefore defined.

R⁵ as an oxodihydroisoquinolinyl group preferably has the oxo group ortho to the ring nitrogen atom, preferably in the 1-position in the isoquinoline ring system. It is preferably linked to the remainder of the molecule of formula I via the ring carbon atom meta to the ring nitrogen atom, i.e. the 4-position in the isoquinoline ring system. An especially preferred oxodihydroisoquinolinyl group is of formula 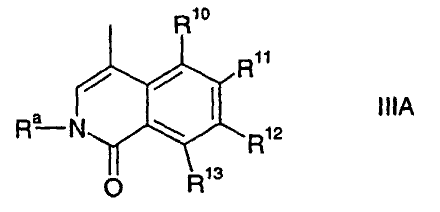 where R¹⁰, R¹¹, R¹² and R¹³ are as hereinbefore defined and R^{a} is hydrogen or C₁-C₄-alkyl,

Preferred among the compounds of formula I in free or salt form are those where
R¹ is hydrogen or C₁-C₄-alkyl optionally substituted by hydroxy, C₁-C₄-alkoxy or C₁-C₄-alkylthio,
R² is hydrogen, C₁-C₈-alkyl, hydroxy-C₁-C₈-alkyl, C₁-C₄-alkylcarbonyloxy -C₁-C₈-alkyl, C₁-C₄-alkoxy -C₁-C₈-alkyl, C₁-C₄-alkylthio-C₁-C₈-alkyl, C₂-C₄-alkenyl, C₃-C₈-cycloalkyl-C₁-C₄-alkyl, heterocyclyl-C₁-C₄-alkyl where the heterocyclyl group is a 5- or 6- membered heterocyclyl group having one or two hetero atoms selected from nitrogen and oxygen atoms in the ring, phenyl-C₁-C₄-alkyl in which the phenyl ring is optionally substituted by one or more substituents selected from C₁-C₄-alkoxy, amino, C₁-C₄-alkylamino, di(C₁-C₄-alkyl)amino, C₁-C₄-alkylcarbonylamino, halogen, C₁-C₄-alkylsulfonylamino, or di(C₁-C₄-alkyl)aminosulfonylamino, and is optionally fused to a 5- membered heterocyclic ring having two oxygen or two nitrogen atoms in the ring,
R³ is hydrogen or C₁-C₄-alkyl optionally substituted by hydroxy, C₁-C₄-alkoxy or C₁-C₄-alkylthio,
R⁴ is hydrogen or C₁-C₄-alkyl,
R⁵ is a quinolinyl, isoquinolinyl or oxodihydroisoquinolinyl group optionally fused to a 5- membered heterocyclic group having two oxygen or two nitrogen atoms in the ring and optionally substituted by one or more substituents selected from halogen, cyano, carboxy hydroxy, C₁-C₄-alkyl, hydroxy-C₁-C₄-alkyl, C₁-C₄-alkoxy-C₁-C₄-alkyl, C₁-C₄-alkylthio-C₁-C₄-alkyl, C₁-C₄-alkoxy, C₁-C₄-alkylthio, C₂-C₄-alkenyl, C₂-C₄-alkynyl, C₁-C₄-alkylcarbonyl, a group -N(R⁶)R⁷ or phenyl optionally substituted by one or more substituents selected from halogen or C₁-C₄-alkoxy and
R⁶ and R⁷ are each independently hydrogen or C₁-C₄-alkyl optionally substituted by hydroxy or alkoxy, or one of R⁶ and R⁷ is hydrogen and the other is C₁-C₄-alkylcarbonyl, or
R⁶ and R⁷ together with the nitrogen atom to which they are attached denote a 5- or 6- membered heterocyclyl group having one or two nitrogen atoms and, optionally, an oxygen atom in the ring.

Further preferred among the compounds of formula I are those where
R¹ is hydrogen or C₁-C₄-alkyl, R² is hydrogen, C₁-C₈-alkyl, hydroxy -C₁-C₈-alkyl, or C₁-C₄-alkylcarbonyloxy-C₁-C₈-alkyl, C₂-C₄-alkenyl, C₃-C₆-cycloalkyl-C₁-C₄-alkyl, heterocyclyl-C₁-C₄-alkyl where the heterocyclyl group is a 5- membered heterocyclyl group having one nitrogen or oxygen atom in the ring, phenyl-C₁-C₄-alkyl in which the phenyl ring is optionally substituted by one or two substituents selected from C₁-C₄-alkoxy, amino, C₁-C₄-alkylcarbonylamino, chlorine, bromine, C₁-C₄-alkylsulfonylamino, or di(C₁-C₄-alkyl)aminosulfonylamino and is optionally fused to a 5- membered heterocyclic ring having two oxygen atoms in the ring,
R³ is hydrogen or C₁-C₄-alkyl,
R⁴ is hydrogen or C₁-C₄-alkyl,
R⁵ is a quinolinyl group of formula II, an isoquinolinyl group of formula III or an oxodihydroisoquinolinyl group of formula IIIA, where R⁸, R⁹, R¹⁰, R¹¹, R¹² and R¹³ are each independently selected from hydrogen, halogen, cyano, carboxy, hydroxy, C₁-C₄-alkyl, hydroxy-C₁-C₄-alkyl, C₁-C₄-alkoxy-C₁-C₄-alkyl, C₁-C₄-alkylthioC₁-C₄-alkyl, C₁-C₄-alkoxy, C₁-C₄-alkylthio, C₂-C₄-alkenyl, C₂-C₄-alkynyl, C₁-C₄-alkylcarbonyl, a group -N(R⁶)R⁷ or phenyl optionally substituted by one or two substituents selected from halogen or C₁-C₄-alkoxy, or R¹¹ and R¹² together with the carbon atoms to which they are attached denote a 5-membered heterocyclic group having two oxygen atoms in the ring, and
R⁶ and R⁷ are each independently hydrogen or C₁-C₄-alkyl optionally substituted by hydroxy or alkoxy or one of R⁶ and R⁷ is hydrogen and the other is C₁-C₄-alkylcarbonyl, or R⁶ and R⁷ together with the nitrogen atom to which they are attached denote a 6-membered heterocyclyl group having one or two nitrogen atoms, or one nitrogen atom and one oxygen atom, in the ring.

Amongst the further preferred compounds hereinbefore described, especially preferred compounds are usually those in which R⁵ is an isoquinolinyl group of formula III in which R⁸ is hydrogen, C₁-C₄-alkyl, halogen, cyano, -N(R⁶)R⁷ where R⁶ and R⁷ are independently C₁-C₄-alkyl or R⁶ and R⁷ together with the nitrogen atom to which they are attached denote a 6-membered heterocyclyl group having one or two nitrogen atoms, or one nitrogen atom and one oxygen atom, in the ring, or phenyl substituted by one or two C₁-C₄-alkoxy groups; R⁹ and R¹⁰ are each independently hydrogen, C₁-C₄-alkyl or halogen; R¹¹ and R¹² are each independently hydrogen, halogen, cyano, carboxy, hydroxy, C₁-C₄-alkyl, C₁-C₄-alkoxy or C₂-C₄-alkynyl, or R¹¹ and R¹² together with the carbon atoms to which they are attached denote a 5- membered heterocycle having two oxygen atoms in the ring; and R¹³ is hydrogen or halogen.

Specific especially preferred compounds of formula I are those hereinafter described in the Examples. More preferred amongst these compounds are those of Examples 7, 10, 15, 35, 45, 49, 55, 60, 68 and 70.

Compounds of formula I may be in the form of salts, particularly pharmaceutically acceptable salts. Pharmaceutically acceptable acid addition salts of compounds of formula I include those of inorganic acids, for example, hydrohalic acids such as hydrofluoric acid, hydrochloric acid, hydrobromic acid or hydroiodic acid, nitric acid, sulfuric acid, phosphoric acid; and organic acids, for example aliphatic monocarboxylic acids such as formic acid, acetic acid, trifluoroacetic acid, propionic acid and butyric acid, aliphatic hydroxy acids such as lactic acid, citric acid, tartaric acid or malic acid, dicarboxylic acids such as maleic acid or succinic acid, aromatic carboxylic acids such as benzoic acid, p-chlorobenzoic acid, diphenylacetic acid or triphenylacetic acid, aromatic hydroxy acids such as o-hydroxybenzoic acid, p-hydroxybenzoic acid, 1-hydroxynaphthalene-2-carboxylic acid or 3-hydroxynaphthalene-2-carboxylic acid, and sulfonic acids such as methanesulfonic acid or benzenesulfonic acid. Pharmaceutically acceptable base salts of compounds of formula I where R³ is hydrogen include metal salts, particularly alkali metal or alkaline earth metal salts such as sodium, potassium, magnesium or calcium salts, and salts with ammonia or pharmaceutically acceptable organic amines or heterocylic bases such as ethanolamines, benzylamines or pyridine. These salts may be prepared from free compounds of formula I or other salts of compounds of formula I by known salt-forming procedures.

The present invention also provides a process for the preparation of compounds of formula I in free or salt form which comprises
1)
   (a) dehydrating a compound of formula 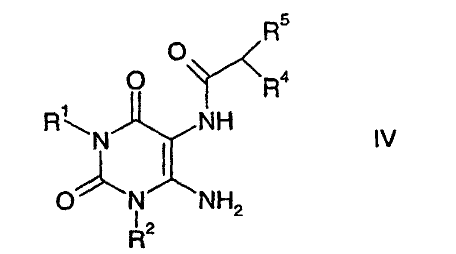 where R¹, R², R⁴ and R⁵ are as hereinbefore defined; or
   (b) for the preparation of a compound of formula I in free or salt form where R³ is alkyl optionally substituted by hydroxy, alkoxy or alkylthio, reacting a compound of formula I in free or salt form with an appropriate alkylating agent, or
   (c) for the preparation of a compound of formula I in free or salt form where R² is aralkyl substituted in the aryl ring by alkylsulfonylamino or dialkylaminosulfonylamino, reacting a compound of formula I in salt form where R² is aralkyl substituted by amino with, respectively, an alkylsulfonyl halide or dialkylaminosulfonyl halide; or
   (d) for the preparation of a compound of formula I in free or salt form where R² is hydroxy-substituted alkyl, hydration of a compound of formula I where R² is alkenyl; or
   (e) for the preparation of a compound of formula I in free or salt form where R² is alkyl substituted by alkylcarbonyloxy, appropriate esterification of a compound of formula I where R² is hydroxy-substituted alkyl; or
   (f) for the preparation of a compound of formula I in free or salt form where R² is aralkyl substituted in the aryl ring by amino, hydrolysing a compound of formula I where R² is aralkyl substituted in the aryl ring by acylamino; or
   (g) for the preparation of a compound of formula I in free or salt form where R⁵ is quinolinyl or isoquinolinyl substituted by hydroxy, dealkylation of a compound of formula I where R⁵ is respectively quinolinyl or isoquinolinyl substituted by alkoxy, particularly methoxy; or
   (h) for the preparation of a compound of formula I in free or salt form where R⁵ is quinolinyl or isoquinolinyl substituted by halogen, halogenation of a compound of formula I where R⁵ is respectively quinolinyl or isoquinolinyl having an unsubstituted ring carbon atom available for halogenation; or
   (i) for the preparation of a compound of formula I in free or salt form where R² is a cyclopropyl group, optionally substituted by alkyl, subjecting a compound of formula I where R² is alkenyl to a Simmons Smith cyclopropanation reaction; and
2) recovering the resulting product of formula I in free or salt form.

Process (a) may be carried out by heating, or by reaction with an inorganic or organic base. It may be effected in an organic or aqueous solvent or mixed aqueous/organic solvent. The reaction with base may be carried out at ambient temperature or, more conveniently, at elevated temperature. The reaction is preferably carried out by treatment with aqueous alkali metal hydroxide in an alcoholic solvent at elevated temperature, for example as described hereinafter in the Examples. The compound of formula IV is preferably a compound where R⁵ is a group of formula II or III. Compounds of formula IV may be prepared by reacting a compound of formula 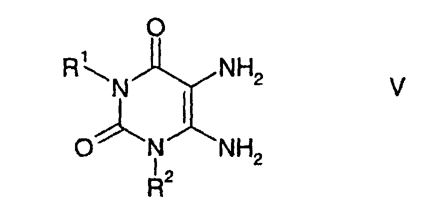 where R¹ and R² are as hereinbefore defined, with a compound of formula 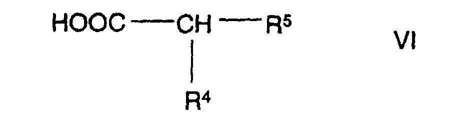 or an amide-forming derivative thereof, where R⁴ and R⁵ are as hereinbefore defined. The reaction may be effected by treating the carboxylic acid of formula VI with a peptide coupling agent to form in situ an activated ester or mixed anhydride, followed by treatment with the compound of formula V in an organic, e.g. dipolar aprotic, or mixed aqueous-organic (e.g. chlorohydrocarbon) solvent. The latter treatment may be carried out at sub-ambient, ambient or elevated temperature, conveniently at ambient temperature. Preferably, the acid of formula VI is treated with a carbodiimide derivative in the presence of hydroxybenzotriazole and, optionally, a base, or is treated with a benzotriazolyl-(trisdialkylamino)-oxyphosphonium salt. The resulting intermediate is preferably treated with the compound of formula V in a dipolar aprotic solvent or mixed chlorohydrocarbon-aqueous solvent at ambient temperature. Procedures may be as hereinafter described in the Examples.

Compounds of formula V may be prepared by reduction of a compound of formula 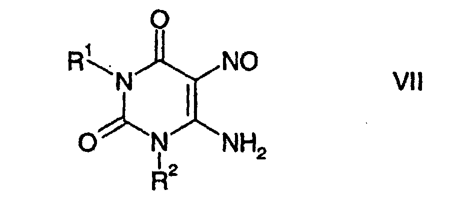 where R¹ and R² are as hereinbefore defined.

The reduction may be effected using known procedures, for example by treating the compound of formula VII with a reducing agent in an organic or aqueous solvent. The reaction may be carried out at ambient or, more conveniently, at elevated temperature. Preferred reducing agents are alkali metal dithionite salts in aqueous media or hydrogen in the presence of a noble metal catalyst. Treatment with sodium dithionite in aqueous solution at 80-90°C is particularly preferred.

Compounds of formula VII may be prepared by nitrosation of a compound of formula 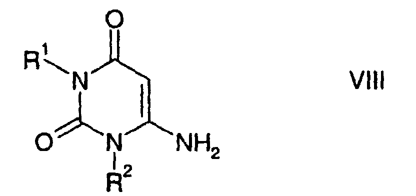 where R¹ and R² are as hereinbefore defined, for example with an organic or inorganic nitrosating agent in an organic or aqueous or mixed organic-aqueous solvent. Nitrosation may be effected using known procedures at sub-ambient, ambient or elevated temperature, preferably with an alkali metal nitrite such as sodium nitrite in the presence of an acid such acetic acid at sub-ambient or ambient temperature, preferably in a mixed alcoholic-aqueous solvent such as aqueous ethanol.

Compounds of formula VIII may be prepared by reacting a compound of formula 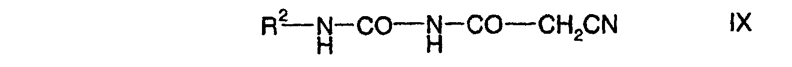 where R² is as hereinbefore defined with an inorganic or organic base to effect cyclisation, followed, where R¹ is an optionally substituted alkyl group, by reaction with an alkylating agent. The cyclisation reaction may be effected using conventional procedures. It is conveniently carried out in an aqueous, organic or mixed organic-aqueous solvent. Reaction may be effected at ambient or, more conveniently, elevated temperature. The base is preferably an alkali metal hydroxide, especially sodium hydroxide, which is preferably reacted in a mixed aqueous-alcoholic solvent, preferably at elevated temperature, e.g. 80-90° C. The optional alkylation step can be effected using known procedures, for example in the presence of an inorganic or organic base, for example in an aqueous, organic or mixed aqueous-organic solvent. Alkylation may be carried out at sub-ambient temperature or, more conveniently, at ambient or elevated temperature. Preferred alkylating agents are alkyl iodides or, especially, dialkyl sulfates. Preferred bases are alkali metal hydroxides in aqueous alcoholic solvents, especially aqueous ethanol.

Compounds of formula IX may be prepared by reacting a compound of formula 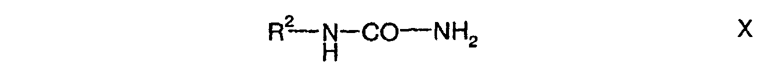 with cyanoacetic acid or an amide-forming derivative thereof such as an ester or acid halide thereof, preferably the acid or its ethyl ester. The reaction may be effected using known procedures, for example in an organic solvent, preferably an anhydride such as acetic anhydride. The reaction temperature may be ambient or, more conveniently, elevated temperature, e.g. 65 to 70°C.

Compounds of formula X may be prepared using conventional procedures, for example from an isocyanate R²NCO by reaction with gaseous or aqueous ammonia or from an amine R²NH₂ by reaction with a metal cyanate, for example as hereinafter described in the Examples.

Compounds of formula VIII where R¹ is alkyl optionally substituted by hydroxy, alkoxy or alkylthio and R² is as hereinbefore defined other than hydrogen, may be prepared by hydrogenolysis of a compound of formula 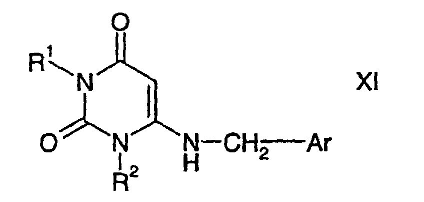 where R¹ is alkyl optionally substituted by hydroxy, alkoxy or alkylthio, R² is as hereinbefore defined other than hydrogen and Ar is phenyl optionally substituted by one or more C₁-C₄-alkoxy, preferably methoxy, groups. The hydrogenolysis may be carried out in a known manner, e.g. by treatment with hydrogen or a source of hydrogen and a metal catalyst such as a platinum or, preferably, palladium catalyst. The reaction may be carried out in an organic solvent. The reaction temperature may be ambient or elevated. Preferably hydrogenolysis is effected using palladium black in formic acid, e.g. as hereinafter described in the Examples.

Compounds of formula XI may be prepared by reacting a compound of formula 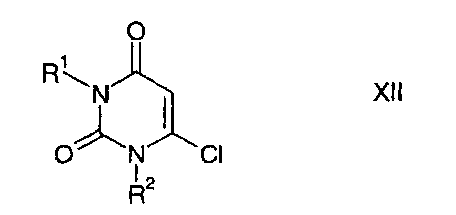 where R¹ and R² are as hereinbefore defined for formula XI, with a compound of formula ArCH₂NH₂ where Ar is as hereinbefore defined. The reaction may be carried out in a known manner, e.g. in an organic solvent, preferably an alcohol such as n-butanol, at ambient or elevated temperature, or analogously as hereinafter described in the Examples.

Compounds of formula XII may be prepared by reacting a compound of formula 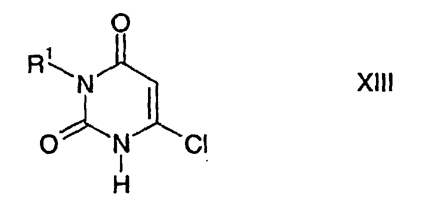 where R¹ is as hereinbefore defined for formula XI, with a compound of formula R²X where R² is as hereinbefore defined for formula XI and X is halogen or hydroxy, where X is hydroxy, the reaction being carried out in the presence of activating reagents, preferably an azodicarboxylate such as di-t-butyl azodicarboxylate together with a triarylphosphine such as diphenylpyridylphosphine. The reaction may be carried out in an organic solvent, preferably an ether such as dioxan. The reaction temperature may be sub-ambient or, preferably, ambient or elevated temperature. The reaction may be carried out using the procedure of Mitsonobu, Synthesis 1981, 1, or analogously as hereinafter described in the Examples. Compounds of formula XIII are known or may be prepared by known procedures.

Compounds of formula VI may be prepared, for example, (i) from benzaldehyde or a substituted benzadehyde using the procedure of Dyke et al, Tetrahedron 1968, 24, 1467 or (ii) from an optionally substituted, N-protected 1,2-dihydroisoquinoline by reaction with a 2-oxo-carboxylic acid using the procedure of Dyke et al, Tetrahedron 1968, 24, 1467, optionally followed by conversion of the resulting carboxylic acid into a methyl ester and then an alkali metal salt using the procedure of J. March, Advanced Organic Chemistry, 4th Edition, Wiley, New York, 1992, pages 393 and 378 or (iii) from an optionally substituted quinoline or isoquinoline by reaction with a hydride reducing agent followed by a 2-oxocarboxylic ester using the procedure of Minter et al, J. Org. Chem. 1988, 53, 2653 or (iv) by introducing substituents onto the N-containing ring of an acid of formula VI using the procedures of Janin and Biagani, Tetrahedron 1993, 39, 10305, or Ford et al, J. Med. Chem, 1985, 28, 164.

Certain preferred compounds of formula VI may be prepared by
(i) the reaction sequence 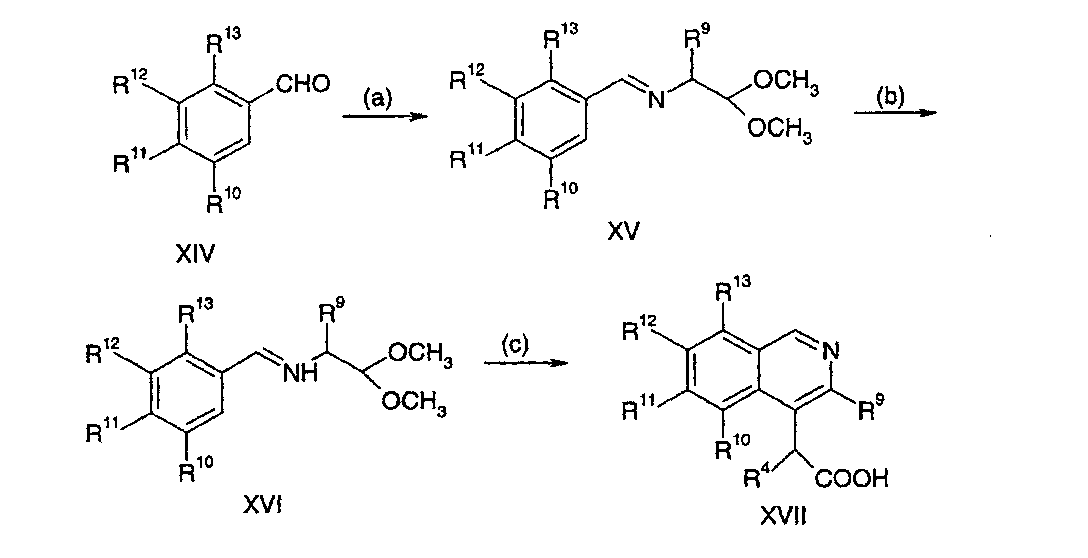 where R⁴, R⁹, R¹⁰, R¹¹, R¹² and R¹³ are as hereinbefore defined. Steps (a) to (c) may be carried out in a known manner, e.g. using the procedure of Dyke et al, Tetrahedron 1968, 24, 1467, or analogously as hereinafter described in the Examples;
(ii) the reaction sequence 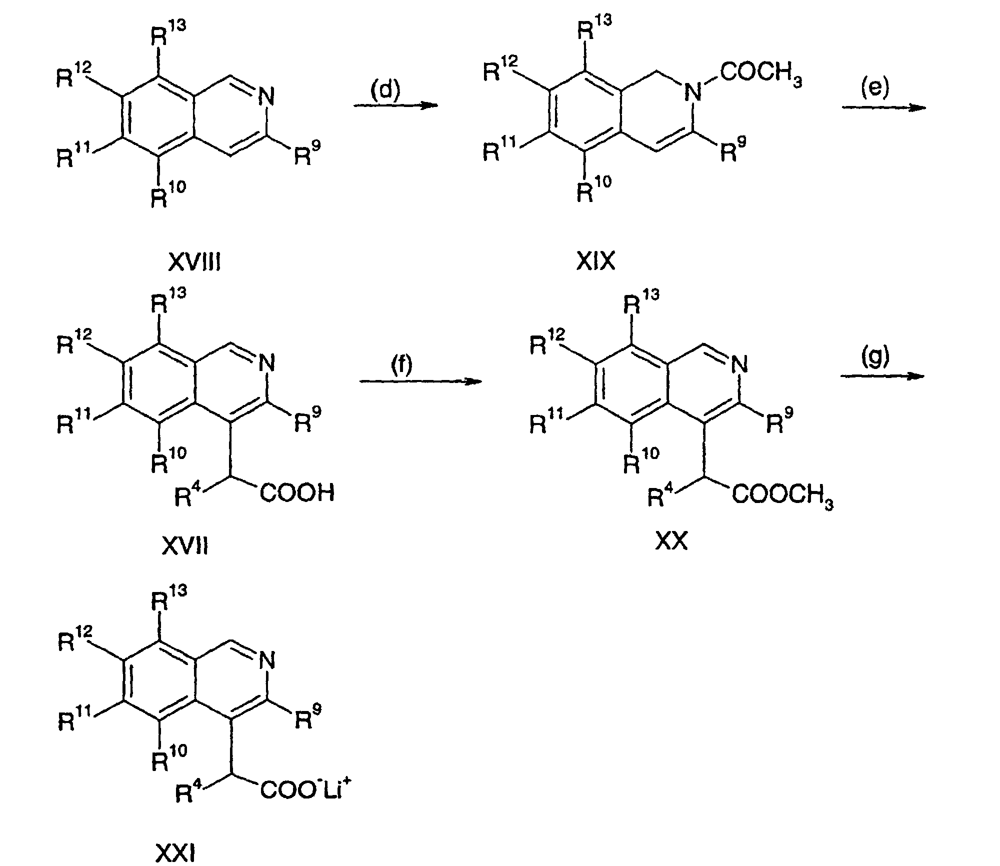 where R⁴, R⁹, R¹⁰, R¹¹, R¹² and R¹³ are as hereinbefore defined. Steps (d) to (g) may be carried out in a known maner, e.g. step (d) using the procedure of Katayama et al, Chem. Pharm. Bull, 1980, 28, 2226, step (e) using the procedure of Dyke et al, Tetrahedron 1968, 24, 1467 and steps (f) and (g) using the procedure of J. March, Advanced Organic Chemistry, 4th Edition, Wiley, New York, 1992, pages 393 and 378, or analogously as hereinafter described in the Examples;
(iii) the reaction 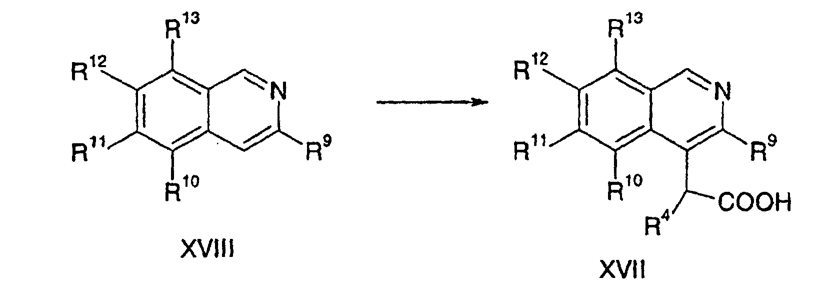 e.g. by treatment of XVIII with a hydride reducing agent, followed by a 2-oxo-carboxylic ester, using the procedure of Minter et al, J. Org. Chem. 1988, 53, 2653, or analogously as described hereinafter in the Examples;
(iv) for the preparation of compounds of formula VI in which R⁵ is a 4-isoquinolinyl group substituted in the 1- position, the reaction sequence 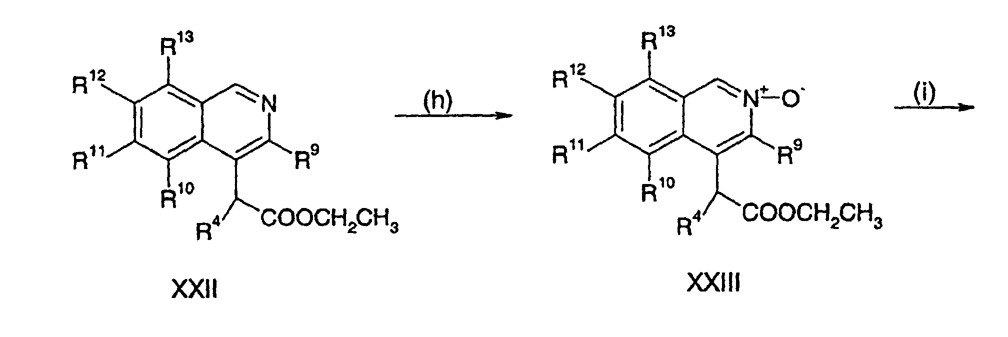 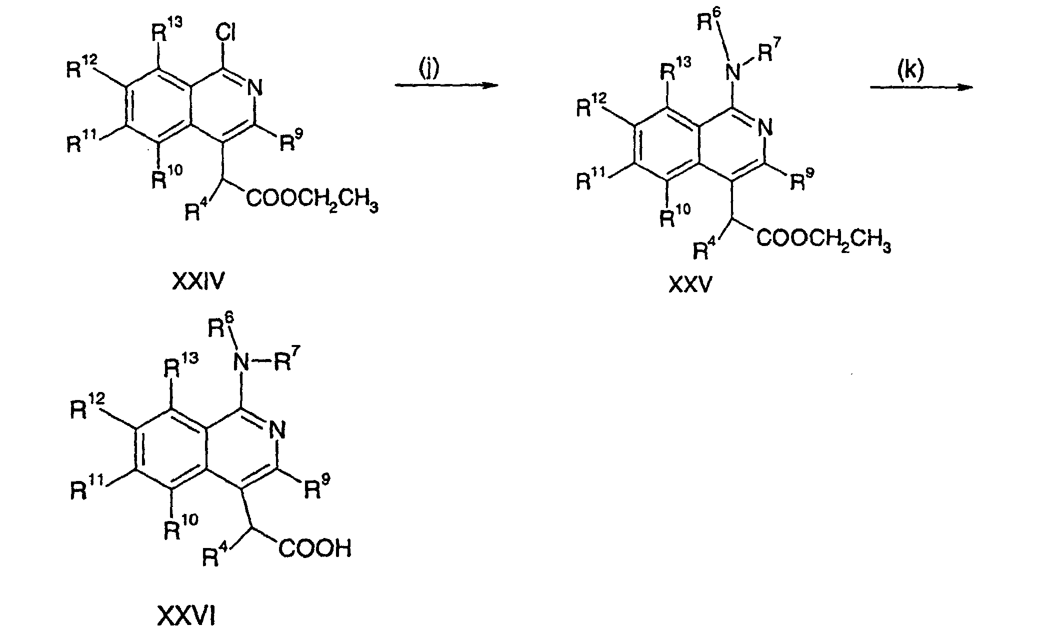 where R⁴, R⁶, R⁷, R⁹, R¹⁰, R¹¹, R¹² and R¹³ are as hereinbefore defined. Steps (h) to (k) may be carried out in a known manner, e.g. steps (h) to (j) using the procedure of Janin and Biagni, Tetrahedron, 1993, 39, 10305 and step (k) using the procedure of J. March, Advanced Organic Chemistry, 4th Edition, New York, 1992, page 378 or analogously as hereinafter described in the Examples;
(v) for the preparation of compounds of formula VI in which R⁵ is an isoquinolinyl group of formula III in which R⁸ is cyano, the reaction sequence 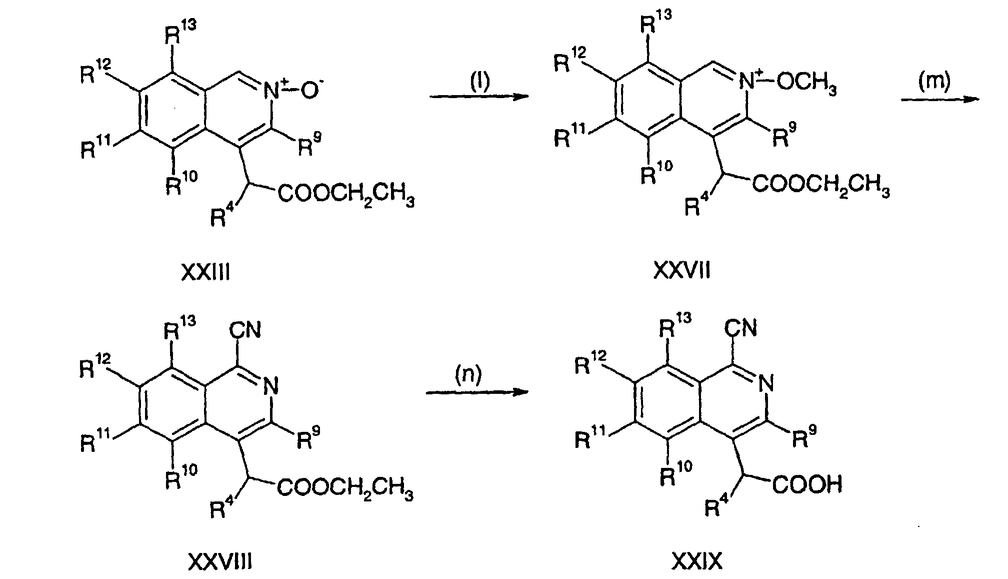 where R⁴, R⁹, R¹⁰, R¹¹, R¹² and R¹³ are as hereinbefore defined. Steps (l) to (n) may be carried out in a known manner, e.g. steps (l) and (m) using the procedure of Ford et al, J. Med. Chem., 1985, 28, 164 and step (n) using the procedure of J. March, op.cit., page 378;
(vi) for the preparation of compounds of formula VI in which R⁵ is an oxodihydroisoquinolinyl group, the reaction sequence 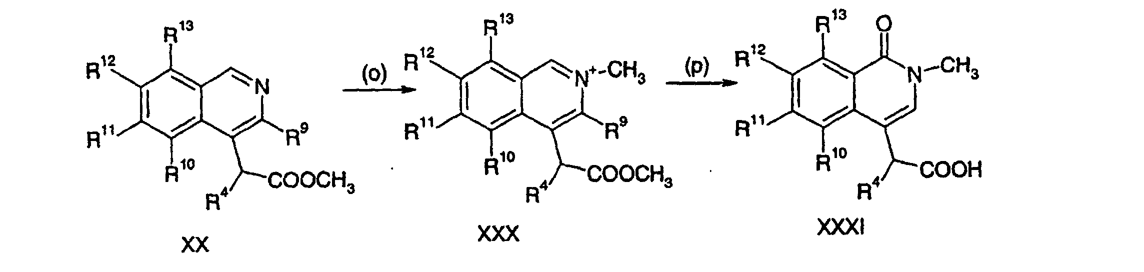 where R⁴, R⁹, R¹⁰, R¹¹, R¹² and R¹³ are as hereinbefore defined. Steps (o) and (p) may be carried out in a known manner, e.g. using the procedure of Holzgrabe, Arch. Pharm. (Weinheim, Ger.), 1988, 321, 767, or analogously as hereinafter described in the Examples;
(vii) for preparation of compounds of formula VI where R⁵ is a quinolinyl group, the reaction 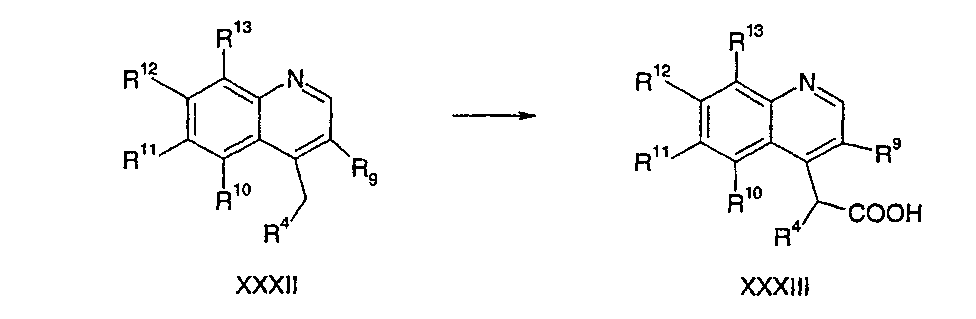 where R⁴, R⁹, R¹⁰, R¹¹, R¹² and R¹³ are as hereinbefore defined, which may be carried out in a known manner, e.g. by treatment with a strong base, preferably an alkali metal dialkylamide such as lithium diisopropylamide, followed by treatment with carbon dioxide, e.g. using the procedure of using Brown and Curless, Tetrahedron Lett., 1986, 27, 6005, or analogously as hereinafter described in the Examples.
(viii) for the preparation of compounds of formula VI where R⁵ is a 4-isoquinolinyl group, 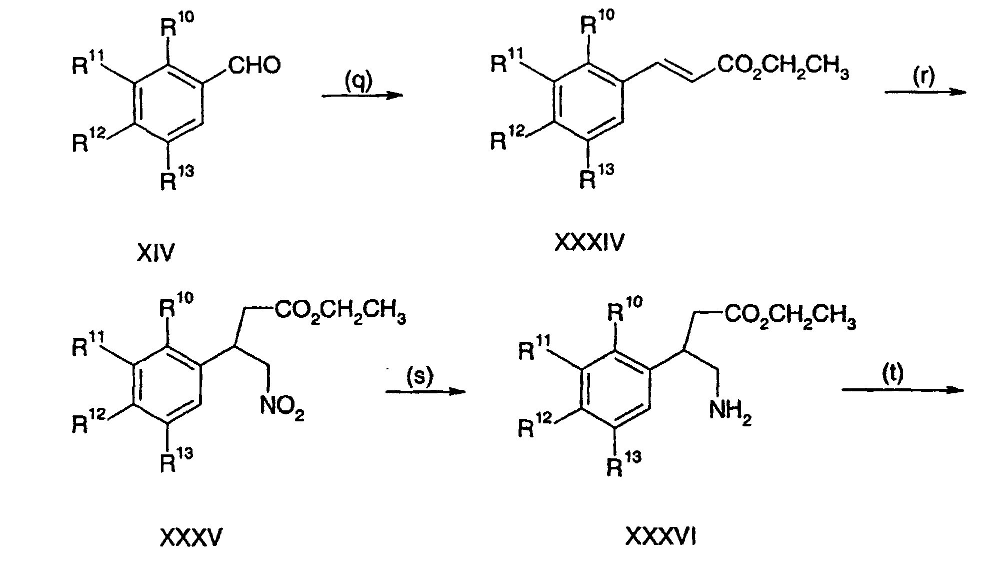 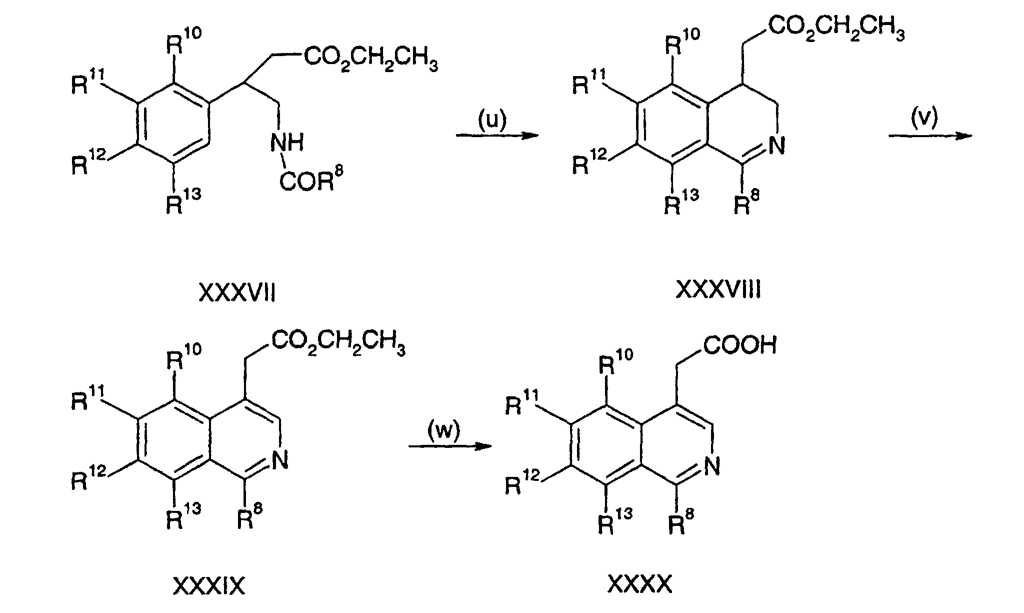 where R⁸, R¹⁰, R¹¹, R¹² and R¹³ are as hereinbefore defined. Steps (q) to (w) may be carried out in a known manner; e.g. step (q) by treatment with a carboxyethyltriarylphosphonium ylid, preferably carboxyethyltriphenylphosphonium ylid in an organic solvent, preferably an ether or hydrocarbon, especially toluene, at sub-ambient, elevated or, preferably, ambient temperature; step (r) by treatment with nitromethane in the presence of an inorganic or, preferably, amine base, especially tetramethylguanidine, for example in the presence of a solvent or, preferably, in the absence of a solvent, at sub-ambient, ambient or, preferably, elevated temperature, e.g. 60-80°C; step (s) by treatment with a reducing agent, preferably a tin (II) salt, especially tin (II) chloride hydrate, in an aqueous or, preferably, organic solvent, preferably an alcohol such as ethanol, at sub-ambient, ambient or, preferably, elevated temperature, e.g. under reflux; step (t) by treatment with an acid halide or anhydride, preferably the acid chloride, of the acid R⁸COOH, at elevated or, preferably, sub-ambient or ambient temperature, e.g. 0°C to ambient temperature, in an aqueous or, preferably, organic solvent, especially a chlorinated solvent such as dichloromethane, preferably in the presence of a base, especially an amine such as triethylamine; step (u) by treatment with a phosphorus (V) halide or oxyhalide, preferably phosphorous pentachloride or phosphorus oxychloride, preferably in an organic solvent such as a hydrocarbon or nitrite, especially acetonitrile, preferably at ambient or, especially, elevated temperature, e.g. under reflux; step (v) by treatment with a noble metal, preferably palladium, catalyst, preferably in an organic solvent, especially a hydrocarbon such as decalin, preferably at elevated temperature, e.g. under reflux; step (w) by treatment with an alkali metal hydroxide, preferably lithium or sodium hydroxide, in organic, aqueous or mixed organic-aqueous solvent, preferably THF-water, at sub-ambient, elevated or, preferably, ambient temperature; specific methods for steps (q) to (w) being as hereinafter described in the Examples.
(ix) the reaction sequence 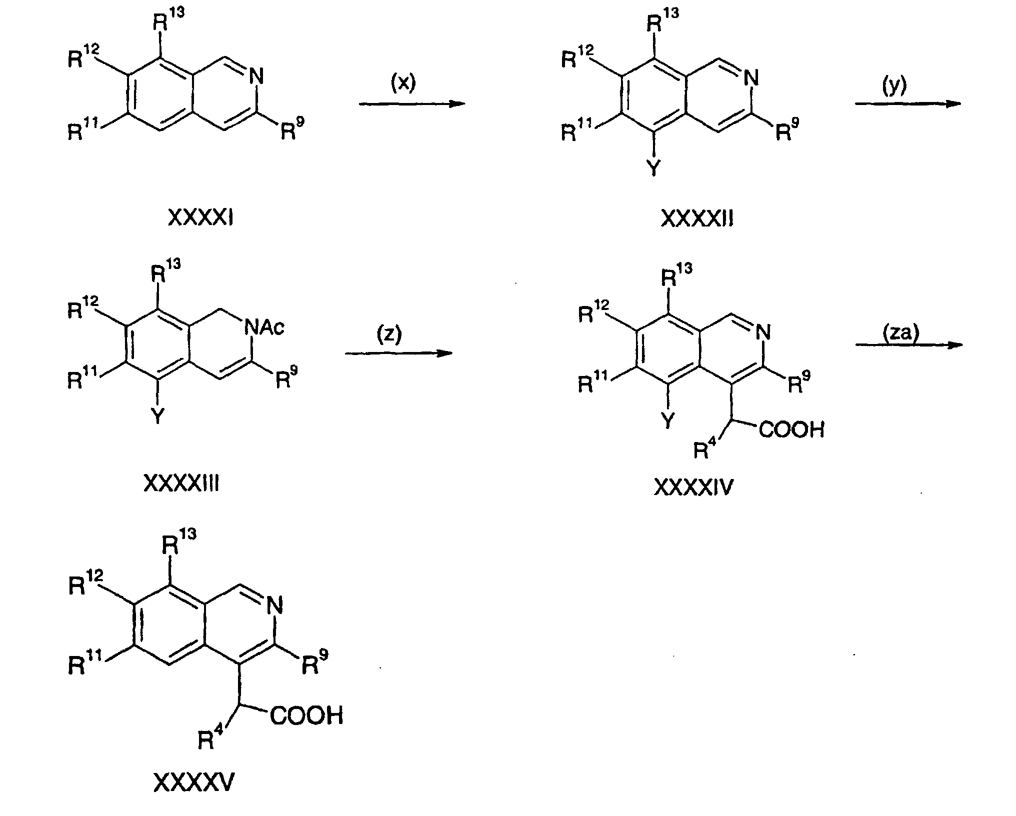
where R⁹, R¹¹, R¹² and R¹³ are as hereinbefore defined, Ac is an acyl group, and Y is halogen. Steps (x) to (za) may be effected in a known manner, e.g. step (x) by reaction with a halogenation agent, e.g. bromine or a N-halosuccinimide, preferably N-chlorosuccinimide, e.g. as described in J.March, op.cit., page 531; step (y) by reaction with a reducing agent, e.g. a metal hydride, in the presence of an acylating agent, e.g. acetic anhydride, e.g. as described in Katayama et al, op.cit; step (z) by reaction with a 2-oxocarboxylic acid, preferably glyoxylic acid, in the presence of a mineral acid, e.g. as described in Dyke et al, Tetrahedron 1968, 24, 1467; and step (za) by treatment with a reducing agent, e.g. as described in J.March et al, op.cit, page 566; or analogously as described hereinafter in the Examples.

Certain compounds of formula V are novel, including Intermediates 1 to 10 as described hereinafter. Certain compounds of formula VI are novel, including Intermediates 20 to 48 as described hereinafter.

Process variant (b) may be carried out in a known manner, for example by reacting a compound of formula I where R³ is hydrogen with an appropriate alkylating agent, preferably an alkyl iodide or dialkyl sulfate, such as a compound of formula R³I or (R³)₂SO₄ where R³ is C₁-C₄-alkyl. The reaction may be conducted in the presence of an inorganic or organic base, for example in an aqueous, organic or mixed aqueous-organic solvent. Alkylation may be carried out at sub-ambient temperature or, more conveniently, at ambient or elevated temperature. Preferred bases are alkali metal carbonates. Preferred solvents are organic dipolar aprotic solvents, especially N,N-dimethylformamide.

Process variant (c) may be effected using known sulfonylation procedures, e.g. in the presence of an organic or inorganic base, preferably a tertiary organic base such as pyridine. The reaction temperature may be sub-ambient, ambient or, preferably, elevated. Preferred procedures are as hereinafter described in the Examples.

Process variant (d) may be effected using known procedures, e.g. by treating a compound of formula I wherein R² is alkenyl with a hydroborating agent, followed by oxidative basic work-up. Hydroboration may be carried out at sub-ambient or, more conveniently, at ambient or elevated, temperature. Preferred hydroborating agents are dialkylboranes such as 9-borabicyclo[2.2.0]nonane, which are preferably reacted under reflux. Oxidative work-up is preferably conducted with hydrogen peroxide and an alkali metal hydroxide, preferably sodium hydroxide. The work-up temperature is preferably 40-60°C.

Process variant (e) may be carried out using conventional esterification procedures, e.g. by reacting the compound of formula I wherein R² is hydroxy with a carboxylic acid or halide thereof, preferably an acid chloride, in the presence of an organic or inorganic base, at sub-ambient or, preferably, ambient or elevated (e.g. 40-60°C) temperature. Preferred bases are organic tertiary bases such as pyridine.

Process variant (f) may be carried out using known procedures for conversion of acylamino into amino, e.g. by treatment with a mineral acid such as sulphuric or, preferably, hydrochloric acid. The reaction is preferably carried out in a mixed aqueous-organic solvent such as aqueous ethanol. The reaction temperature is conveniently ambient or, preferably, elevated temperature, especially reflux temperature.

Process variant (g) may be effected using known dealkylation methods, e.g. by reaction with HBr or HI, usually at elevated temperature, preferably by heating with concentrated hydrobromic acid, e.g. as hereinafter described in the Examples.

Process variant (h) may be effected using known halogenation procedures, e.g. by reaction with bromine or chlorine in a solvent such as acetic acid. The reaction is conveniently carried at ambient temperature, e.g. as hereinafter described in the Examples.

Process variant (i) may be effected using known procedures for the Simmons Smith reaction, e.g. by reaction with diethyl zinc and chloroiodomethane. The reaction is usually carried out in an organic solvent, preferably a halohydrocarbon. The reaction is suitably carried out at ambient temperature, e.g. as hereinafter described in the Examples.

Compounds of formula I in free form may be converted into salt form, and vice versa, in a conventional manner. The compounds in free or salt form can be obtained in the form of hydrates or solvates containing a solvent used for crystallization. The compounds of formula I in free or salt form can be recovered from reaction mixtures in a conventional manner. Isomer mixtures can be separated into individual isomers, e.g. enantiomers, in a conventional manner, e.g. by fractional crystallization.

Compounds of formula I in free or pharmaceutically acceptable salt form, hereinafter referred to alternatively as agents of the invention, are useful as pharmaceuticals. In particular, they are inhibitors of cyclic guanosine-3',5'-monophosphate phosphodiesterases (cGMP PDEs), especially PDE5. Agents of the invention are selective PDES inhibitors; in particular, they exhibit good selectivity for inhibition of PDE5 relative to inhibition of other phosphodiesterases, particularly PDE1 and PDE6, indicating a low side-effect profile.

Furthermore agents of the invention have an appropriate duration of action and many have a rapid onset of action. The inhibiting properties of agents of the invention may be demonstrated in the following test procedure:

PDE5 Assay: A 10 mM solution of a test compound in DMSO is diluted 100-fold with aqueous 20% v/v DMSO to give a 100 µM stock solution, which is further diluted with aqueous 20% v/v DMSO to give ten solutions having concentrations from 10 µM to 0.00051 µM. 10 µL of each of these solutions is transferred to a selected well of a 96-well Optiplate microtitre plate (ex Packard). To determine total binding, 10 µl of aqueous 20% v/v DMSO is added to other selected wells. To determine non-specific binding, a 10 mM solution of sildenafil in 100% DMSO is diluted 20-fold with aqueous 20% v/v DMSO and 10 µl of the resulting solution is added to further selected wells of the Optiplate plate. To all wells containing test compound solution, aqueous DMSO or sildenafil solution is added 80 µl of Assay Mix, prepared by mixing PDE Assay Buffer (2 ml), an aqueous solution of bovine serum albumin (BSA) containing 5mg BSA/ml (2 ml), an aqueous 75 µM solution of cGMP sodium salt (0.2 ml), 3H-cGMP (ex Amersham, 10 µl) and distilled water (11.8 ml). (The PDE Assay Buffer is prepared by dissolving Tris-base (7.56g) in water (800 ml), adding 1M aqueous MgCl₂ (10.325 ml) and 0.5 M EDTA (4.25 ml), adjusting the pH to 7.5 with 1N hydrochloric acid and making up to 1 litre with water). A solution of human PDE5, partially purified from human platelets (11µl, containing 0.017 units of PDE5 per ml, where 1 unit hydrolyses 1.0 µmole of 3',5'-cyclic GMP to 5'-GMP per minute at pH 7.5 at 37°C), in 20mM Hepes, pH7.4, 100mM sodium chloride, 10% v/v glycerol, 1mM benzamidine and 2mM dithiotherietol, is diluted 200-fold, with Enzyme Buffer prepared by adding 0.5M EDTA (2 ml) to a solution of Tris-Base (1.21g) in water (800 ml), adjusting the pH to 7.5 with 1N HCl and making up to 1 litre with water. The diluted PDE5 solution (10 µl) is added to all wells containing test compound, aqueous DMSO or sildenafil solution. The plate is incubated at room temperature for 1 hour. 50 µl of a suspension of 500 mg PDE Yttrium silicate SPA beads (ex Amersham) in 28 ml water is added to each of the wells and the plate is incubated for a further 20 minutes and then sealed using Top Seal-S (ex Packard) according to the manufacturer's instructions. The resulting scintillations are counted using a Canberra Packard Top Count (1 minute per well), as a measure of the extent to which binding of PDE5 to the beads is inhibited. The concentration of test compound at which 50% inhibition of PDE5 binding to the beads occurs (IC₅₀) is determined from concentration-inhibition curves in a conventional manner.

Compounds of the Examples hereinbelow have IC₅₀ values of the order of from 0.0005µM to 10µM in the above assay. For example, the compounds of Examples 7, 10, 15, 35, 45, 49, 55, 60, 68 and 70 have IC₅₀ values of 0.007µM, 0.01µM, 0.006µM, 0.010µM, 0.002µM, 0.0037µM, 0.0055µM, 0.0028µM, 0.007µM and 0.009µM respectively in the above assay.

Having regard to their inhibition of PDE5, agents of the invention are useful in the treatment of conditions which are mediated by PDE5. Treatment in accordance with the invention may be symptomatic or prophylactic.

Accordingly, agents of the invention are useful in the treatment of sexual dysfunction, including male erectile dysfunction and female sexual dysfunction, premature labour, dysmenorrhoea, benign prostatic hyperplasia, bladder outlet obstruction, incontinence, stable, unstable and variant (Prinzmetal) angina, hypertension, pulmonary hypertension, congestive heart failure, atherosclerosis, conditions of reduced blood vessel patency, e.g. post-percutaneous transluminal coronary angioplasty, peripheral vascular disease, bronchitis, asthma, allergic rhinitis, glaucoma, tinnitus, diseases characterised by disorders of gut motility, e.g. irritable bowel syndrome, pre-eclampsia, Kawasaki's syndrome, nitrate tolerance, multiple sclerosis, peripheral diabetic neuropathy, stroke, Alzheimer's disease, acute respiratory failure, psoriasis, skin necrosis, cancer, metastasis, baldness, nutcracker oesophagus, anal fissure and hypoxic vasoconstriction.

Agents of the invention are of particular interest for use in the treatment of sexual dysfunction, especially male erectile dysfunction.

In accordance with the foregoing, the invention also provides a method for the treatment of a condition mediated by PDE5, for example a condition mentioned hereinbefore, particularly sexual dysfunction, especially male erectile dysfunction, which comprises administering to a subject, particularly a human subject, in need thereof an effective amount of a compound of formula I in free form or in the form of a pharmaceutically acceptable salt. In another aspect, the invention provides a compound of formula I, in free form or in the form of a pharmaceutically acceptable salt, for use in the manufacture of a medicament for the treatment of a condition mediated by PDE5, for example a condition as mentioned hereinbefore, particularly sexual dysfunction, especially male erectile dysfunction.

Agents of the invention may be administered by any appropriate route, e.g. orally, for example in the form of a tablet, a capsule, a solution or a suspension; parenterally, for example intravenously, intracavernosally, intramuscularly or subcutaneously; intranasally, for example in the form of an aerosol or aqueous dispersion; by inhalation, for example as an aerosol, a nebulized aqueous dispersion or a dry powder; buccally or sublingually, for example in the form of a tablet or lozenge; topically to the skin, for example in the form of a cream or ointment; or rectally, e.g. as a suppository.

In a further aspect the invention provides a pharmaceutical composition comprising as active ingredient a compound of formula I in free form or in the form of a pharmaceutically acceptable salt, optionally together with a pharmaceutically acceptable diluent or carrier therefor. Such compositions may be prepared using conventional excipients and techniques known in the galenic art. Thus oral dosage forms may include tablets and capsules. Compositions for inhalation may include aerosol or other atomizable formulations or dry powder formulations. Compositions for topical administration to the skin may include creams, ointments or gels.

The agents of the invention may also be used in combination with other PDES inhibitors or with other therapeutic agents suitable for the treatment of sexual dysfunction, particularly male erectile dysfunction, e.g. α adrenergic receptor antagonists such as phentolamine methanesulfonate, dopamine D2 agonists such as apomorphine or NO donors such as L-arginine. An agent of the invention may be mixed with the co-therapeutic agent in a pharmaceutical composition or it may be administered separately, before, simultaneously with or after the co-therapeutic agent.

This invention is illustrated by the following Examples.

Intermediates of formula V are prepared as follows:

### Intermediate 1

Methallylamine (211g, 2.97mol) is added to a solution of concentrated hydrochloric acid (250ml) in water (1.91), followed by portionwise addition of potassium cyanate (240g, 2.97mol). The reaction is then heated for 2 hours at 80°C, prior to cooling and evaporation to afford (2-methyl-allyl)-urea (244.5g), mp 114-115°C. The urea (268g, 2.35mol) is added to a solution of cyanoacetic acid (220g, 2.59mol) in acetic anhydride (536ml) and the reaction is heated at 70°C for 1 hour, cooled to 0°C and diluted with ether. The resultant solid is collected by filtration, washed with ether, suspended in water (2.21) and heated to 75°C. 2M aqueous sodium hydroxide solution is then added portionwise over 30 min to maintain pH between 8 and 9.5. The reaction is cooled to room temperature, treated with acetic acid (12ml), further cooled to 10°C and the resultant solid is collected by filtration, washed with cold water and dried to afford 6-amino-1-(2-methyl-allyl)-1*H*-pyrimidine-2,4-dione, mp 267-269°C. The uracil (253g, 1.40mol) is added to a solution of sodium hydroxide (123g, 3.07mol) in water (2.51) and allowed to exotherm then cooled to 20°C. Dimethyl sulfate (196ml, 2.06mol) is added portionwise over 1 hour. After standing overnight, the reaction is cooled to 5°C and the solid collected by filtration to give 6-amino-3-methyl-1-(2-methyl-allyl)-1*H*-pyrimidine-2,4-dione, mp 162-163°C. The methyluracil (165g, 0.85mol) is suspended in water (1.551) and concentrated hydrochloric acid (72ml). A solution of sodium nitrite (58.4g, 0.85mol) in water (117ml) is then added dropwise over 30 minutes and the reaction is stirred at 20°C for 3 hours. The solid is collected by filtration, washed successively with water, methanol and ether to afford 6-amino-3-methyl-1-(2-methyl-allyl)-5-nitroso-1*H*-pyrimidine-2,4-dione, mp 213°C (dec). The nitrosouracil (190g, 0.85mol) is suspended in water (950ml), heated to 85°C and sodium dithionite (85%, 347.2g, 1.69mol) is added portionwise. After cooling to room temperature, the solid is collected by filtration to afford 5,6-diamino-3-methyl-1-(2-methyl-allyl)-1*H*-pyrimidine-2,4-dione, mp 152-153°C.

### Intermediate 2

Using the general procedure for Intermediate 1, (3-nitrobenzyl)-urea [J. Med. Chem. 1996, 39, 1924] is converted into 6-amino-3-methyl-1-(3-nitro-benzyl)-1*H*-pyrimidine-2,4-dione, [M-H]⁻ 275. A suspension of this compound (4.88g, 17.7mmol) and 10% Pd/C (0.484g) in ethanol (200ml) is hydrogenated at 1 atmosphere for 1.5 hours. The reaction mixture is filtered through a celite plug and evaporated to give 6-amino-1-(3-amino-benzyl)-3-methyl-1*H*-pyrimidine-2,4-dione acetic acid salt [M-H]⁻ 245. Acetic anhydride (1.85ml, 19.57mmol) is added to a cooled (0°C) suspension of 6-amino-1-(3-amino-benzyl)-3-methyl-1*H*-pyrimidine-2,4-dione acetic acid salt (5.01g, 16.35mmol) in pyridine (50ml). The reaction mixture is warmed to room temperature, stirred for 6 hours and the solvent evaporated. The residue is triturated with water and the solid collected by filtration and dried to afford N-[3-(6-amino-3-methyl-2,4-dioxo-3,4-dihydro-2*H*-pyrimidin-1-ylmethyl)-phenyl]-acetamide, ¹H NMR (400MHz, DMSO): δ: 2.00 (s 3H), 3.09 (s 3H), 4.72 (s 1H), 5.02 (s 2H), *6.75* (s 2H), 6.88 (d *J* 6 1H), 7.25 (t *J* 6 1H), 7.30 (s 1H), 7.55 (d *J* 6 1H). Using the general procedure for Intermediate 1, this compound is converted into N-[3-(5,6-diamino-3-methyl-2,4-dioxo-3,4-dihydro-2H-pyrimidin-1-ylmethyl)-phenyl]-acetamide [MH]⁺ 304.

### Intermediate 3

Using the general procedure for Intermediate 1, (4-nitro-benzyl)-urea [*J. Med. Chem*. 1996, 39, 1924] is converted into 6-amino-3-methyl-1-(4-nitro-benzyl)-1*H*-pyrimidine-2,4-dione, [MH]⁺ 277. A solution of calcium chloride (4.94g, 45 mmol) in water (100ml) is added to a solution of 6-amino-3-methyl-1-(4-nitro-benzyl)-1*H*-pyrimidine-2,4-dione (19.08g, 69.0mmol) in acetic acid (300ml). Zinc dust (58.8g, 900mmol) is then added portionwise with external cooling. The reaction is stirred at room temperature for 1.5 hours, filtered through a celite plug and washed successively with ethanol and acetic acid. Evaporation of the combined filtrate and washings affords 6-amino-1-(4-amino-benzyl)-3-methyl-1*H*-pyrimidine-2,4-dione acetic acid salt, [M-3H]⁻ 243. Acetic anhydride (7.2ml, 76.0mmol) is added to a cooled (0°C) suspension of 6-amino-1-(4-amino-benzyl)-3-methyl-1*H*-pyrimidine-2,4-dione acetic acid salt (17.0g, 69.0mmol) in pyridine (260ml). The reaction mixture is warmed to room temperature, stirred for 6 hours and the solvent evaporated. The residue is triturated with water and the solid collected by filtration and dried to afford N-[4-(6-amino-3-methyl-2,4-dioxo-3,4-dihydro-2*H*-pyrimidin-1-ylmethyl)-phenyl]-acetamide, [MH]⁺ 289. Using the general procedure for Intermediate 1, this compound is converted into N-[4-(5,6-diamino-3-methyl-2,4-dioxo-3,4-dihydro-2H-pyrimidin-1-ylmethyl)-phenyl]-acetamide, [MH]⁺ 304.

### Intermediate 4

To a cooled (0°C) slurry of 6-chloromethyluracil (2.0g, 12mmol) in THF/dioxan (1:1, 16ml) is added 2-pyridyldiphenylphosphine (3.60g, 13.7mmol) and cyclobutanemethanol (1.3ml, 13.8mmol), followed by di-t-butylazodicarboxylate (3.15g, 13.7mmol). The reaction is stirred overnight at ambient temperature, treated with 4M HCI in dioxan (15ml) and evaporated. The residue is taken up in dichloromethane, washed with 3.5M HCl, dried over magnesium sulfate and evaporated. The crude product is purified by flash chromatography (100:1 dichloromethane-methanol elution) to afford 6-chloro-1-cyclobutylmethyl-3-methyl-1*H*-pyrimidine-2,4-dione, ¹H NMR (400MHz, CDCl₃) δ 1.70-2.0 (m 6H), 2.60 (m 1H), 3.20 (s 3H), 4.00 (d *J* 7 2H), 5.78 (s 1H), which is dissolved in n-butanol (50ml). Veratrylamine (4ml, 26.5mmol) is added and the reaction heated to reflux for 16 hours.

The solvent is evaporated and the residue taken into dichloromethane, washed with 1M aqueous HCl, dried over magnesium sulfate and evaporated. The crude product is purified by flash chromatography (50:1 dichloromethane-methanol elution) to afford 1-cyclobutylmethyl-6-(3,4-dimethoxy-benzylamino)-3-methyl-1*H*-pyrimidine-2,4-dione, ¹H NMR (400MHz, CDCl₃) d 1.60-1.80 (m 4H), 1.80-2.00 (m 2H), 2.50 (m 1H), 3.21 (s 3H), 3.80 (s 6H), *3.85* (d *J* 7 2H), 4.11 (d *J* 5 1H), *4.25* (m 1H), 4.84 (s 1H), 6.74 (s 1H), 6.80 (s 2H), which is dissolved in formic acid (50ml) and Pd black (0.26g) added. The reaction is heated at 40°C for 21 hours, filtered through Celite, evaporated and purified by preparative HPLC to afford 6-amino-1-cyclobutylmethyl-3-methyl-1H-pyrimidine-2,4-dione, M⁺ 209, which is converted using the general procedure for Intermediate 1 into 5,6-diamino-1-cyclobutylmethyl-3-methyl-1*H*-pyrimidine-2,4-dione, HPLC retention time 0.17 mins (30-95% acetonitrile water gradient in 4 minutes).

### Intermediate 5

5,6-Diamino-3-methyl-1-(tetrahydro-furan-2-ylmethyl)-1H-pyrimidine-2,4-dione, mp 115-116°C, is prepared from (tetrahydro-furan-2-ylmethyl)-urea (Collect. Czech. Chem. Commun. 1972, 37, 2786) using the general procedure for Intermediate 1.

### Intermediate 6

5,6-Diamino-3-methyl-1-(2-methyl-butyl)-1H-pyrimidine-2,4-dione, mp 163-165°C, is prepared using the general procedure for Intermediate 1.

### Intermediate 7

5,6-Diamino-1-hexyl-3-methyl-1H-pyrimidine-2,4-dione, is prepared from 6-amino-1-hexyl-1*H*-pyrimidine-2,4-dione (J. Med. Chem. 1993, 36,1465) using the general procedure for Intermediate 1, HPLC retention time (0-95% acetonitrile water gradient over 8 minutes) 6.01 min.

### Intermediate 8

5,6-Diamino-1-(3,4-dimethoxy-benzyl)-3-methyl-lH-pyrimidine-2,4-dione, [M-H]⁻ 305, is prepared from (3,4-dimethoxy-benzyl)-urea (Farmaco, Ed. Sci. 1977, 32, 813) using the general procedure for Intermediate 1.

### Intermediate 9

5,6-Diamino-1-benzo[1,3]dioxol-5-ylmethyl-3-methyl-1H-pyrimidine-2,4-dione, mp 183-186°C, is prepared using the general procedure for Intermediate 1.

### Intermediate 10

5,6-Diamino-1-(2,4-dichloro-benzyl)-3-methyl-1H-pyrimidine-2,4-dione, is prepared using the general procedure for Intermediate 1, ¹H NMR (400MHz DMSO-d6) δ 3.16 (s 3H), 5.05 (s 2H), 6.18 (s 2H), 6.82 (d *J* 9 1H), 7.38 (d *J* 9 1H), 7.62 (s 1H).

Other Intermediates of formula V are prepared according to literature references as indicated below:

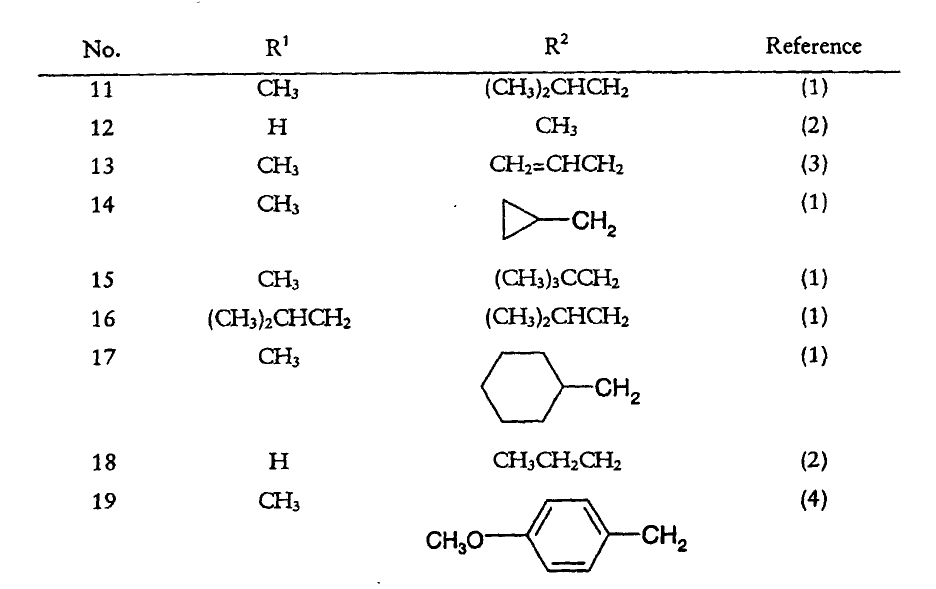

### References:

(1) Eur.J.Med.Chem. 1990, 25, 653
(2) J.Med.Chem. 1996, 39, 2
(3) FR 2 531 085
(4) Eur.J.Med.Chem.-Chim.Ther. 1974, 9, 313

Intermediates of formula VI are prepared as follows:

### Intermediate 20

A mixture of 3-(3,4-dimethoxy-phenyl)-5-nitro-pentanoic acid ethyl ester [J. Med. Chem, 1989, 32, 1450] (0.50g, 1.68mmol) and tin(II)chloride dihydrate (1.90g, 8.4mmol) in ethanol (10ml) is heated to reflux for 2 hours, cooled to ambient temperature and evaporated. The crude product is taken into dichloromethane (15ml), cooled to 0°C and triethylamine (5ml) added, followed by 3,5-dimethoxybenzoyl chloride (0.404g, 2.02mmol). The reaction is stirred at ambient temperature overnight and then evaporated, taken into ethyl acetate, washed with water and dried over sodium sulfate. Evaporation and purification by flash column chromatography (1:1 hexane-ethyl acetate elution) affords 4-(3,5-dimethoxy-benzoylamino)-3-(3,4-dimethoxy-phenyl)-butyric acid ethyl ester, [MH]⁺ 432. This intermediate (0.200g, 0.46mmol) is taken into acetonitrile (8ml) and phosphorus oxychloride (0.211g, 1.38mmol) added, prior to heating at reflux for 3 hours. After evaporation of the solvent, the residue is taken into ethyl acetate, washed with saturated aqueous sodium carbonate, dried over sodium sulfate and evaporated to afford [1-(3,5-dimethoxy-phenyl)-6,7-dimethoxy-3,4-dihydro-isoquinolin-4-yl]-acetic acid ethyl ester, [MH]⁺ 414. This intermediate (0.50g, 1.21mmol) is dissolved in decalin (10ml) and 10% Pd/C (50 mg) added. The reaction is heated at 190°C for 2.5 hours, then cooled to ambient temperature and diluted with dichloromethane. After filtration through Celite, the combined filtrate and washings are evaporated to afford [1-(3,5-dimethoxy-phenyl)-6,7-dimethoxy-isoquinolin-4-yl]-acetic acid ethyl ester, ¹H NMR (400MHz, CDCl₃) δ 1.18 (t *J* 7 3H), 3.78 (s 6H), 3.80 (s 3H), 3.90 (s 2H), 3.99 (s 3H), 4.10 (q *J* 7 2H), 6.50 (d *J* 0.5 1H), 6.75 (d *J* 0.5 2H), 7.20 (s 1H), 7.36 (s 1H), 8.40 (s 1H). This intermediate (0.30g, 0.73mmol) is dissolved in methanol (10ml), 1M aqueous lithium hydroxide (0.80ml, 0.80mmol) is added and the reaction stirred overnight at ambient temperature. After evaporation of the methanol, pH of the residual solution is adjusted to 7 with 1M aqueous HCl and the resultant solid collected by filtration and dried to afford [1-(3,5-dimethoxy-phenyl)-6,7-dimethoxy-isoquinolin-4-yl]-acetic acid.

### Intermediate 21

A mixture of 3-isopropoxy-4-methoxy-benzaldehyde (3.9g, 20mmol) and (ethoxycarbonylmethylene)triphenylphosphorane (6.96g. 20mmol) in toluene (100 ml) is heated at reflux for 2 hours, cooled to ambient temperature and evaporated. The crude product is taken in to dichloromethane and eluted through a pad of silica to afford (E)-3-(3-isopropoxy-4-methoxy-phenyl)-acrylic acid ethyl ester, TLC R_{f} 0.70 (1:1 hexane-ethyl acetate). This intermediate is dissolved in nitromethane (10ml), 1,1,3,3-tetramethylguanidine (0.5ml) is added and the reaction heated at 70°C for 36 hours. The solvent is evaporated, the residue is taken into ethyl acetate and washed with 2N aqueous HCl, water and brine. After drying over sodium sulfate and evaporation, the crude product is purified by flash column chromatography (4:1 hexane-ethyl acetate elution) to afford 3-(3-isopropoxy-4-methoxy-phenyl)-4-nitro-butyric acid ethyl ester, ¹H NMR (400MHz, CDCl₃) δ 1.20 (t *J* 7 3H), 1.38 (d *J* 7 6H), 2.75 (d *J* 6 2H), 3.90 (m 1H), 4.10 (m 2H), 4.48-4.78 (m 3H), 6.75-6.86 (m 3H). This intermediate is converted into [1-(3,5-diisopropoxy-phenyl)-6-isopropoxy-7-methoxy-isoquinolin-4-yl]-acetic acid using the general procedure for Intermediate 20. Characterised as the ethyl ester, [MH]⁺ 496.

### Intermediate 22

A solution of 3-ethoxy-4-methoxybenzaldehyde (3.6g, 20mmol) in ethanol (15ml) is added to 2,2-dimethoxyethylamine (21mmol) and the mixture heated at reflux for 2 hours. After cooling to room temperature, sodium borohydride (0.794g, 21mmol) is added and the mixture stirred at room temperature overnight. Ethanol is removed by evaporation and water added, followed by extraction with ethyl acetate. The organic extracts are combined, washed with water, brine, dried over magnesium sulfate and evaporated to give (2,2-dimethoxy-ethyl)-(3-ethoxy-4-methoxy-benzyl)-amine, [MH]⁺ 270. The intermediate (2.70g, 10mmol) is suspended in 6N HCl (50ml), glyoxylic acid (0.88g, 12mmol) is added and the mixture heated for 1 hour at 100°C. After cooling to room temperature, methanol (30ml) is added and the mixture filtered and characterised as the methyl ester M⁺ 276. The filtrate is treated with lithium hydroxide (10mmol) in THF-methanol-water overnight. After evaporation of the solvents, the crude product is partitioned between water and dichloromethane. The aqueous phase is washed with dichloromethane and evaporated to dryness to afford (7-ethoxy-6-methoxy-isoquinolin-4-yl)-acetic acid lithium salt which is used for xanthine formation without further characterisation.

### Intermediate 23

A solution of (6,7-dimethoxy-isoquinolin-4-yl)-acetic acid ethyl ester (Tetrahedron 1973, 29, 3881) (1.668g, 6.07mmol) in chloroform (20ml) is treated portionwise with m-chloroperoxybenzoic acid (1.153g, 6.67mmol) for 5 hours. The reaction mixture is washed with saturated sodium bicarbonate and brine, dried over MgSO₄ and evaporated to afford (6,7-dimethoxy-2-oxy-isoquinolin-4-yl)-acetic acid ethyl ester 1.71g, 96%). The entire product is dissolved in chloroform (30 ml), added to POCl₃ (3ml, 32.3mmol) and heated at reflux for 2 hours. After evaporation, dichloromethane and ice water are added and the mixture is basified with aqueous ammonia. The aqueous phase is further extracted with dichloromethane, the combined organic phases are washed with brine, dried over magnesium sulfate and evaporated to afford (1-chloro-6,7-dimethoxy-isoquinolin-4-yl)-acetic acid ethyl ester. The chloro ester derivative (0.50g, 1.6mmol) is suspended in 2M sodium hydroxide (15ml). Ethanol (5ml) is added and the solution stirred at room temperature for 2 hours and the solvent evaporated. Adjustment to pH 2 with concentrated hydrochloric acid affords a solid which is collected by filtration and dried to afford (1-chloro-6,7-dimethoxy-isoquinolin-4-yl)-acetic acid. ¹H NMR (400MHz, DMSO-d6) δ: 3.92 (s 3H), 3.96 (s 3H), 4.02 (s 2H), 7.31 (s 1H), 7.44 (s 1H), 8.04 (s 1H).

### Intermediate 24

(2,2-Dimethoxy-ethyl)-(3-methoxy-benzyl)-amine (Tetrahedron, 1973, 29, 3881) is treated with pyruvic acid according to the general procedure for Intermediate 22 to afford 2-(7-methoxy-isoquinolin-4-yl)-propionic acid hydrochloride salt, mp 174-176°C. Treatment with HCl gas in ethanol affords the corresponding ethyl ester hydrochloride, mp 190-192°C, which is then reacted sequentially with m-chloroperoxybenzoic acid and phosphorus oxychloride as described for Intermediate 23 to provide 2-(1-chloro-7-methoxy-isoquinolin-4-yl)-propionic acid ethyl ester, mp 126-128°C. This intermediate (47g, 0.16mol) is dissolved in ethanol (400ml) and 2N sodium hydroxide (150ml) added and the mixture heated at 60°C for one hour, prior to evaporation of the solvent. Crystallisation from acetone affords 2-(1-chloro-7-methoxy-isoquinolin-4-yl)-propionic acid, mp 167-168°C.

### Intermediate 25

Dimethyl sulfate (12.7ml, 0.10mol) is added portionwise to 2-(7-methoxy-2-oxy-isoquinolin-4-yl)-propionic acid ethyl ester (28g, 0.10mol) with an exotherm to 100°C. The reaction is maintained at this temperature for 2 hours, cooled to room temperature and dissolved in water (50ml). A solution of sodium cyanide (15g, 0.31mol) in water (90ml) is added over 30 minutes with external cooling and the reaction is then stirred at room temperature for 3 hours. The crude product is extracted with chloroform, the chloroform extracts are washed with saturated sodium bicarbonate, brine, dried over sodium sulfate and evaporated. Crystallisation from 3N ethanolic HCl-ether affords 2-(1-cyano-7-methoxy-isoquinolin-4-yl)-propionic acid ethyl ester hydrochloride, mp 89-98°C. This compound is hydrolysed converted to the acid as described for Intermediate 22 and used directly crude for xanthine formation.

### Intermediate 26

A solution of sodium triethylborohydride (1M THF, 12.7ml, 12.7mmol) is added dropwise to a solution of isoquinoline (1.64g, 12.7 mmol) in THF (25ml). The reaction is stirred at room temperature for 1 hour, prior to dropwise addition of a solution of ethyl glyoxalate (1.43g, 13.9mmol) in toluene (previously heated at 110°C for 1.5 hours). After a further 4 hours at room temperature, the reaction is cooled to 0°C and sodium hydroxide (0.5M aqueous solution, 25.4ml) followed by hydrogen peroxide (30% aqueous solution, 12.7ml) is added, followed by stirring for 2 hours. The reaction is acidified with 1N HCl, washed with ethyl acetate three times, the aqueous phase is reduced in volume by evaporation and refrigerated overnight. The resultant precipitate is collected by filtration and dried to afford isoquinolin-4-yl-acetic acid hydrochloride salt, MH⁺ 188.

### Intermediate 27

Excess morpholine is added to a suspension of (1-chloro-6,7-dimethoxy-isoquinolin-4-yl)-acetic acid ethyl ester (0.200g, 0.65 mmol) in toluene (1ml) and the mixture heated to reflux until the starting material is consumed. After evaporation, the residue is partitioned between water and dichloromethane, the organic phase is dried over magnesium sulfate and evaporated. to afford (6,7-dimethoxy-1-morpholin-4-yl-isoquinolin-4-yl)-acetic acid ethyl ester [MH]⁺ 361. The crude ester (0.240g, 0.66mmol) is dissolved in ethanol (20ml), treated with 2M sodium hydroxide (3ml) and stirred at room temperature overnight. After adjustment to pH 1 with concentrated hydrochloric acid, the solvent is evaporated and the crude acid used directly for formation of the xanthine derivative.

### Intermediate 28

The procedure for Intermediate 27 is repeated, using excess N-methylpiperazine in place of morpholine to afford [6,7-dimethoxy-1-(4-methyl-piperazin-1-yl)-isoquinolin-4-yl]-acetic acid ethyl ester (0.186g, 38%) ¹H NMR (DMSO-d6) δ 1.19 (t *J* 7 3H), 2.30 (s 3H), 2.61 (m 4H), 3.10-3.30 (m 4H), 3.92 (s 6H), 3.97 (s 2H), 4.10 (q *J* 7 2H), 7.19 (s 1H), 7.37 (s 1H), 7.91 (s 1H). The ester (0.186g, 0.50mmol) is dissolved in ethanol (20ml), treated with 2M sodium hydroxide (3ml) and stirred at room temperature overnight. After adjustment to pH 1 with concentrated hydrochloric acid, the solvent is evaporated and the crude acid used directly for formation of the xanthine derivative.

### Intermediate 29

N-Chlorosuccinimide (0.347g, 2.60mmol) is added to a solution of 6-methoxyisoquinoline (Synth. Commun. 1999, 29, 1617) (0.207g, 1.30mmol) in acetic acid (9ml). The reaction is heated at 50°C for 3 hours, cooled to ambient temperature, evaporated and partitioned between ethyl acetate and 1M aqueous sodium hydroxide. The organic phase is washed with water and brine, dried over magnesium sulfate and evaporated afford 5-chloro-6-methoxyisoquinoline, [MH]⁺ 194. A solution of this intermediate (0.175g, 0.90mmol) in THF (4.5ml) and acetic anhydride (0.101ml, 1.08mmol) is treated with sodium triacetoxyborohydride (0.229g, 1.08mmol) and the reaction is stirred at ambient temperature for 22 hours. The solvent is evaporated, the residue is taken into ethyl acetate, washed with 0.5M aqueous hydrochloric acid, then brine and dried over magnesium sulfate. Evaporation affords 1-(5-chloro-6-methoxy-1*H*-isoquinolin-2-yl)-ethanone, mp 78-80°C. A suspension of this intermediate (0.150g, 0.60mmol) and glyoxylic acid (76mg, 0.80mmol) in 6M aqueous hydrochloric acid (2.8ml) is heated at 100°C for 3 hours. After cooling to ambient temperature, the resultant solid is collected by filtration to afford (5-chloro-6-methoxy-isoquinolin-4-yl)-acetic acid, [MH]⁺ 252. A suspension of this intermediate (0.970g, 3.38mmol) and ammonium formate (1.05g, 16.9mmol) in 1:1 acetic acid-water (25ml) is treated with 10% Pd/C (0.730g) and stirred at ambient temperature for 16 hours. After filtration through Celite®, the combined filtrate and washings are evaporated and purified by Soxhlet extraction with acetone to afford (6-methoxy-isoquinolin-4-yl)-acetic acid [MH]⁺ 218. Alternatively reduction of (5-chloro-6-methoxy-isoquinolin-4-yl)-acetic acid to afford (6-methoxy-isoquinolin-4-yl)-acetic acid is accomplished by stirring a suspension of (5-chloro-6-methoxy-isoquinolin-4-yl)-acetic acid (20g, 69.4mmol) in 1 Molar sodium hydroxide solution (400mL) for 20 min, filtering off the resultant salt and then treating with hydgrogen gas in the presence of 10% Pd/C (1.4g) at atmospheric pressure for 2.25h. The resulting suspension is filtered through glass wool and celite, washing with water (50mL). The solution is then cooled in an ice water bath and slowly (30 min) neutralised and then acidified with 5 Molar hydrochloric acid (80ml). A suspension forms and further crystalisation is encouraged by standing at 5°C for 20h. The resulting crystals are removed by filtration and washed with ice cold ethanol (25ml) drying under reduced pressure gives (6-methoxy-isoquinolin-4-yl)-acetic acid.

### Intermediate 30

Glyoxylic acid (1.37g, 9.28mmol) is added to mixture of 1-(6-chloro-1*H*-isoquinolin-2-yl)-ethanone [J. Org. Chem., 1980, 45, 1950] (1.44g, 5.90mmol) in 6N HCl (24ml). The reaction is heated at 100°C for 3 hours, cooled to RT, washed with ether and evaporated to 10ml volume. After overnight refrigeration, the solid is collected by filtration and dried to afford (6-chloro-isoquinolin-4-yl)-acetic acid hydrochloride. ¹H NMR (400 MHz, DMSO) δ: 4.45 (s 2H), 8.18 (d *J* 9 1H), 8.52 (s 1H), 8.70 (d *J* 8 1H), 8.83 (s1H), 9.96 (s 1H).

### Intermediate 31

Sodium borohydride (1.12g, 29.6mmol) is added portionwise to a cooled (0°C) solution of 6-bromoisoquinoline [J Chem Soc Perkin Trans 2, 1998, 437] (1.544g, 7.42mmol) in acetic acid (10ml) and acetic anhydride (3ml). After heating at 60°C for 4 hours, the mixture is cooled, evaporated and diluted with water. After adjustment to pH10 with potassium carbonate and extraction with ethyl acetate, the combined organic phases are washed twice with 0.5N HCl and brine, then dried over sodium sulphate. Evaporation affords 1-(6-bromo-1*H*-isoqiunolin-2-yl)-ethanone, MH⁺ 253. Glyoxylic acid (0.812g, 8.80mmol) is added to mixture of 1-(6-bromo-1*H*-isoquinolin-2-yl)-ethanone (1.50g, 5.90mmol) in 6N HCl (20ml). The reaction is heated at 100°C for 2 hours, cooled to RT and washed with ethyl acetate. After evaporation, the residue is taken into methanol (20ml), concentrated sulphuric acid (10 drops) is added and the mixture heated at reflux for 14 hours. After partial evaporation of the solvent, the resultant solid is collected by filtration, washed with methanol and dried to afford (6-bromo-isoquinolin-4-yl)-acetic acid methyl ester hydrochloride, MH⁺ 281. Lithium hydroxide hydrate (8.5mg, 0.20mmol) is added to a cooled (0°C) solution of (6-bromo-isoquinolin-4-yl)-acetic acid methyl ester (50mg, 0.18mmol) in 3:1 THF-water (3ml). After 1 hour the solvent is evaporated to afford (6-bromo-isoquinolin-4-yl)-acetic acid lithium salt, MH⁺ 266.

### Intermediate 32

Trimethylsilylacetylene (0.17ml, 1.23 mmol) is added to a suspension of (6-bromo-isoquinolin-4-yl)-acetic acid methyl ester (0.325g, 1.03mmol) in DMF (1.75ml) and triethylamine (10ml), follwed by copper(I) iodide (40mg, 0.20mmol) and (Ph₃P)₂PdCl₂ (73mg, 0.10mmol). The reaction is heated at 45°C for 40 minutes, cooled to ambient temperature and diluted with ethyl acetate. After washing with water and brine, the organic phase is dried over magnesium sulfate, evaporated and purified by flash column chromatography (1:1 ethyl acetate-hexane elution) to afford (6-trimethylsilanylethynyl-isoquinolin-4-yl)-acetic acid methyl ester, [MH]⁺ 298. This intermediate (0.221g, 0.74mmol) is dissolved in methanol (7.5ml) and treated with potassium carbonate (75mg, 0.54mmol). The reaction is stirred for 30 minutes at ambient temperature, evaporated and purified by flash chromatography (5:1 dichloromethane-methanol elution) to afford (6-ethynyl-isoquinolin-4-yl)-acetic acid, [MH]⁺ 212.

### Intermediate 33

Bromine (0.211ml, 6.28mmol) in dichloromethane (10ml) is added to a cooled (0°C) solution of 6-methoxyisoquinoline [Synth. Commun. 1999, 29, 1617] and the reaction is stirred at ambient temperature for 20 hours. After pouring into 1M aqueous sodium hydroxide, the organic phase is washed with brine, dried over magnesium sulfate and evaporated. The crude product is purified by flash column chromatography (20:1 dichloromethane-methanol elution) to afford 5-bromo-6-methoxyisoquinoline, [MH]⁺ 240. This material is then converted according to the procedure for Intermediate 29 into (5-bromo-6-methoxy-isoquinolin-4-yl)-acetic acid [MH]⁺ 298.

### Intermediate 34

[1-(3,5-Diisopropoxy-phenyl)-6,7-dimethoxy-isoquinolin-4-yl]-acetic acid is prepared using the general procedure for Intermediate 20, ¹H NMR (400MHz CDCl₃) δ 1.25 (d *J* 6 12H), 3.78 (s 3H), 3.86 (s 2H), 3.92 (s 3H), 6.46 (d *J* 0.5 1H), 6.65 (d *J* 0.5 2H), 7.20 (s 2H), 8.30 (s 1H).

The following are prepared analogously to Intermediate 21:

Intermediate 35: 1-(3,5-Dimethoxy-phenyl)-6-isopropoxy-7-methoxy-isoquinolin-4-yl]-acetic acid, [MH]⁺ 412.

Intermediate 36: (1-.tert.-Butyl-6-isopropoxy-7-methoxy-isoquinolin-4-yl)-acetic acid, ¹H NMR (400MHz, CDCl₃) δ 1.32 (d J 7 6H), 1.52 (s 9H), 3.80 (s 2H), 3.90 (s 3H), 4.75 (heptet *J* 7 1H), 7.28 (s 1H), 7.66 (s 1H), 8.08 (s 1H).

Intermediate 37: (6-Isopropoxy-1-isopropyl-7-methoxy-isoquinolin-4-yl)-acetic acid, [MH]⁺ 318.

The following are prepared analogously to Intermediate 20:

Intermediate 38: (6,7-Dimethoxy-1-methyl-isoquinolin-4-yl)-acetic acid, [MH]⁺ 262.

Intermediate 39: (1-tert.-Butyl-6,7-dimethoxy-isoquinolin-4-yl)-acetic acid, ¹H NMR (400MHz, CDCl₃) d 1.75 (s 9H), 3.95 (s 6H), 4.04 (s 2H), 7.28 (s 1H), 7.75 (s 1H), 8.66 (s 1H).

Intermediate 40: (1-Isopropyl-6,7-dimethoxy-isoquinolin-4-yl)-acetic acid, characterised as the ethyl ester ¹H NMR (400MHz, CDCl₃) δ 1.25 (t *J* 7 3H), 1.45 (d *J* 7 3H), 3.82 (heptet *J* 7 1H), 3.90 (s 2H), 3.08 (s 2H), 4.15 (q *J 7 2H)*, 7.28 (s 1H), 7.48 (s 1H), 8.30 (s 1H).

Intermediate 41: 2-(7-Methoxy-1-morpholin-4-yl-isoquinolin-4-yl)-propionic acid mp 225-227°C, is prepared according to the procedure for Intermediate 27.

The following are prepared analogously to Intermediate 22:

Intermediate 42: [MH]⁺ 332 (7-Hydroxy-6-methoxy-isoquinolin-4-yl)-acetic acid lithium salt, via (3-benzyloxy-4-methoxy-benzyl)-(2,2-dimethoxy-ethyl)-amine.

Intermediate 43: (6,7-Dimethoxy-3-methyl-isoquinolin-4-yl)-acetic acid, ¹H NMR (400MHz, DMSO) δ: 2.50 (s 3H), 3.91 (s 3H), 3.93 (s 3H), 4.02 (s 2H), 7.30 (s 1H), 7.43 (s 1H), 8.30 (s 1H).

Intermediate 44: (6-Ethoxy-7-methoxy-isoquinolin-4-yl)-acetic acid, 3 M⁺ 261.

The following are prepared analogously to Intermediate 22, using pyruvic acid in place of glyoxylic acid:

Intermediate 45: 2-(6-Ethoxy-7-methoxy-isoquinolin-4-yl)-propionic acid lithium salt, characterised as the methyl ester, M⁺ 290.

Intermediate 46: 2-(7-Ethoxy-6-methoxy-isoquinolin-4-yl)-propionic acid lithium salt, characterised as the methyl ester, M⁺ 290.

Intermediate 47: 2-(6,7-dimethoxy-isoquinolin-4-yl)-propionic acid, characterised as the methyl ester, M⁺ 276.

Intermediate 48: 8-Fluoro-6-methoxy-isoquinolin-4-yl)-acetic acid, is prepared according to the procedure for Intermediate 31 and characterised as the methyl ester, [MH]⁺ 250.

Intermediate 49: (6,7-dimethoxy-isoquinolin-4-yl)-acetic acid, and Intermediate 50, [1,3]dioxolo[4,5-.g.]isoquinolin-8-yl-acetic acid, are prepared as described in Dyke et al, Tetrahedron 1968, 24, 1467.

Intermediate 51: (7-methoxy-isoquinolin-4-yl)-acetic acid is prepared according to Dyke et al, Tetrahedron, 1973, 29, 3881.

Intermediate 52: 2,2-Dimethoxyethylamine (13.85ml, 0.13mol) is added to a solution of 3-fluoro-4-methoxybenzaldehyde (20g, 0.13mol) in toluene (200ml). The resulting solution is flushed with nitrogen gas and then heated overnight under reflux in a Dean-Stark apparatus. The solvent is then removed under reduced pressure to yield (2,2-dimethoxy-ethyl)-[1-(3-fluoro-4-methoxy-phenyl)-methylidene]-amine. This intermediate (31g, 0.13mol) is dissolved in ethylacetate and acetic anhydride ( 13.1g, 0.13mol) added. Platinum oxide (0.3g) is then added, under a blanket of nitrogen, and the resulting mixture is stirred under a hydrogen atmosphere until uptake is complete. Filtration, washing with saturated aqueous NaHCO₃ (3x 100ml) , brine and water, drying over MgSO₄ and concentration then gives N-(2,2-dimethoxy-ethyl)-N-(3-fluoro-4-methoxy-benzyl)-acetamide. This intermediate (38.9g, ca 0.13mol) is dissolved in anhydrous CH₂Cl₂ and then added slowly over 20mins to a stirred mixture of AlCl₃ (90g) and CH₂Cl₂ under an atmosphere of nitrogen. The total volume of CH₂Cl₂ is 250ml. The mixture is stirred for a further 10mins at room temperature and is then cooled with an ice bath during the addition of aqueous 40% NaOH. The mixture is further diluted with water (250ml), filtered through glass wool, the organic phase separated and the aqueous phase further extracted with CH₂Cl₂ (2x 200ml). Drying over MgSO₄ and evaporation under reduced pressure yields a crude oil which is purified by flash silica chromatography (eluant: 1% methanol in CH₂Cl₂) to give as one of the products 1-(7-fluoro-6-methoxy-1H-isoquinolin-2-yl)-ethanone. This intermediate (0.60g, 2.7mmol) is mixed with glyoxylic acid (0.325g, 3.5mmol) and water (10ml) and the resulting mixture is stirred at room temperature for 20min. Concentrated hydrochloric acid (10ml) is then added and the mixture heated to reflux for 1h. Concentration and purification by preparative HPLC gives (7-fluoro-6-methoxy-isoquinolin-4-yll)-acetic acid, [MH]⁺ 236.

The following are prepared analogously to Intermediate 20 :

Intermediate 53: (1-Methyl-6-methoxy-isoquinolin-4-yl)-acetic acid.

Intermediate 54: (6-Isopropoxy-1-methyl-isoquinolin-4-yl)-acetic acid.

Intermediate 55: (6-Ethoxy-1-methyl-isoquinolin-4-yl)-acetic acid.

Intermediate 56: A solution of (6-bromo-isoquinolin-4-yl)-acetic acid methyl ester (52mg, 0.19mmol), prepared as described as an intermediate for Intermediate 31, in DMF (3ml) is added to zinc dicyanide (26mg, 0.22mmol) under a nitrogen atmosphere. To the resulting mixture is added 1,1'-bis(diphenylphosphino)ferrocene (15mg) and tris(dibenzylideneacetone)dipalladium(0) (8mg) and the resulting mixture stirred at 120°C for 22h. The solution is cooled and diluted with chloroform (30ml) and washed with water (2 x 20ml) followed by brine (20ml). Further chlorofrom is added (40ml) and the solution dried over MgSO₄, filtered and concentrated. Repetitive flash silica column chromatography (eluants 40:1 CH₂Cl₂:methanol, then 50:1 CH₂Cl₂:methanol) gives (6-cyano-isoquinolin-4-yl)-acetic acid methyl ester [MH]⁺ 227. This intermediate is saponified by treatment with LiOH in 3:1 THF/water. The resulting mixture is partially evaporated to remove the THF, diluted to 10ml with water then washed with ethyl acetate. The aqeous phase is then neutralised with 1M hydrochloric acid (to pH 4-5) and exhaustively extracted with ethylacetate. The organic phase is dried over MgSO₄, filtered and concentrated to give (6-cyano-isoquinolin-4-yl)-acetic acid M⁺ 212.

Intermediate 57: (5-Chloro-6-methoxy-isoquinolin-4-yl)-acetic acid is prepared as described in the procedure for intermediate 29.

Intermediate 58: To a solution of (6-trimethylsilanylethynyl-isoquinolin-4-yl)-acetic acid methyl ester, as prepared for Intermediate 32 (0.19g, 0.64mmol), in anhydrous methanol (7ml) is added K₂CO₃ (72mg) and the resulting mixture stirred for 1h. Additional K₂CO₃ (11mg) is then added and stirring continued for 30min. The mixture is then neutralised with glacial acetic acid and concentrated. Purification by flash silica column chromatography (ethylacetate/hexane 1:1) gives (6-ethynyl-isoquinolin-4-yl)-acetic acid methyl ester M⁺ 225. This intermediate (79mg, 0.35mmol) is dissolved in methanol under an inert atmosphere and 10% Pd on carbon (79mg) added. The resulting suspension is stirred vigorously under an atmosphere of gaseous hydrogen. After 90mins, filtration, washing with methanol and concentration give (6-ethyl-isoquinolin-4-yl)-acetic acid methyl ester M⁺ 229. To a solution of this intermediate (68mg, 0.30mmol) in THF/methanol/water (3:1:1, 3.5ml) is added LiOH (12.5mg) and the mixture stirred for 20h at room temperature. Concentration under reduced pressure gives lithium (6-ethyl-isoquinolin-4-yl)-acetate M⁺ 221.

Intermediate 59: A solution of Intermediate 29 (0.5g, 2.3mmol) is suspended in aqueous 48% HBr (10ml) and then heated at 100°C for 48h. Further aqueous 48% HBr (10ml) is then added and heating continued at 100°C for an additional 24h. The reaction mixture is cooled to 5°C for 4h and the resulting solid seperated by filtration. Washing with water and drying under high vacumn at 50°C gives (6-hydroxy-isoquinolin-4-yl)-acetic acid hydrobromide [MH]⁺ 204.4. This intermediate (0.15g, 0.53mmol) is suspended in DMF (2ml) and K₂CO₃ (0.22g, 1.58mmol) added followed by ethyliodide (0.085ml, 1.06mmol) and the resulting mixture stirred at room temperature for 2h. Concentration and purification by flash silica column chromatography (eluant: CH₂Cl₂/methanol 10:1) gives (6-ethoxy-isoquinolin-4-yl)-acetic acid ethyl ester [MH]⁺ 260. This intermediate (25mg, 0.11mmol) is dissolved in water (1ml) and LiOH added (5mg, 0.11mmol). The resulting mixture is stirred for 30min at room temperature. Acidification with minimumn 6N HCl and concentration gives crude (6-ethoxy-isoquinolin-4-yl)-acetic acid.

### Examples 1- 70

Compounds of formula I which are also of formula 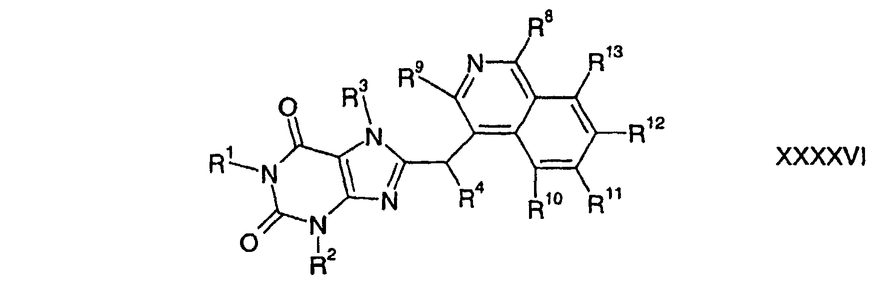 where R¹ to R⁴ and R⁸ to R¹³ are as hereinbefore defined, in free or salt form, and their methods of preparation are shown in the following table, the methods being described hereinafter. R³ is H in all Examples except No 44, where it is CH₃. R⁴ is H in all examples except Nos 25 - 27 and 41 - 43, where it is CH₃. R⁹ is H in all Examples except No 29, where it is CH₃. R¹⁰ is H in all Examples except No 57, where it is Br and No 75 where it is Cl. R¹³ is H in all Examples except Nos 56 where it is F, and 65 and 66, where it is Br.

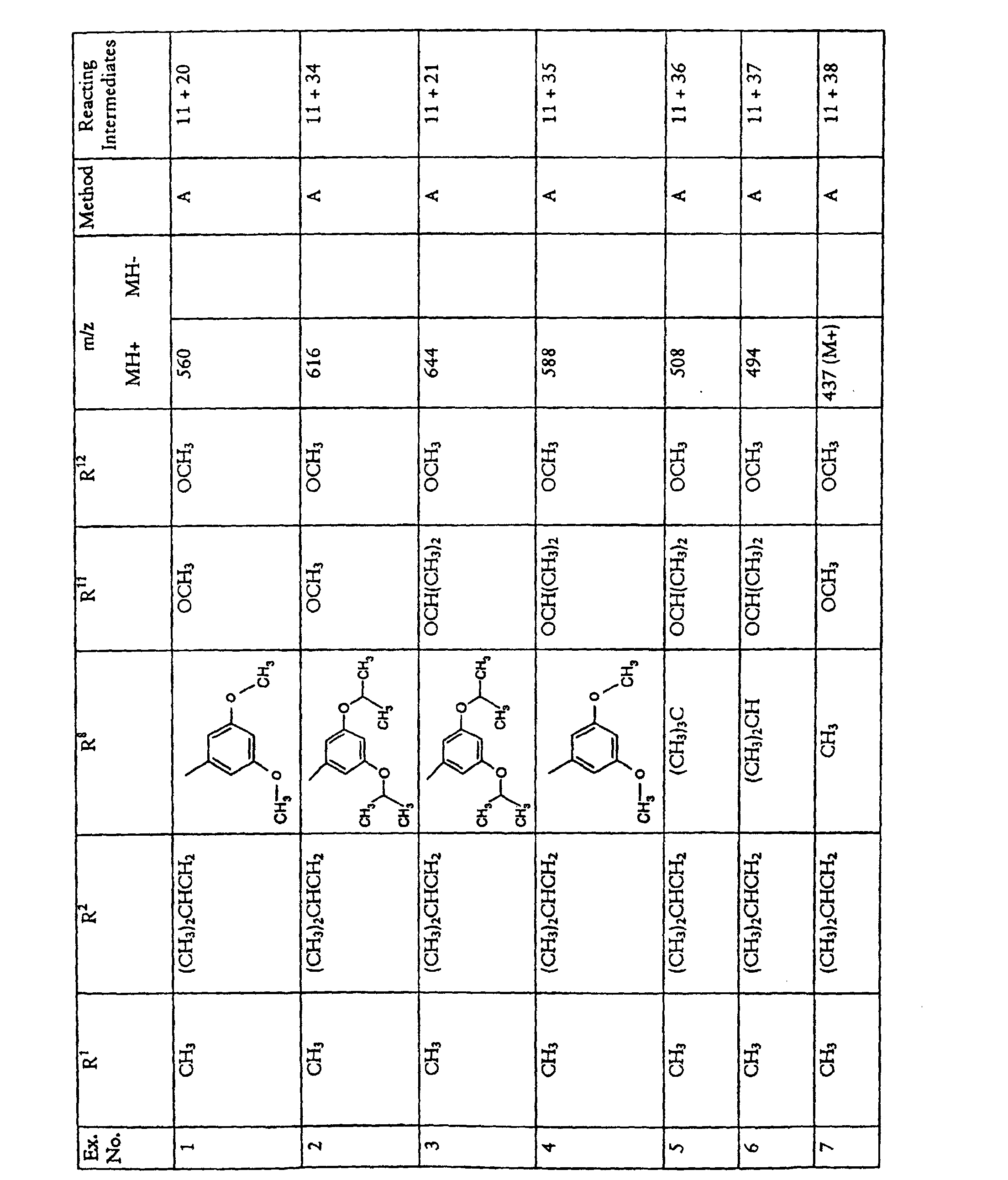

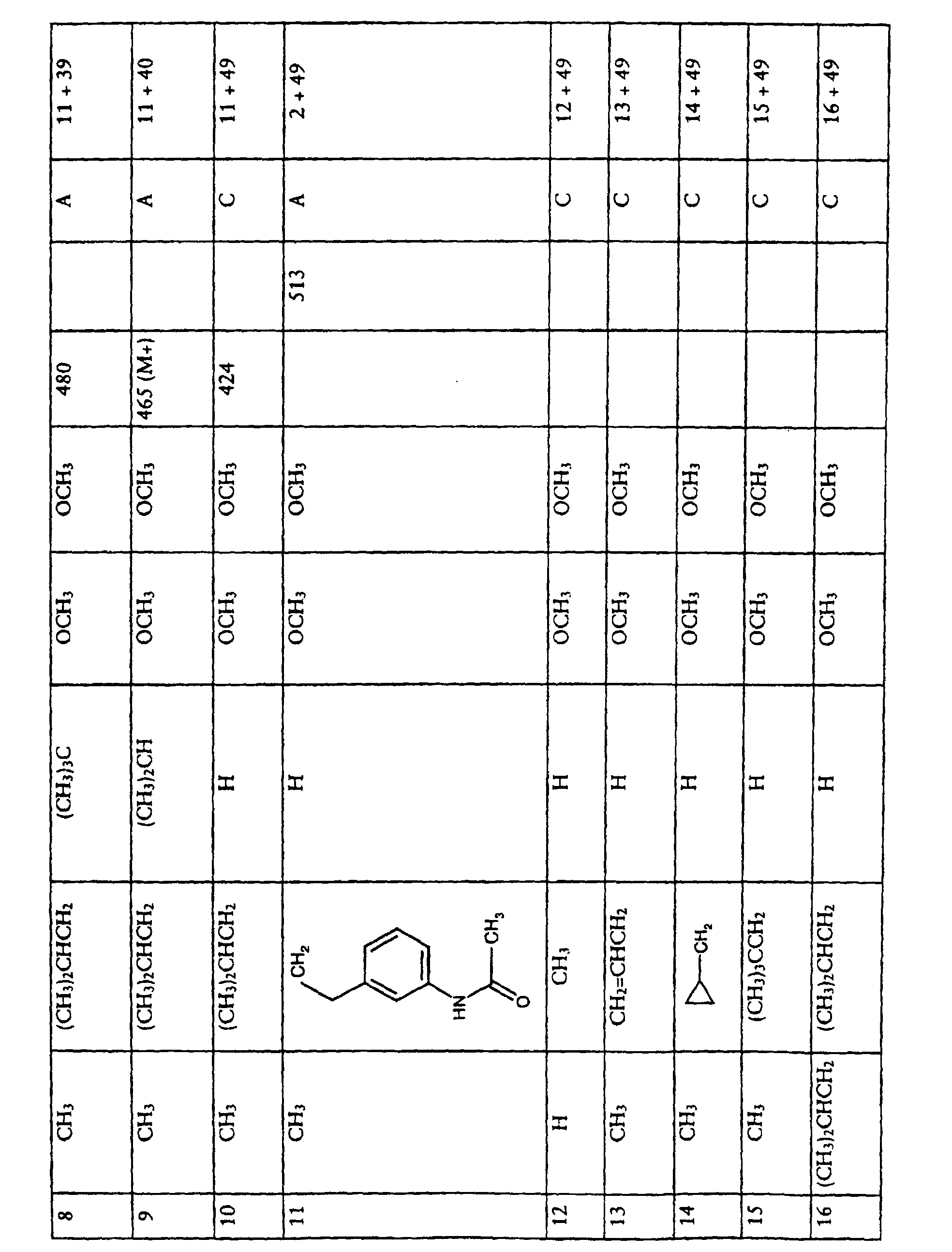

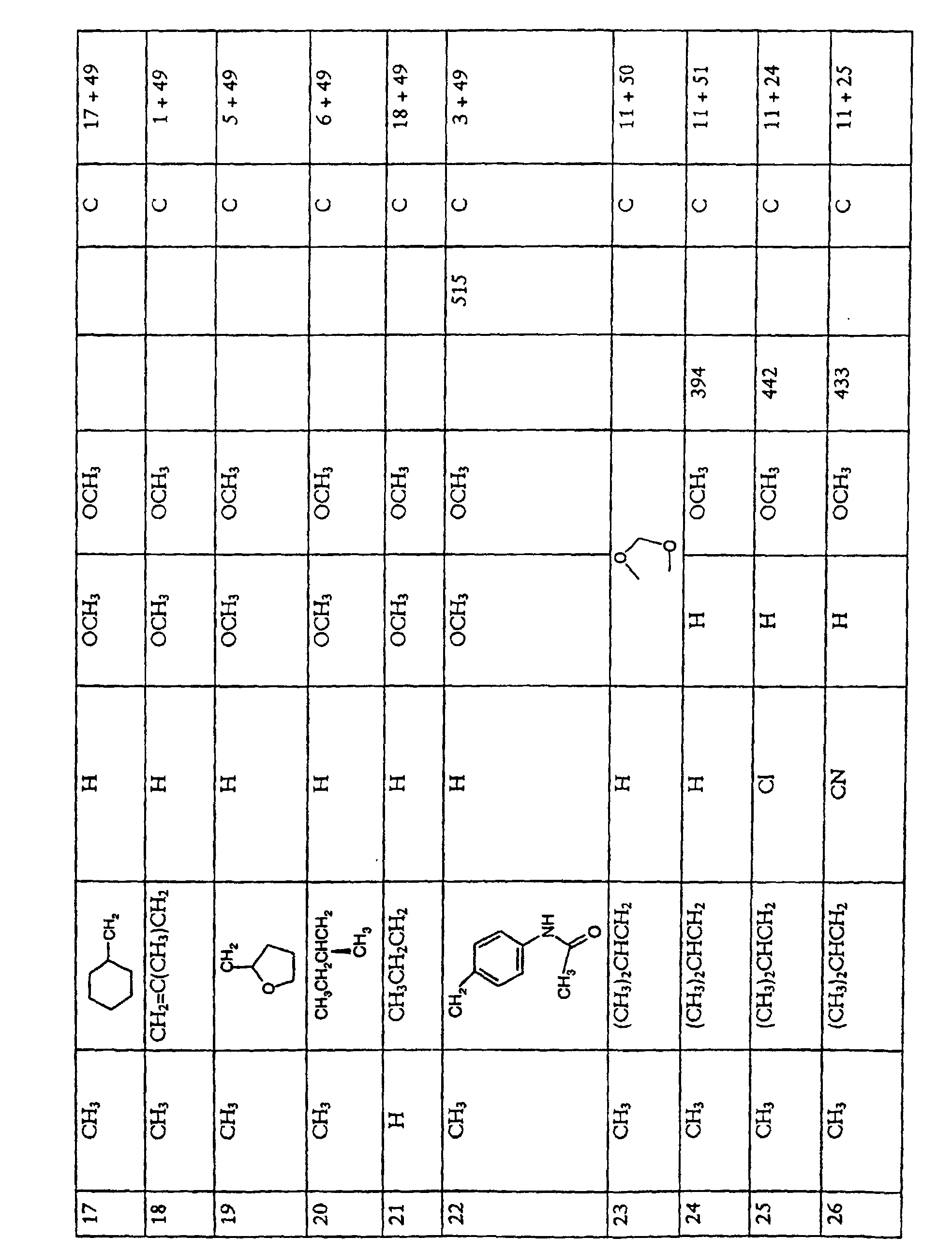

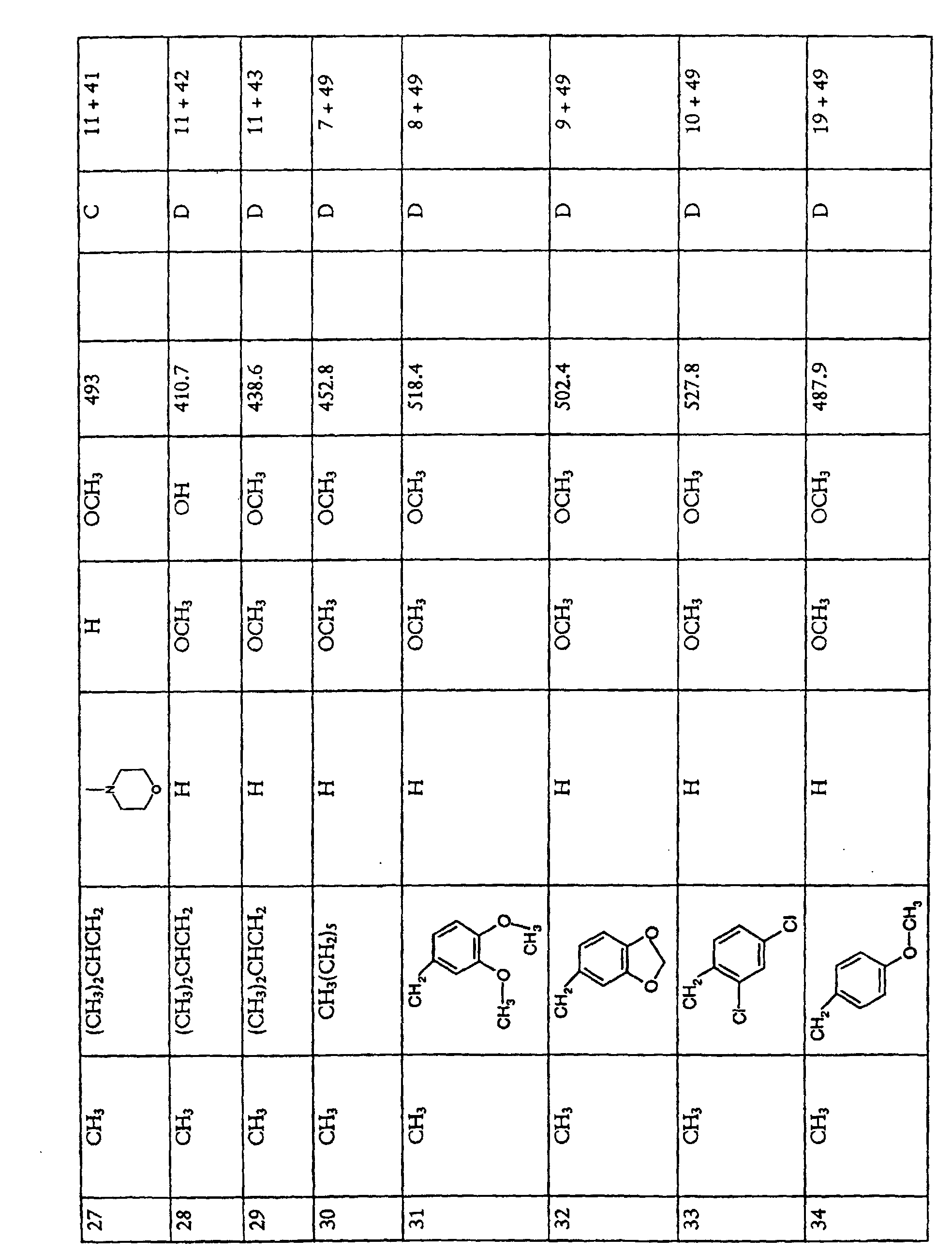

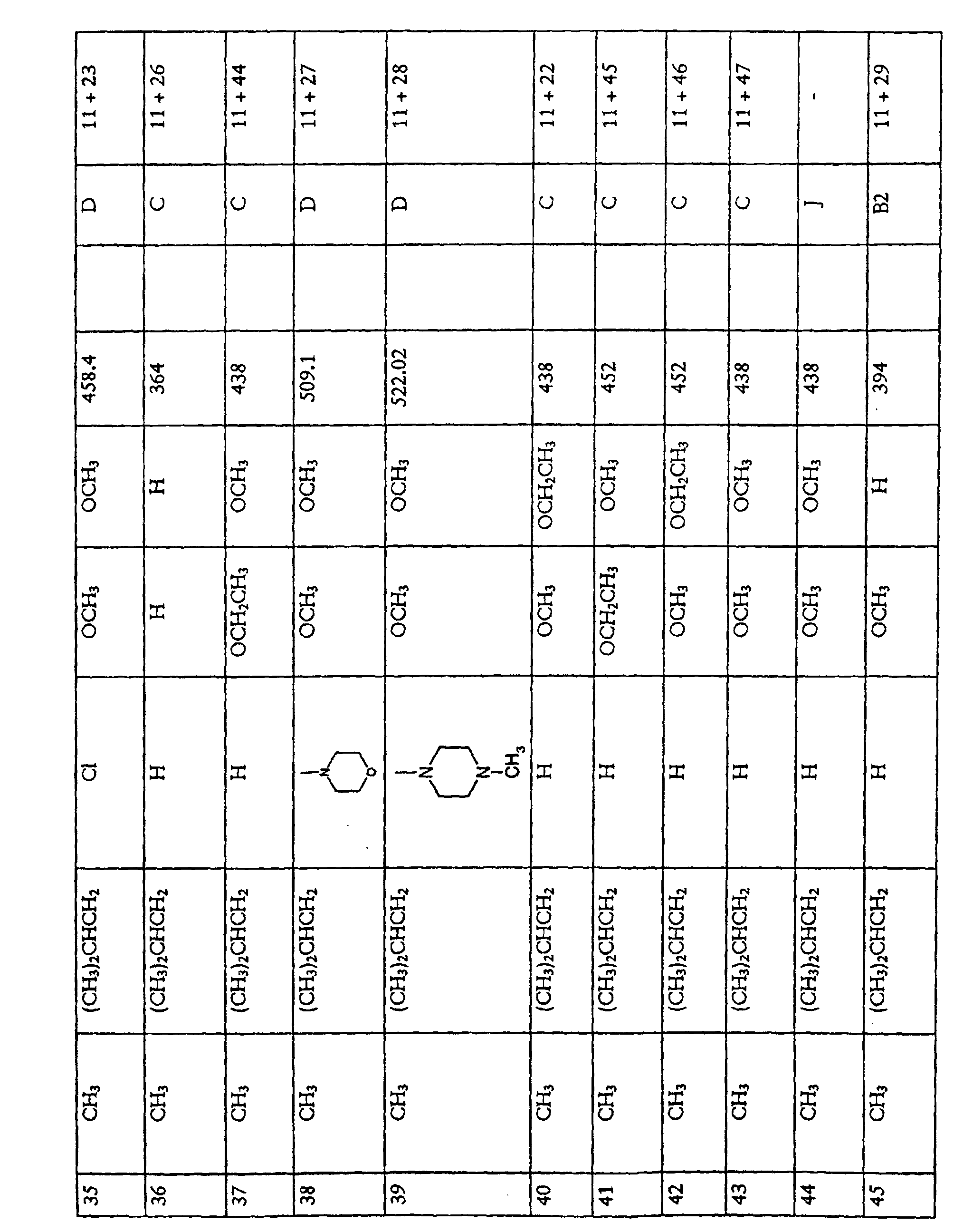

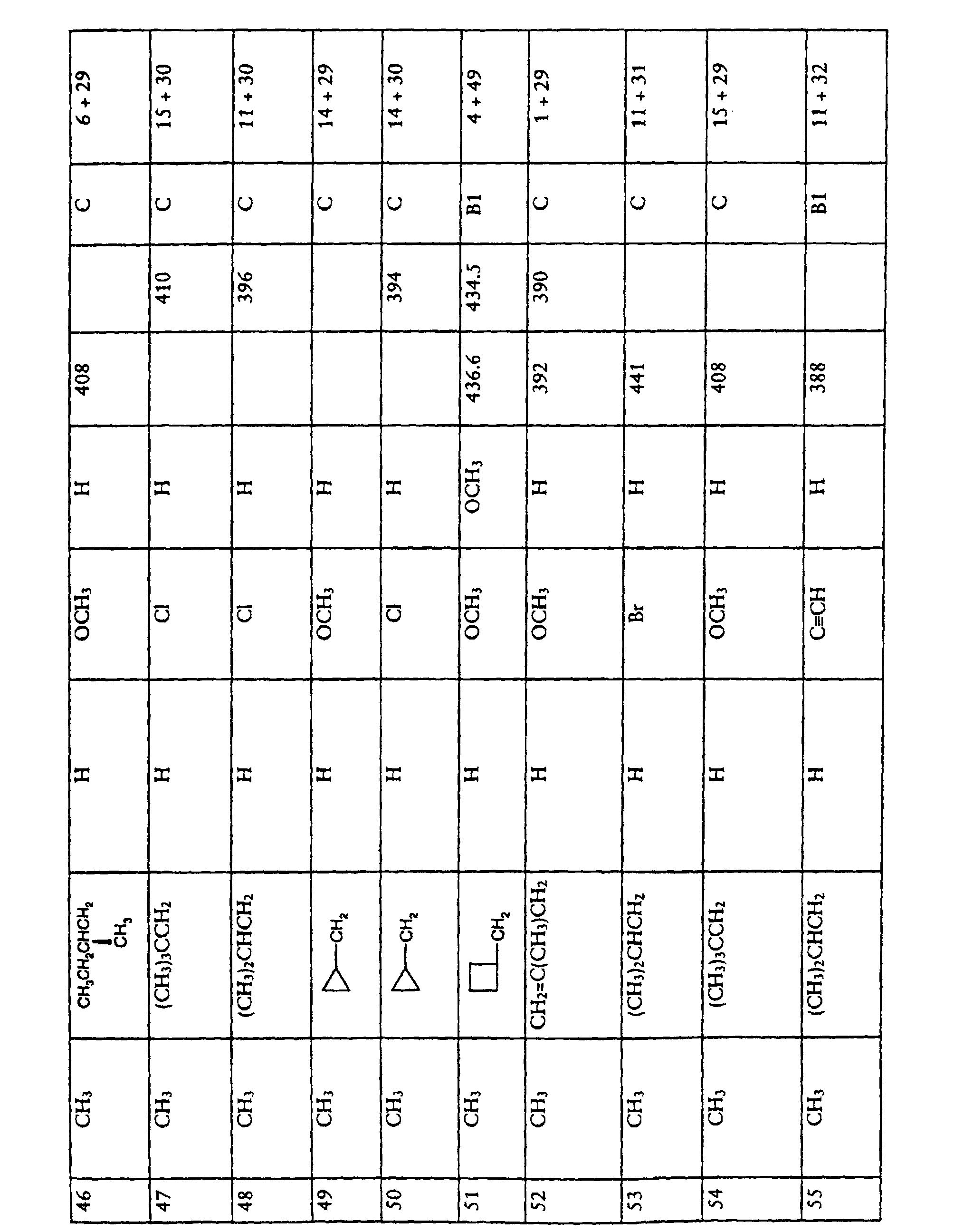

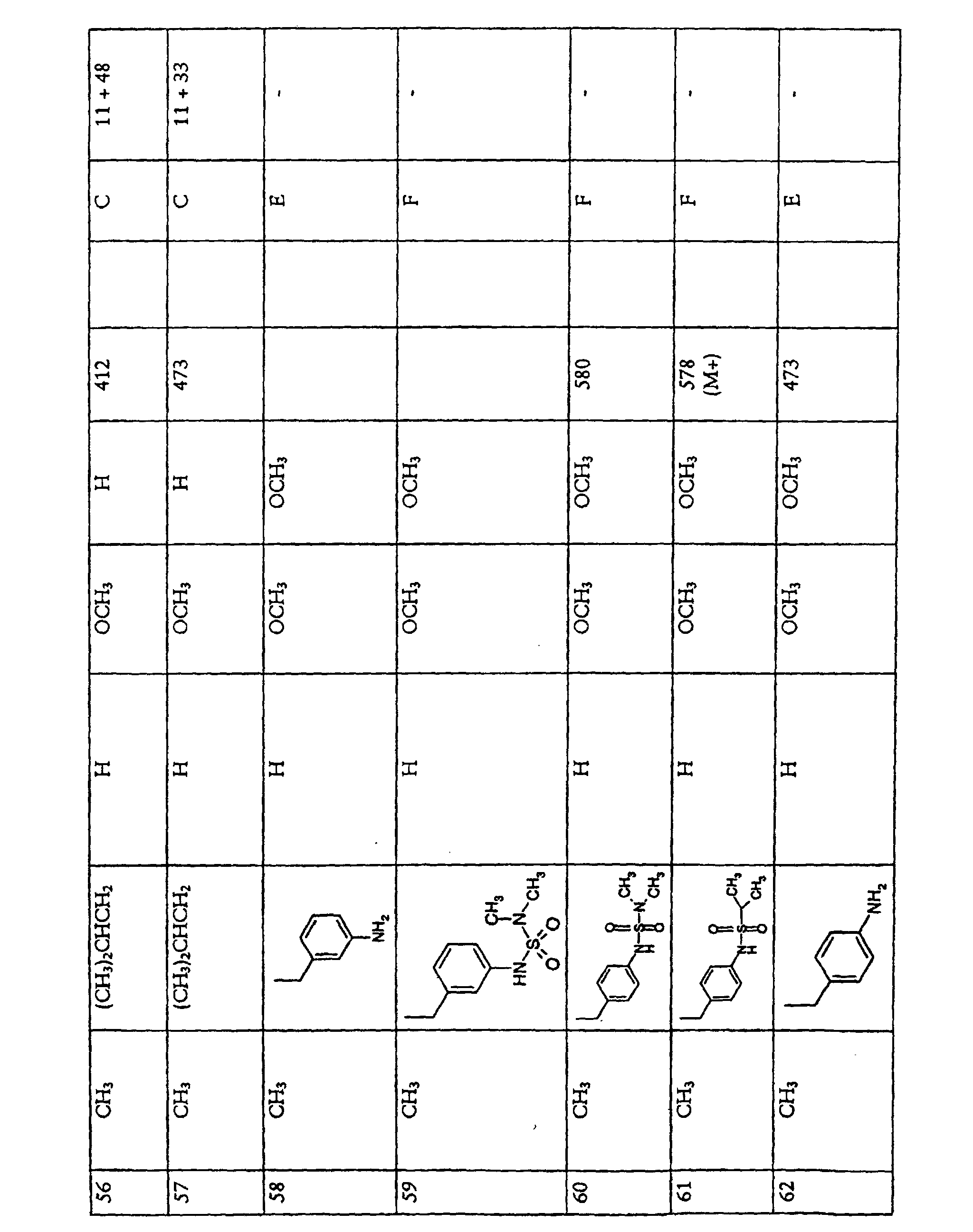

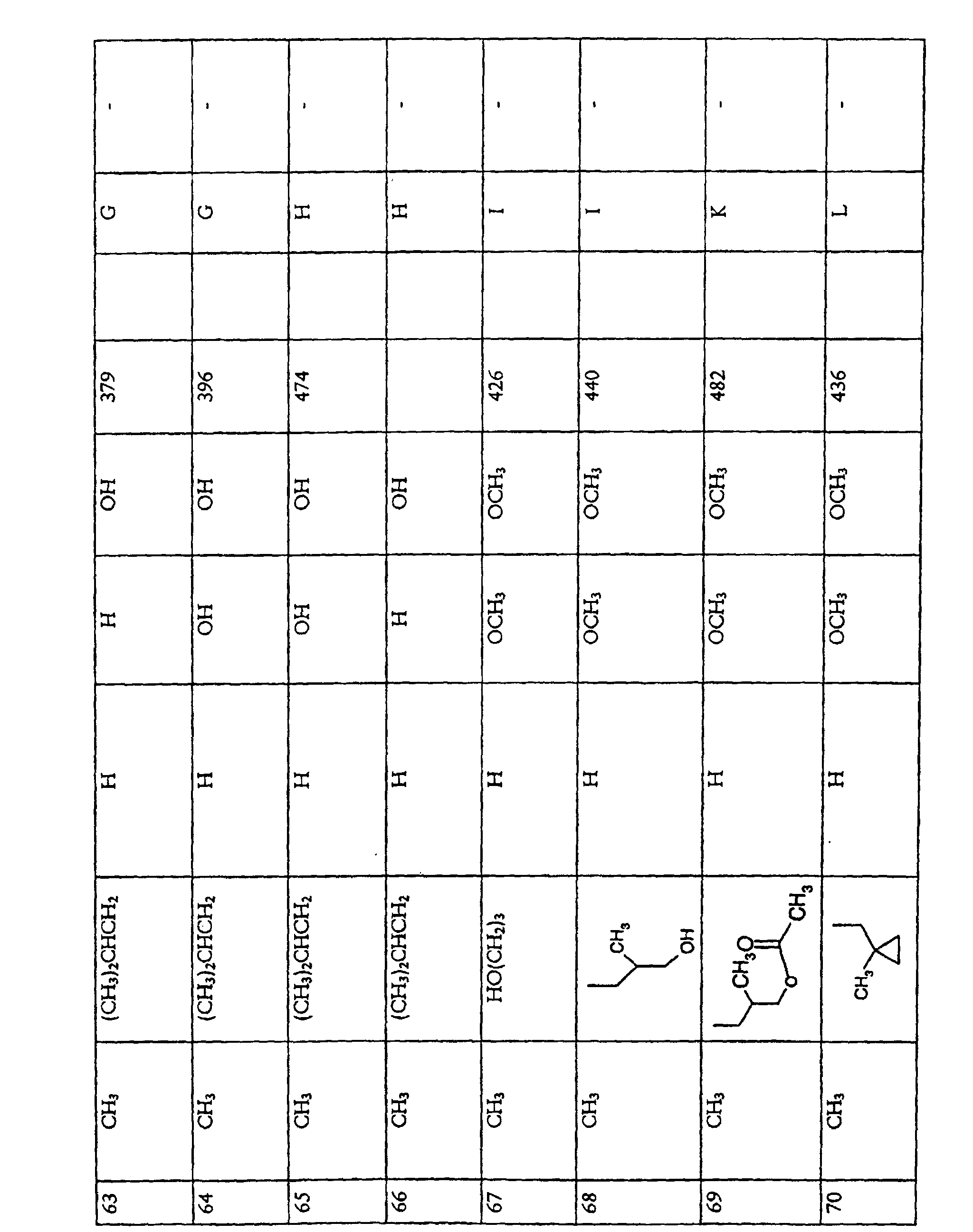

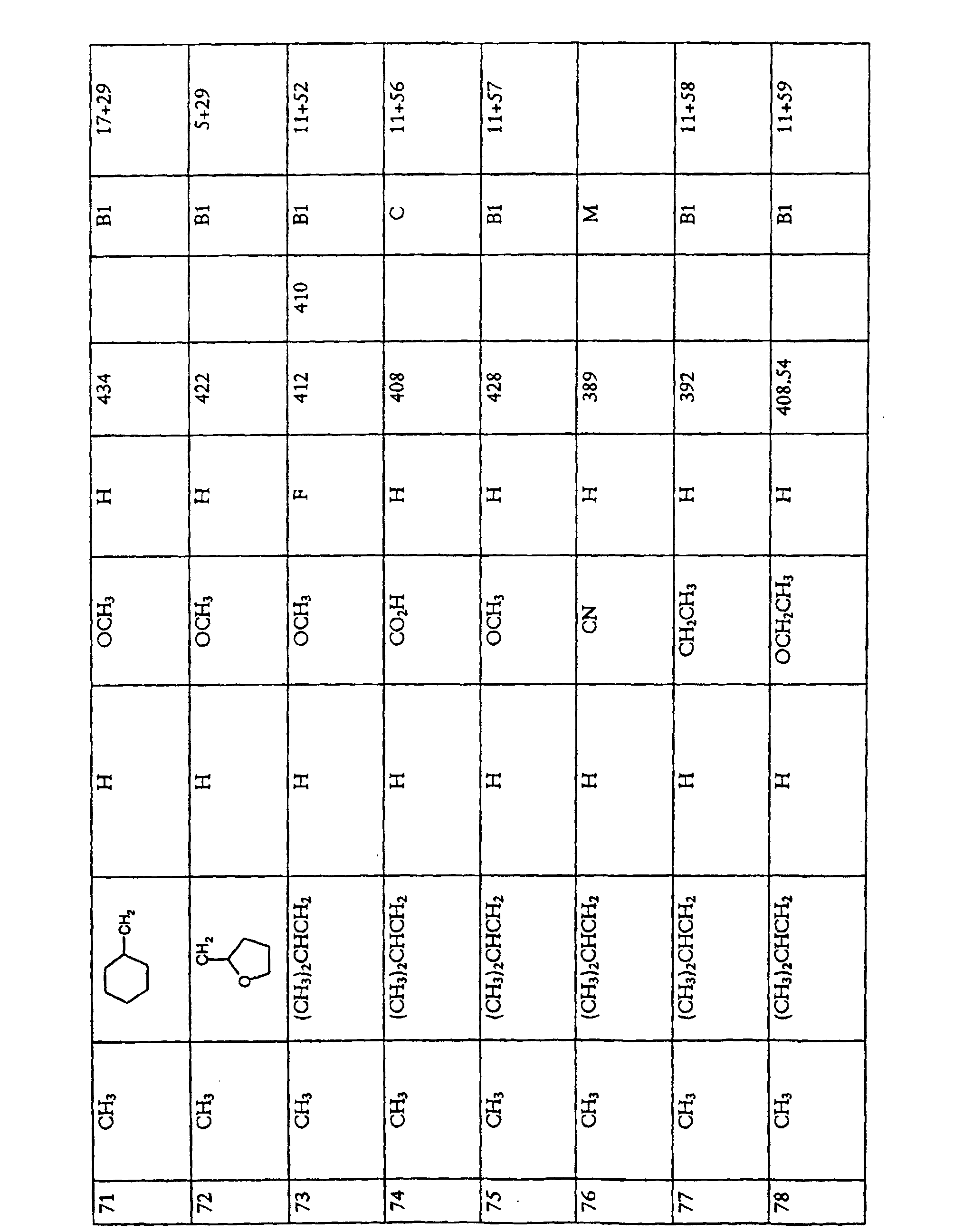

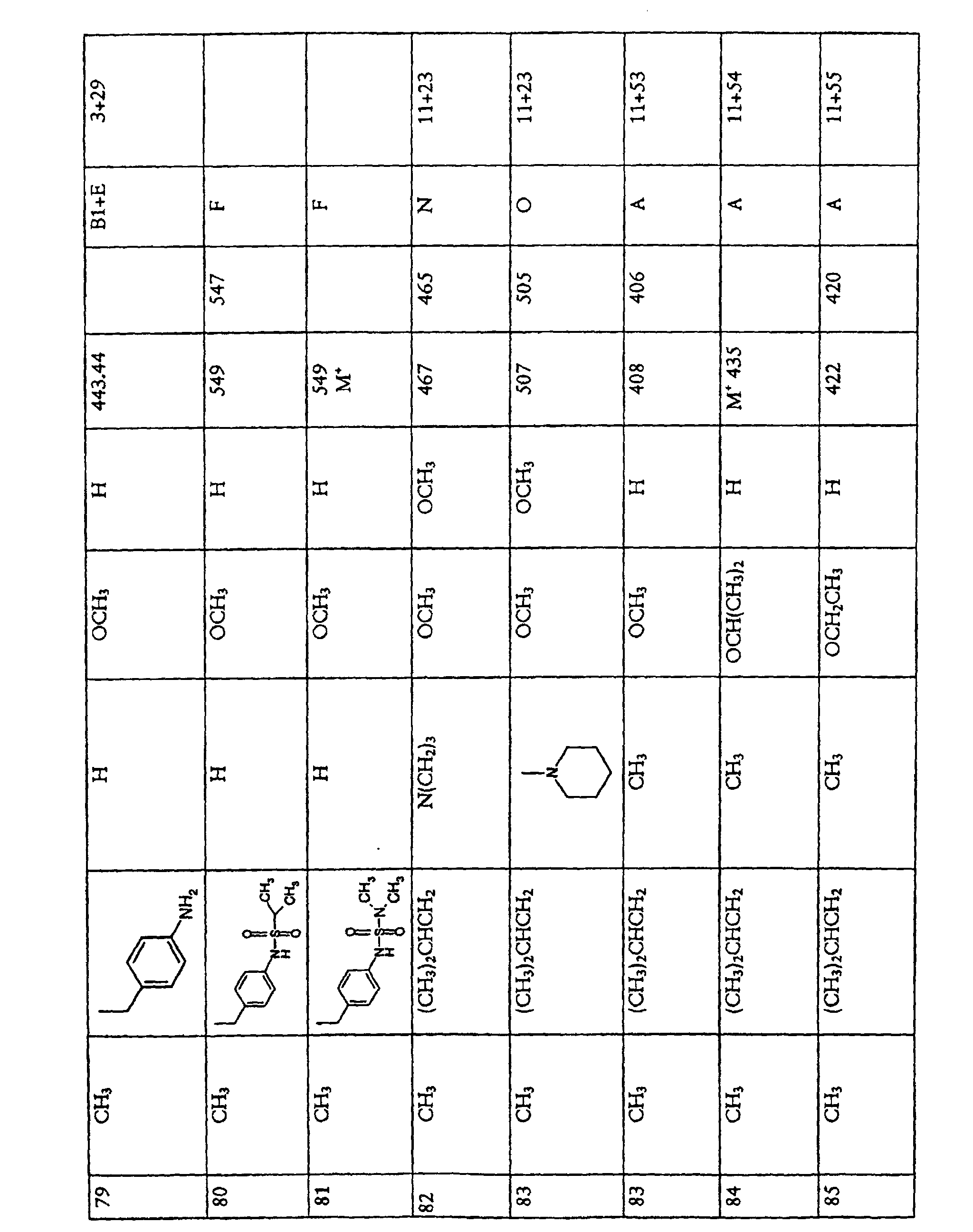

### Method A

1-(3-Dimethylaminopropyl)-3-ethylcarbodiimide hydrochloride (0.201g, 1.30mmol) is added to 5,6-diamino-1-isobutyl-3-methyl-1*H*-pyrimidine-2,4-dione (0.223g, 1.05mmol)) and (6,7-dimethoxy-1-methyl-isoquinolin-4-yl)-acetic acid *(0.25g,* 0.96mmol) in methanol (5ml) and water (1ml) and the mixture is stirred at ambient temperature for 16 hours. The methanol is evaporated and the resultant solid collected by filtration, taken into methanol (5ml) and 5M aqueous sodium hydroxide (0.5ml) is added. The reaction is heated to reflux for 1 hour, cooled to ambient temperature and evaporated. The residue is dissolved in water and extracted with dichloromethane, the combined organic extracts are dried over sodium sulfate and evaporated to afford 8-(6,7-dimethoxy-1-methyl-isoquinolin-4-ylmethyl)-3-isobutyl-1-methyl-3,7-dihydro-purine-2,6-dione, M+ 437.

### Method B1

(6-Ethynyl-isoquinolin-4-yl)-acetic acid (58mg, 0.28mmol) is dissolved in DMF (1ml) and O-(7-azabenzotriazo-1-yl)-N,N,N',N'-tetramethyluronium hexafluorophosphate (0.125g, 0.33mmol) and Hunig's base (0.180ml, 1.03mmol) are added, followed by a solution of 5,6-diamino-1-isobutyl-3-methyl-1*H*-pyrimidine-2,4-dione (58mg, 0.28mmol) in DMF (0.7ml). The reaction is stirred at room temperature for 2 hours. The solvent is evaporated and the residue purified by flash column chromatography (30:1 dichloromethane-methanol elution). The intermediate is dissolved in methanol (2ml) and water (2.75ml) added, followed by 4M aqueous sodium hydroxide (0.25ml). The reaction is heated at 40°C for 2 hours, then stirrred for 16 hours at ambient temperature. The solvent is evaporated and the crude product purified by flash column chromatography (30:1 dichloromethane-methanol elution) to afford 8-(6-ethynyl-isoquinolin-4-ylmethyl)-3-isobutyl-1-methyl-3,7-dihydro-purine-2,6-dione, [MH]⁺ 388.

### Method B2

A suspension of (6-methoxy-isoquinolin-4-yl)-acetic acid (3.5g, 13.82mmol) in acetonitrile (70ml) is treated sequentially with Hunig's base (6.15ml, 36mmol), O-(7-benzotriazo-1-yl)-N,N,N',N'-tetramethyluronium hexafluorophosphate (6.29g, 16.6mmol) and 5,6-diamino-1-isobutyl-3-methyl-1*H*-pyrimidine-2,4-dione (3.22g, 15.2 at mmol) while the solution is stirred at room temperature. The reaction is stirred at ambient temperature for 2h, prior to evaporation of the solvent. The residue is tritruated with ethyl acetate (50ml) filtered and washed with ethyl acetate and then dried at 50°C under reduced pressure. The resulting intermediate is suspended in a mixture of methanol (30ml) and 4M aqueous sodium hydroxide (60ml) and heated at 80°C for 45 minutes. This suspension is neutralised with acetic acid and cooled to 0-5°C overnight. The resultant solid is collected by filtration, and washed with methanol/water 1:9 (30ml) followed by methanol (30ml). Drying under high vacuum at 50°C affords 3-isobutyl-8-(6-methoxy-isoquinolin-4-ylmethyl)-1-methyl-3,7-dihydro-purine-2,6-dione, [MH]⁺ 394.5.

### Method C

1-(3-Dimethylaminopropyl)-3-ethylcarbodiimide hydrochloride (5.6M aqueous solution, 0.33ml, 1.85mmol) is added to a suspension of 5,6-diamino-1-isobutyl-3-methyl-1*H*-pyrimidine-2,4-dione (0.327g, 1.54mmol), (1-chloro-6,7-dimethoxy-isoquinolin-4-yl)-acetic acid (0.414g, 1.54mmol) and 1-hydroxybenzotriazole (0.251g, 1.85mmol) in CH₂Cl₂ (2ml). Water (2ml) is added, the biphasic mixture is shaken for 18 hours and the the resultant solid is collected by filtration. This intermediate is suspended in methanol (10ml), 4M aqueous NaOH (5ml) is added and the mixture heated to reflux for 4 hours. After evaporation of the methanol, the residue is acidified to pH2 with concentrated hydrochloric acid and the resultant solid collected by filtration and purified by preparative HPLC to afford 8-(1-chloro-6,7-dimethoxy-isoquinolin-4-ylmethyl)-3-isobutyl-1-methyl-3,7-dihydro-purine-2,6-dione hydrochloride, [MH]⁺ 458.

### Method D

1-(3-Dimethylaminopropyl)-3-ethylcarbodiimide (20.6ml, 0.11mol) is added to a mixture of 5,6-diamino-1-isobutyl-3-methyl-1*H*-pyrimidine-2,4-dione (20g, 0.094mol), (5,6-dimethoxy-isoquinolin-4-yl)-acetic acid (26.7g, 0.094mol), and 1-hydroxybenzotriazole (19.2g, 0.142mol) in 1:1 dichloromethane-water (400ml). The reaction is stirred at ambient temperature for 4.5 hours and the resultant solid collected by filtration. Slurrying in water (500ml), filtration and washing with water (250ml) followed by drying, further tritruation with methanol and drying gives an intermediate together with slightly less pure material from concentration of the methanol triturate. The intermediate (16.08g) is dissolved in water (100ml) and methanol (100ml) followed by the addition of 4M aqueous sodium hydroxide (56ml) and the resultant solution is heated at 70°C over night. After cooling to ambient temperature, the methanol is evaporated and the residue acidified to pH 1 with concentrated hydrochloric acid. The resultant hydrochloride salt is collected by filtration and dried. The product is then be converted to the free base by treatment with aqueous sodium hydroxide to pH 11 and washing with water to afford 3-isobutyl-8-(5,6-dimethoxy-isoquinolin-4-ylmethyl)-1-methyl-3,7-dihydro-purine-2,6-dione [MH]⁺ 424.6.

### Method E

A suspension of the product of Example 11 (72mg, 0.13mmol) in 6N HCl (2.5ml) and ethanol (1.5ml) is heated to reflux for 5 hours then stood at room temperature overnight. The resultant precipitate is collected by filtration, washed with water and dried to afford 3-(3-amino-benzyl)-8-(6,7-dimethoxy-isoquinolin-4-ylmethyl)-1-methyl-3,7-dihydro-purine-2,6-dione dihydrochloride, ¹H NMR (400MHz, DMSO) δ: 3.20 (s 3H), 3.95 (s 3H), 4.00 (s 3H), 4.75 (s 2H), 5.15 (s 2H), 7.15 (m 2H), 7.20 (s 1H), 7.30 (t *J* 6 1H), 7.65 (s 1H), 7.95 (s 1H), 8.50 (s 1H), 9.50 (s 1H), 13.6 (br s 1H).

### Method F

The product of Example *58* (37mg, 0.07mmol) is suspended in pyridine (1.5ml) and dimethylsulfamoyl chloride (23ml, 0.21mmol) is added. The reaction is heated at 50°C for 22 hours and the solvent is evaporated. Trituration with water gives a solid which is collected by filtration and dried to afford 3-[3-(N,N-dimethylsulfamoyl)amino-benzyl]-8-(6,7-dimethoxy-isoquinolin-4-ylmethyl)-1-methyl-3,7-dihydro-purine-2,6-dione, ¹H NMR (400MHz, DMSO) δ: 2.64 (s 6H), 3.26 (s 3H), 3.86 (s 3H), 3.98 (s 3H), 4.50 (s 2H), 5.15 (s 2H), 6.98 (d *J* 6 1H), 7.08 (d *J* 6 1H), 7.15 (s 1H), 7.22 (t *J* 6 3H), 7.55 (s 1H), 7.62 (s 1H), 8.38 (s 1H), 9.15 (s 1H), 9.82 (s 1H), 13.60 (s 1H).

### Method G

The product of Example 24 (100mg, 0.25mmol) is heated at 100°C in concentrated hydrobromic acid (5ml) for 36 hours. The solvent is evaporated and the crude product purified by preparative HPLC to afford 8-(7-hydroxy-isoquinolin-4-ylmethyl)-3-isobutyl-1-methyl-3,7-dihydro-purine-2,6-dione, [M]⁺ 379.

### Method H

The product of Example 64 (41mg, 0.09mmol) is dissolved in acetic acid (2ml) and treated with bromine in acetic acid (148mg/ml solution: 100µl). After 1 hour at room temperature the solvent is evaporated, the residue dissolved in hot methanol, filtered and evaporated to afford 8-(8-bromo-6,7-dihydroxy-isoquinolin-4-ylmethyl)-3-isobutyl-1-methyl-3,7-dihydropurine-2,6-dione, M⁺ 474.

### Method I

A suspension of the product of Example 13, 3-allyl-8-(6,7-dimethoxy-isoquinolin-4-ylmethyl)-1-methyl-3,7-dihydro-purine-2,6-dione hydrochloride salt (0.760g, 1.87mmol), 9-borabicyclo[2.2.0]nonane (0.5M THF solution, 18.7ml, 9.35mmol) and diisopropylethylamine (0.33ml, 1.89mmol) in THF (9ml) is heated to reflux for 2.5 hours. Sodium hydroxide (4M aqueous solution, 6ml) and hydrogen peroxide (27.5%, 3ml) are added sequentially and the reaction heated at 50°C for *1.5* hours. After evaporation, the crude product is purified by flash chromatography (19:1 CH₂Cl₂:-methanol elution) and triturated with water to afford 8-(6,7-dimethoxy-isoquinolin-4-ylmethyl)-3-(3-hydroxypropyl)-1-methyl-3,7-dihydro-purine-2,6-dione, [MH]⁺ 426.

### Method J

Potassium carbonate (48mg, 0.35mmol) and iodomethane (0.018ml, 0.295mmol) are added to a solution of the product of Example 10, 8-(6,7-dimethoxy-isoquinolin-4-ylmethyl)-3-isobutyl-1-methyl-3,7-dihydro-purine-2,6-dione (0.100g, 0.24mmol) in DMF (2ml). The reaction is stirred overnight and purified by preparative HPLC to afford 8-(6,7-dimethoxy-isoquinolin-4-ylmethyl)-3-isobutyl-1,7-dimethyl-3,7-dihydro-purine-2,6-dione, [MH]⁺ 438.

### Method K

A suspension of the product of Example 68, 8-(6,7-dimethoxy-isoquinolin-4-ylmethyl)-3-(3-hydroxy-2-methyl-propyl)-1-methyl-3,7-dihydro-purine-2,6-dione (63mg, 0.14mmol) and acetyl chloride (18ml, 0.25mmol) in pyridine (1ml) is heated at 50°C for 18 hours. After evaporation, flash chromatography (19:1 dichloromethane-methanol elution) affords acetic acid 3-[8-(6,7-dimethoxy-isoquinolin-4-ylmethyl)-1-methyl-2,6-dioxo-1,2,6,7-tetrahydropurin-3-yl]-2-methyl-propyl ester, [MH]⁺ 482.

### Method L

The product of Example 18, 8-(6,7-dimethoxy-isoquinolin-4-ylmethyl)-1-methyl-3-(2-methyl-allyl)-3,7-dihydro-purine-2,6-dione (100mg, 0.24mmol) is suspended in 1,2-dichloroethane (30ml). Diethyl zinc (1M hexane solution, 1.2ml, 1.20mmol) is added, followed by chloroiodomethane (0.174ml, 0.24mmol) and the reaction is stirred at ambient temperature for 1 hour, prior to quenching with saturated aqueous NH₄Cl. After extraction with chloroform, the organic phase is washed with water, dried over MgSO₄ and evaporated. Purification by preparative HPLC affords 8-(6,7-dimethoxy-isoquinolin-4-ylmethyl)-1-methyl-3-(1-methyl-cyclopropylmethyl)-3,9-dihydro-purine-2,6-dione, [MH]⁺ 436.

### Method M

The product of Example 53, 8-(6-bromo-isoquinolin-4-ylmethyl)-3-isobutyl-1-methyl-3,7-dihydro-purine-2,6-dione (245mg, 0.554mmol) is dispersed in a mixture of triethylamine (0.085ml, 0.61mmol) and CH₂Cl₂ (4ml). To the stirred mixture is added dropwise a solution of di-tert butoxycarbonate (133mg, 0.61mmol) in CH₂Cl₂(1ml); after 2h triethylamine (0.170ml, 1.2mmol), di-tert butoxycarbonate (130mg, 0.60mmol) and DMF (0.3ml) are added and the mixture is stirred at room temperature for 2.5 days. Concentration, partitioning between water and hexane, sonication filtration, reconcentration followed by purification by flash silica column chromatography (eluant 19:1 CH₂Cl₂: methanol) gives 8-(6-bromo-isoquinolin-4-ylmethyl)-3-isobutyl-1-methyl-2,6-dioxo-1,2,3,6-tetrahydro-purine-7-carboxylic acid .tert.-butyl ester ([MH]⁺ 543). This intermediate (58mg, 0.11mmol) is added to Zn(CN)₂ (15mg, 0.13mmol) followed by 1,1'-bis(diphenylphosphino)ferrocene (9mg), tris(dibenzylideneacetone) dipalladium(0) (5mg) and anhydrous DMF (2.5ml) and the resulting mixture stirred at 120°C for 18h and then for a further 24h at 150°C. Zn(CN)₂ (57mg, 0.49mmol) and anhydrous DMF (1ml) are then added and the mixture is heated for 2h at 155°C for 2h followed by 18h at 145°C. 1,1'-Bis(diphenylphosphino)ferrocene (9mg), tris (dibenzylideneacetone)dipalladium(0) (9mg) are then added and the reaction is heated for a further 6h at 145°C. Concentration, tritruation with water, filtration, washing with 1:1 saturated NaHCO₃ / water, followed by extraction with CH₂Cl₂ and 1:1 methanol: CH₂Cl₂ and repetitive flash silica column chromatography ( eluants 10:1 CH₂Cl₂:methanol then 20:1 CH₂Cl₂:methanol) gives 4-(3-isobutyl-1-methyl-2,6-dioxo-2,3,6,7-tetrahydro-1*H*-purin-8-ylmethyl)-isoquinoline-6-carbonitrile [MH]⁺ 389.

### Method N

1-(3-Dimethylaminopropyl)-3-ethylcarbodiimide hydrochloride (0.29g, 1.9mmol) is added to 5,6-diamino-1-isobutyl-3-methyl-1*H*-pyrimidine-2,4-dione (0.40g, 1.9mmol)) and (1-chloro-6,7-dimethoxy-isoquinolin-4-yl)-acetic acid (0.39g, 1.78mmol) in methanol and water and the mixture is stirred at ambient temperature for 2 hours. The methanol is evaporated and the resultant solid collected by filtration and recrystalised from ethylacetate/methanol. The resulting solid is heated in a sealed tube (100°C, 8h) with 40% aqueous dimethylamine. The mixture is evaporated and extracted with ethylacetate. The ethylacetate solution is then washed with water and brine, dried over sodium sulphate, filtered and concentrated. Further purification by flash silica column chromatography (eluant: ethylacetate/methanol) yields 8-(1-dimethylamino-6,7-dimethoxy-isoquinolin-4-ylmethyl)-3-isobutyl-1-methyl-3,7-dihydro-purine-2,6-dione, MH⁺ 467.

### Method O

1-(3-Dimethylaminopropyl)-3-ethylcarbodiimide hydrochloride (0.29 g, 1.9mmol) is added to 5,6-diamino-1-isobutyl-3-methyl-1*H*-pyrimidine-2,4-dione (0.40g, 1.9mmol)) and (1-chloro-6,7-dimethoxy-isoquinolin-4-yl)-acetic acid (0.39g, 1.78mmol) in methanol and water and the mixture is stirred at ambient temperature for 2 hours. The methanol is evaporated and the resultant solid collected by filtration and recrystalised from ethylacetate/methanol. The resulting solid is heated under reflux with piperidine for 8h. The solution is filtered and the resulting solution washed with water and brine, dried over sodium sulphate, filtered and concentrated. Further purification by flash silica column chromatography (eluant: ethylacetate/methanol) yields a solid which is dissolved 20% 1N NaOH/ methanol and heated to reflux for 2h. Concentration, addition of water and extraction with ethyl acetate gives an organic fraction which is washed with water and brine, dried over Na2SO4, filtered and concentrated to give 8-(6,7-dimethoxy-1-piperidin-1-yl-isoquinolin-4-ylmethyl)-3-isobutyl-1-methyl-3,7-dihydro-purine-2,6-dione, MH⁺ 507.

### NMR data for Examples (¹H 400MHz DMSO-d6)

### Example 12

δ 3.25 (s 3H), 3.92 (s 3H), 4.02 (s 3H), 4.65 (s 2H), 7.70 (s 1H), 7.88 (s 1H), 8.45 (s 1H), 9.42 (s 1H), 11.1 (s 1H), 13.60 (s 1H)

### Example 13

δ 3.20 (s 3H), 4.95 (s 3H), 4.00 (s 3H), 4.52 (d *J* 4 2H), 4.70 (s 2H), 5.04 (d *J* 18 1H), 5.09 (d *J* 10 1H), 5.88 (m 1H), 7.60 (s 1H), 7.88 (s 1H), 8.46 (s 1H), 9.42 (s 1H), 13.7 (s 1H).

### Example 14

δ 0.20-0.40 (m 4H), 1.10-1.30 (m 1H), 3.21 (s 3H), 3.81 (m 2H), 3.98 (s 3H), 4.03 (s 3H), 4.66 (s 2H), 7.65 (s 1H), 7.85 (s 1H), 8.45 (s 1H), 9.39 (s 1H), 13.70 (s 1H).

### Example 15

δ 0.82 (s 9H), 3.20 (s 3H), 3.78 (s 2H), 3.99 (s 3H), 4.04 (s 3H), 7.62 (s 1H), 7.90 (s 1H), 8.45 (s 1H), 9.44 (s 1H), 13.60 (s 1H).

### Example 16

δ 0.81 (d *J* 7 12H), 1.98 (m 1H), 2.12 (m 1H), 3.70 (d *J* 8 2H), 3.78 (d *J* 7 2H), 3.99 (s 3H), 4.05 (s 3H), 4.70 (s 2H), 7.65 (s 1H), 7.90 (s 1H), 8.46 (s 1H), 9.45 (s 1H), 13.6 (s 1H).

### Example 17

δ 0.80-1.10 (m 6H), 1.40-1.60 (m 4H), 1.80 (m 1H), 3.15 (s 1H), 3.76 (d *J* 8 2H), 3.91 (s 3H), 4.02 (s 3H), 4.68 (s 2H), 7.60 (s 1H), 7.88 ( s 1H), 8.44 (s 1H), 13.60 (s 1H).

### Example 18

δ 1.69 (s 3H), 3.21 (s 3H), 3.98 (s 3H), 4.01 (s 3H), 4.46 (s 2H), 4.52 (s 1H), 4.68 (s 2H), 4.76 (s 1H), 7.58 (s 1H), 7.84 (s 1H), 8.45 (s 1H), 9.42 (s 1H), 13.60 (s 1H).

### Example 19

δ 1.50-1.85 (m 4H), 3.18 (s 3H), 3.50-3.85 (m 4H), 3.95 (s 3H), 4.02 (s 3H), 4.10-4.20 (m 1H), 4.70 (s 2H), 7.75 (s 1H), 7.920 (s 1H), 8.50 (s 1H), 9.50 (s 1H), 13.60 (br s 1H).

### Example 20

δ 0.70-0.80 (m 6H), 0.99-1.10 (m 1H), 1.20-1.25 (m 1H), 1.88-2.00 (m 1H), 3.21 (s 3H), 3.64-3.80 (m 2H), 3.95 (s 3H), 4.00 (s 3H), 4.68 (s 2H), 7.60 (s 1H), 7.80 (s 1H), 8.45 (s 1H), 9.42 (s 1H), 13.60 (br s 1H).

### Example 21

δ 0.83 (t *J* 8 3H), 1.63 (sextet *J* 8 2H), 3.83 (t *J* 8 2H), 3.99 (s 3H), 4.05 (s 3H), 4.69 (s 2H), 7.64 (s 1H), 7.88 (s 1H), 8.44 (s 1H), 9.42 (s 1H), 11.10 (s 1H), 13.60 (s 1H).

### Example 23

δ 0.80 (d *J* 7 6H), 3.18 (s 3H), 3.75 (d *J* 8 2H), 4.60 (s 2H), 6.32 (s 2H), 7.71 (s 1H), 7.82 (s 1H), 8.50 (s 1H), 9.42 (s 1H), 13.50 (s 1H).

### Example 49

δ 0.12-0.25 (m 4H), 1.02-1.10 (m 1H), 3.20 (s 3H), 3.68 (d *J* 7 2H), 4.00 (s 3H), 4.80 (s 2H), 7.70 (d *J* 9 1H), 8.21 (d *J* 9 1H), 8.38 (s 1H), 9.20 (s 1H), 13.10 (s 1H).

### Example 86 - 3-Isobutyl-1-methyl-8-[1-(6-methyl-5-oxo-5,6-dihydro-[1,3]dioxolo[4,5-.g.]isoquinolin-8-yl)-ethyl]-3,7-dihydro-purine-2,6-dione

Benzo[1,3]dioxol-5-ylmethyl-(2,2-dimethoxy-ethyl)-amine (Tetrahedron 1968, 24, 1467) is treated with pyruvic acid according to the general procedure for Intermediate 22 to afford 2-[1,3]dioxolo[4,5-.g.]isoquinolin-8-yl-propionic acid hydrochloride, mp 224-226°C. Treatment with HCl gas in ethanol affords the corresponding ethyl ester hydrochloride, mp 223-225°C. A solution of this compound (2.73g, 10mmol) in benzene (20ml) is treated with dimethyl sulfate (1.26g, 10mmol), stirred at room temperature for 5 hours and the solvent is evaporated. The crude oil is dissolved in water (20ml), cooled to 0-5°C and a solution of K₃Fe(CN)₆ (5.72g, 17.4mmol) in water (25ml) and sodium hydroxide (2.04g, 51mmol) in water (15ml) are added. After 1.5 hours at 5°C, the reaction is adjusted to pH 2 with concentrated hydrochloric acid and the product collected by filtration then crystallised from methanol-dichloromethane to afford 2-(6-methyl-5-oxo-5,6-dihydro-[1,3]dioxolo[4,5-g.]isoquinolin-8-yl)-propionic acid, mp 290°C (dec). This intermediate is then converted to the xanthine according to the general procedure of Method D, [MH]⁺ 452.

### Example 87 - 8-(6,7-Dimethoxy-quinolin-4-ylmethyl)-3-isobutyl-1-methyl-3,7-dihydro-purine-2,6-drone

Lithium diisopropylamide (2M pentane solution 2.46ml, 4.92mmol) and potassium t-butoxide (0.552g, 4.92mmol) are added to THF (10ml) at -70°C, followed by 6,7-dimethoxy-4-methyl-quinoline [J. Org. Chem., 1997, 623, 568] (1.0g, 4.92mmol). After 1 hour the reaction is poured on to an excess of crushed dry ice and warmed to room temperature overnight. Pyridine hydrochloride (0.57g, 4.92mmol) is added and the reaction partitioned between ether and water. The aqueous phase is evaporated, taken into hot methanol, treated with charcoal, filtered through celite and evaporated to afford (6,7-dimethoxy-quinolin-4-yl)-acetic acid, MH⁺ 248. This intermediate is then converted to the xanthine acording to the general procedure of Method C, mp >250°C.

## Claims

1. A compound of formula 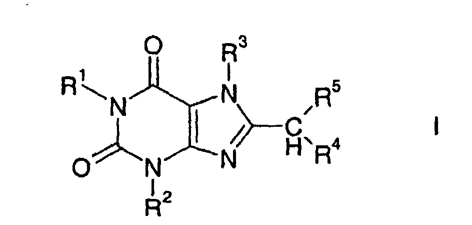 in free or salt form, where
R¹ is hydrogen or alkyl optionally substituted by hydroxy, alkoxy, or alkylthio,
R² is hydrogen, alkyl, hydroxyalkyl, alkylcarbonyloxyalkyl, alkoxyalkyl, alkylthioalkyl, alkenyl, cycloalkylalkyl, heterocyclylalkyl, aralkyl in which the aryl ring thereof is optionally fused to a 5-membered heterocyclic group or is optionally substituted by one or more substituents selected from alkoxy, amino, alkylamino, dialkylamino, acylamino, halogen, hydroxy, aminosulfonyl, alkylaminosulfonyl, dialkylaminosulfonyl, alkylsulfonylamino or dialkylaminosulfonylamino,
R³ is hydrogen or alkyl optionally substituted by hydroxy, alkoxy, or alkylthio,
R⁴ is hydrogen or alkyl,
R⁵ is a quinolinyl, isoquinolinyl or oxodihydroisoquinolinyl group optionally fused to a 5- membered heterocyclic group and optionally substituted by one or more substituents selected from halogen, cyano, hydroxy, alkyl, hydroxyalkyl, alkoxyalkyl, alkylthioalkyl, alkoxy, alkylthio, alkenyl, alkoxycarbonyl, alkynyl, carboxyl, acyl, a group of formula -N(R⁶)R⁷, aryl optionally substituted by one or more substituents selected from halogen or alkoxy, or heteroaryl having 5 or 6 ring atoms attached through a ring carbon atom to the indicated carbon atom, and
R⁶ and R⁷ are each independently hydrogen or alkyl optionally substituted by hydroxy or alkoxy or one of R⁶ and R⁷ is hydrogen and the other is acyl, or R⁶ and R⁷ together with the nitrogen atom to which they are attached denote a 5- or 6- membered heterocyclyl group.

2. A compound according to claim 1, in which R⁵ is a quinolinyl group of formula 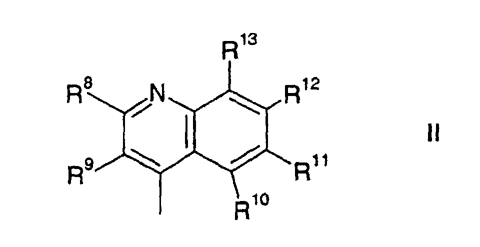 or an isoquinolinyl group of formula 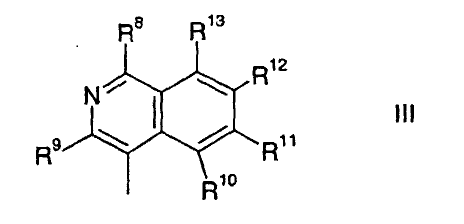 or an oxodihydroisoquinolinyl group of formula 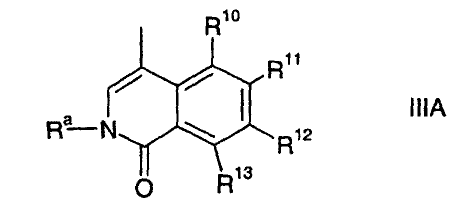 where R⁸, R⁹, R¹⁰, R¹¹, R¹² and R¹³ are each independently hydrogen or a substituent selected from halogen, cyano, hydroxy, alkyl, hydroxyalkyl, alkoxyalkyl, alkylthioalkyl, alkoxy, alkylthio, alkenyl, alkoxycarbonyl, alkynyl, carboxyl, acyl, a group of formula -N(R⁶)R⁷, aryl optionally substituted by one or more substituents selected from halogen or alkoxy, or heteroaryl having 5 or 6 ring atoms, and R⁶ and R⁷ are as defined in claim 1, or R¹¹ and R¹² together with the carbon atoms to which they are attached denote a 5- membered heterocyclic group having two oxygen or nitrogen atoms in the ring, and R^{a} is hydrogen or C₁-C₄-alkyl.

3. A compound according to claim 1, in which
R¹ is hydrogen or C₁-C₄-alkyl optionally substituted by hydroxy, C₁-C₄-alkoxy or C₁-C₄-alkylthio,
R² is hydrogen, C₁-C₈-alkyl, hydroxy-C₁-C₈-alkyl, C₁-C₄-alkylcarbonylonxy-C₁-C₈-alkyl, C₁-C₄-alkoxy-C₁-C₈-alkyl, or C₁-C₄-alkylthio-C₁-C₈-alkyl, C₂-C₄-alkenyl, C₃-C₈-cycloalkyl-C₁-C₄-alkyl, heterocyclyl-C₁-C₄-alkyl where the heterocyclyl group is a 5- or 6- membered heterocyclyl group having one or two hetero atoms selected from nitrogen and oxygen atoms in the ring, phenyl-C₁-C₄-alkyl in which the phenyl ring is optionally substituted by one or more substituents selected from C₁-C₄-alkoxy, amino, C₁-C₄-alkylamino, di(C₁-C₄-alkyl)amino, C₁-C₄-alkylcarbonylamino, halogen, C₁-C₄-alkylsulfonylamino, or di(C₁-C₄-alkyl)aminosulfonylamino, and is optionally fused to a 5- membered heterocyclic ring having two oxygen or two nitrogen atoms in the ring,
R³ is hydrogen or C₁-C₄-alkyl optionally substituted by hydroxy, C₁-C₄-alkoxy or C₁-C₄-alkylthio,
R⁴ is hydrogen or C₁-C₄-alkyl,
R⁵ is a quinolinyl, isoquinolinyl or oxodihydroisoquinolinyl group optionally fused to a 5-membered heterocyclic group having two oxygen or two nitrogen atoms in the ring and optionally substituted by one or more substituents selected from halogen, cyano, carboxy, hydroxy, C₁-C₄-alkyl, hydroxy-C₁-C₄-alkyl, C₁-C₄-alkoxy-C₁-C₄-alkyl, C₁-C₄-alkylthio-C₁-C₄-alkyl, C₁-C₄-alkoxy, C₁-C₄-alkylthio, C₂-C₄-alkenyl, C₂-C₄-alkynyl, C₁-C₄-alkylcarbonyl, a group -N(R⁶)R⁷ or phenyl optionally substituted by one or more substituents selected from halogen or C₁-C₄-alkoxy and
R⁶ and R⁷ are each independently hydrogen or C₁-C₄-alkyl optionally substituted by hydroxy or alkoxy, or one of R⁶ and R⁷ is hydrogen and the other is C₁-C₄-alkylcarbonyl, or
R⁶ and R⁷ together with the nitrogen atom to which they are attached denote a 5- or 6-membered heterocyclyl group having one or two nitrogen atoms and, optionally, an oxygen atom in the ring.

4. A compound according to claim 2, in which
R¹ is hydrogen or C₁-C₄-alkyl, R² is hydrogen, C₁-C₈-alkyl, hydroxy-C₁-C₈-alkyl, or C₁-C₄-alkylcarbonyloxy-C₁-C₈-alkyl, C₂-C₄-alkenyl, C₃-C₆-cycloalkyl-C₁-C₄-alkyl, heterocyclyl-C₁-C₄-alkyl where the heterocyclyl group is a 5- membered heterocyclyl group having one nitrogen or oxygen atom in the ring, phenyl-C₁-C₄-alkyl in which the phenyl ring is optionally substituted by one or two substituents selected from C₁-C₄-alkoxy, amino, C₁-C₄-alkylcarbonylamino, chlorine, bromine, C₁-C₄-alkylsulfonylamino, or di(C₁-C₄-alkyl)aminosulfonylamino and is optionally fused to a 5- membered heterocyclic ring having two oxygen atoms in the ring,
R³ is hydrogen or C₁-C₄-alkyl,
R⁴ is hydrogen or C₁-C₄-alkyl,
R⁵ is a quinolinyl group of formula II, an isoquinolinyl group of formula III or an oxodihydroisoquinolinyl group of formula IIIA, where R⁸, R⁹, R¹⁰, R¹¹, R¹² and R¹³ are each independently selected from hydrogen, halogen, cyano, carboxy, hydroxy, C₁-C₄-alkyl, hydroxy-C₁-C₄-alkyl, C₁-C₄-alkoxy-C₁-C₄-alkyl, C₁-C₄-alkylthioC₁-C₄-alkyl, C₁-C₄-alkoxy, C₁-C₄-alkylthio, C₂-C₄-alkenyl, C₂-C₄-alkynyl, C₁-C₄-alkylcarbonyl, a group -N(R⁶)R⁷ or phenyl optionally substituted by one or two substituents selected from halogen or C₁-C₄-alkoxy, or R¹¹ and R¹² together with the carbon atoms to which they are attached denote a 5-membered heterocyclic group having two oxygen atoms in the ring, and
R⁶ and R⁷ are each independently hydrogen or C₁-C₄-alkyl optionally substituted by hydroxy or alkoxy or one of R⁶ and R⁷ is hydrogen and the other is C₁-C₄-alkylcarbonyl, or R⁶ and R⁷ together with the nitrogen atom to which they are attached denote a 6-membered heterocyclyl group having one or two nitrogen atoms, or one nitrogen atom and one oxygen atom, in the ring.

5. A compound according to claim 4, in which R⁵ is an isoquinolinyl group of formula III in which R⁸ is hydrogen, C₁-C₄-alkyl, halogen, cyano, -N(R⁶)R⁷ where R⁶ and R⁷ are each independently C₁-C₄-alkyl or R⁶ and R⁷ together with the nitrogen atom to which they are attached denote a 6-membered heterocyclyl group having one or two nitrogen atoms, or one nitrogen atom and one oxygen atom, in the ring, or phenyl substituted by one or two C₁-C₄-alkoxy groups; R⁹ and R¹⁰ are each independently hydrogen, C₁-C₄-alkyl or halogen; R¹¹ and R¹² are each independently hydrogen, halogen, cyano, carboxy, hydroxy, C₁-C₄-alkyl, C₁-C₄-alkoxy or C₂-C₄-alkynyl, or R¹¹ and R¹² together with the carbon atoms to which they are attached denote a 5- membered heterocycle having two oxygen atoms in the ring; and R¹³ is hydrogen or halogen.

6. A compound of formula XXXXVI 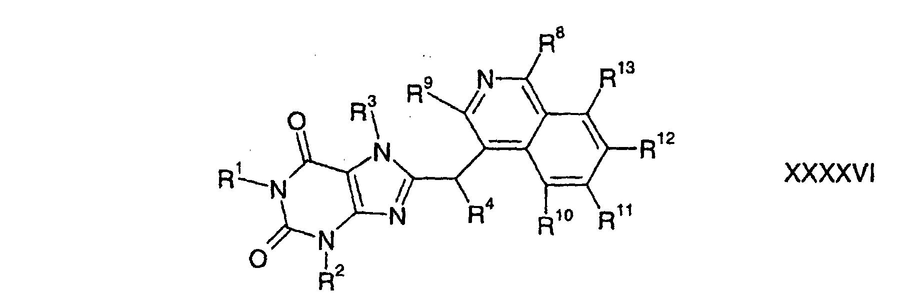 in free or salt form, where
(i) R¹ is CH₃, R² is (CH₃)₂CHCH₂, R³ and R⁴ are each H, R⁸ is CH₃, R⁹ and R¹⁰ are each H, and R¹¹ and R¹² are each OCH₃; or
(ii) R¹ is CH₃, R² is (CH₃)₂CHCH₂, R³, R⁴, R⁸, R⁹ and R¹⁰ are each H, and R¹¹ and R¹² are each OCH₃; or
(iii) R¹ is CH₃, R² is (CH₃)₃CCH₂, R³, R⁴, R⁸, R⁹ and R¹⁰ are each H, and R¹¹ and R¹² are each OCH₃; or
(iv) R¹ is CH₃, R² is (CH₃)₂CHCH₂, R³, R⁴, R⁹ and R¹⁰ are each H, R⁸ is Cl and R¹¹ and R¹² are each OCH₃; or
(v) R¹ is CH₃, R² is (CH₃)₂CHCH₂, R³, R⁴, R⁸, R⁹ and R¹⁰ are each H, R¹¹ is OCH₃ and R¹² is H; or
(vi) R¹ is CH₃, R² is cyclopropylmethyl, R³, R⁴, R⁸, R⁹, R¹⁰ and R¹² are each H and R¹¹ is OCH₃; or
(vii) R¹ is CH₃, R² is (CH₃)₂CHCH₂, R³, R⁴, R⁸, R⁹, R¹⁰ and R¹² are each H and R¹¹ is CH ≡C; or
(viii) R¹ is CH₃, R² is 4-(N-dimethylaminosulfonylamino)benzyl, R³, R⁴, R⁸, R⁹ and R¹⁰ are each H and R¹¹ and R¹² are each OCH₃; or
(ix) R¹ is CH₃, R² is HOCH₂CH(CH₃)CH₂, R³, R⁴, R⁸, R⁹ and R¹⁰ are each H and R¹¹ and R¹² are each OCH₃; or
(x) R¹ is CH₃, R² is 1-methylcyclopropylmethyl, R³, R⁴, R⁸, R⁹ and R¹⁰ are each H and R¹¹ and R¹² are each OCH₃.

7. A compound according to any one of claims 1 to 6 for use as a pharmaceutical.

8. A pharmaceutical composition comprising as active ingredient a compound according to any one of claims 1 to 6, optionally together with a pharmaceutically acceptable diluent or carrier.

9. The use of a compound according to any one of claims 1 to 6 for the manufacture of a medicament for the treatment of a condition mediated by PDE5.

10. The use of a compound according to any one of claims 1 to 6 for the manufacture of a medicament for the treatment of sexual dysfunction, particularly male erectile dysfunction.

11. A process for the preparation of a compound of formula I in free or salt form which comprises
1)
(a) dehydrating a compound of formula 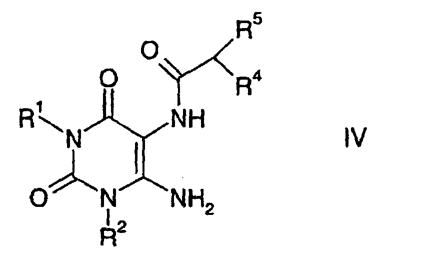 where R¹, R², R⁴ and R⁵ are as defined in claim 1; or
(b) for the preparation of a compound of formula I in free or salt form where R³ is alkyl optionally substituted by hydroxy, alkoxy or alkylthio, reacting a compound of formula I in free or salt form with an appropriate alkylating agent; or
(c) for the preparation of a compound of formula I in free or salt form where R² is aralkyl substituted in the aryl ring by alkylsulfonylamino or dialkylaminosulfonylamino, reacting a compound of formula I in salt form where R² is aralkyl substituted by amino with, respectively, an alkylsulfonyl halide or dialkylaminosulfonyl halide; or
(d) for the preparation of a compound of formula I in free or salt form where R² is hydroxy-substituted alkyl, hydration of a compound of formula I where R² is alkenyl; or
(e) for the preparation of a compound of formula I in free or salt form where R² is alkyl substituted by alkylcarbonyloxy, appropriate esterification of a compound of formula I where R² is hydroxy-substituted alkyl; or
(f) for the preparation of a compound of formula I in free or salt form where R² is aralkyl substituted in the aryl ring by amino, hydrolysing a compound of formula I where R² is aralkyl substituted in the aryl ring by acylamino; or
(g) for the preparation of a compound of formula I in free or salt form where R⁵ is quinolinyl or isoquinolinyl substituted by hydroxy, dealkylation of a compound of formula I where R⁵ is respectively quinolinyl or isoquinolinyl substituted by alkoxy; or
(h) for the preparation of a compound of formula I in free or salt form where R⁵ is quinolinyl or isoquinolinyl substituted by halogen, halogenation of a compound of formula I where R⁵ is respectively quinolinyl or isoquinolinyl having an unsubstituted ring carbon atom available for halogenation; or
(i) for the preparation of a compound of formula I in free or salt form where R² is a cyclopropyl group, optionally substituted by alkyl, subjecting a compound of formula I where R² is alkenyl to a Simmons Smith cyclopropanation reaction; and
2) recovering the resulting product of formula I in free or salt form.

12. A compound of formula IV 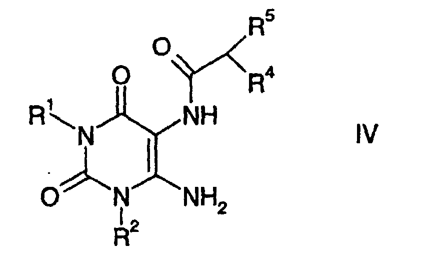 where R¹, R², R⁴ and R⁵ are as defined in claim 1.

## Patentansprüche

1. Verbindung der Formel 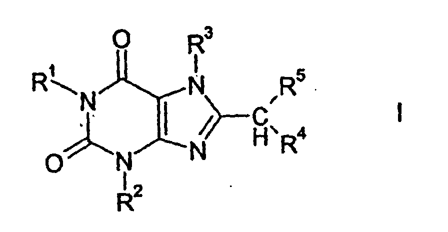 in freier Form oder Salzform, worin
R¹ fiir Wasserstoff oder Alkyl steht, das wahlweise mit Hydroxy, Alkoxy oder Alkylthio substituiert ist,
R² steht für Wasserstoff, Alkyl, Hydroxyalkyl, Alkylcarbonyloxyalkyl, Alkoxyalkyl, Alkylthioalkyl, Alkenyl, Cycloalkylalkyl, Heterocyclylalkyl, Aralkyl, worin der Arylring hiervon wahlweise an eine fünfgliedrige heterocyclische Gruppe fusioniert ist oder wahlweise mit einem oder mehreren Substituenten substituiert ist, ausgewählt aus Alkoxy, Amino, Alkylamino, Dialkylamino, Acylamino, Halogen, Hydroxy, Aminosulfonyl, Alkylaminosulfonyl, Dialkylaminosulfonyl, Alkylsulfonylamino oder Dialkylaminosulfonylamino,
R³ für Wasserstoff oder Alkyl steht, das wahlweise mit Hydroxy, Alkoxy oder Alkylthio substituiert ist,
R⁴ für Wasserstoff oder Alkyl steht,
R⁵ für eine Chinolinyl-, Isochinolinyl- oder Oxodihydroisochinolinylgruppe steht, die wahlweise mit einer fünfgliedrigen heterocyclischen Gruppe fusioniert ist und wahlweise substituiert ist mit einem oder mehreren Substituenten, ausgewählt aus Halogen, Cyano, Hydroxy, Alkyl, Hydroxyalkyl, Alkoxyalkyl, Alkylthioalkyl, Alkoxy, Alkylthio, Alkenyl, Alkoxycarbonyl, Alkinyl, Carboxyl, Acyl, eine Gruppe der Formel -N(R⁶)R⁷, Aryl, das wahlweise substituiert ist mit einem oder mehreren Substituenten, ausgewählt aus Halogen oder Alkoxy, oder Heteroaryl mit 5 oder 6 Ringatomen, das über ein Ringkohlenstoffatom an das angegebene Kohlenstoffatom gebunden ist, und
R⁶ und R⁷ jeweils unabhängig für Wasserstoff oder Alkyl stehen, das wahlweise mit Hydroxy oder Alkoxy substituiert ist oder eines von R⁶ und R⁷ für Wasserstoff steht und das andere für Acyl steht, oder R⁶ und R⁷ zusammen mit dem Stickstoffatom, an das sie gebunden sind, für eine fünf- oder sechsgliedrige Heterocyclylgruppe stehen.

2. Verbindung nach Anspruch 1, worin R⁵ für eine Chinolinylgruppe der folgenden Formel steht 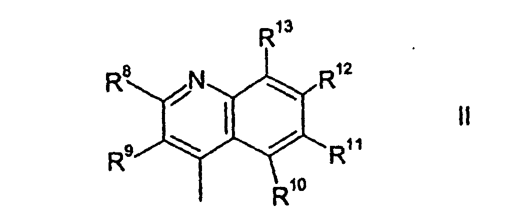 oder eine Isochinolinylgruppe der Formel 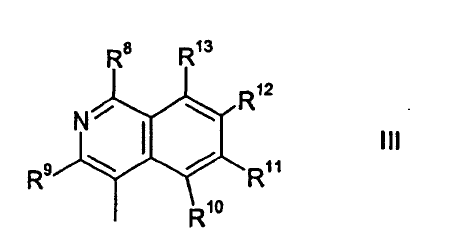 oder eine Oxodihydroisochinolinylgruppe der Formel 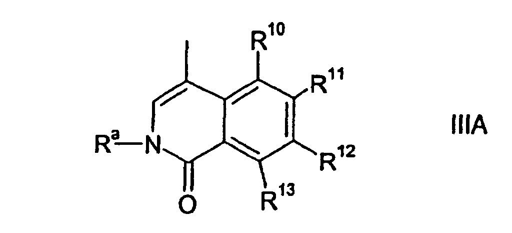 worin R⁸, R⁹, R¹⁰, R¹¹, R¹² und R¹³ jeweils unabhängig für Wasserstoff oder einen Substituenten stehen, ausgewählt aus Halogen, Cyano, Hydroxy, Alkyl, Hydroxyalkyl, Alkoxyalkyl, Alkylthioalkyl, Alkoxy, Alkylthio, Alkenyl, Alkoxycarbonyl, Alkinyl, Carboxyl, Acyl, eine Gruppe der Formel -N(R⁶)R⁷, Aryl, das wahlweise substituiert ist mit einem oder mehreren Substituenten, ausgewählt aus Halogen oder Alkoxy, oder Heteroaryl mit 5 oder 6 Ringatomen, und R⁶ und R⁷ wie in Anspruch 1 definiert sind oder R¹¹ und R¹² zusammen mit den Kohlenstoffatomen, an die sie gebunden sind, für eine fünfgliedrige heterocyclische Gruppe mit zwei Sauerstoff- oder Stickstoffatomen im Ring stehen, und R' für Wasserstoff oder C₁-C₄ Alkyl steht.

3. Verbindung nach Anspruch 1, worin
R¹ für Wasserstoff oder C₁-C₄ Alkyl steht, das wahlweise substituiert ist durch Hydroxy, C₁-C₄ Alkoxy oder C₁-C₄ Alkylthio,
R² steht für Wasserstoff, C₁-C₈ Alkyl, Hydroxy-C₁-C₈-alkyl, C₁-C₄ Alkylcarbonyloxy-C₁-C₈-alkyl, C₁-C₄ Alkoxy-C₁-C₈-alkyl oder C₁-C₄ Alkylthio-C₁-C₈-alkyl, C₂-C₄ Alkenyl, C₃-C₈ Cycloalkyl-C₁-C₄-alkyl, Heterocyclyl-C₁- C₄-alkyl, worin die Heterocyclylgruppe eine fünf- oder sechsgliedrige Heterocyclylgruppe mit ein oder zwei Heteroatomen ist, ausgewählt aus Stickstoff- und Sauerstoffatomen im Ring, Phenyl-C₁-C₄-alkyl, worin der Phenylring hiervon wahlweise mit einem oder mehreren Substituenten substituiert ist, ausgewählt aus C₁-C₄ Alkoxy, Amino, C₁-C₄ Alkylamino, Di(C₁-C₄-alkyl)amino, C₁-C₄ Alkylcarbonylamino, Halogen, C₁-C₄ Alkylsulfonylamino oder Di(C₁-C₄-alkyl)aminosulfonylamino, und wahlweise mit einem fünfgliedrigen heterocyclischen Ring mit zwei Sauerstoff- oder zwei Stickstoffatomen im Ring fusioniert ist,
R³ für Wasserstoff oder C₁-C₄ Alkyl steht, das wahlweise mit Hydroxy, C₁-C₄ Alkoxy oder C₁-C₄ Alkylthio substituiert ist,
R⁴ für Wasserstoff oder C₁-C₄ Alkyl steht,
R⁵ für eine Chinolinyl-, Isochinolinyl- oder Oxodihydroisochinolinylgruppe steht, die wahlweise mit einer fünfgliedrigen heterocyclischen Gruppe fusioniert ist, die zwei Sauerstoff- oder zwei Stickstoffatome im Ring aufweist, und wahlweise substituiert ist mit einem oder mehreren Substituenten, ausgewählt aus Halogen, Cyano. Carboxy Hydroxy, C₁-C₄ Alkyl, Hydroxy-C₁-C₄-alkyl, C₁-C₄ Alkoxy-C₁-C₄-alkyl, C₁-C₄ Alkylthio-C₁-C₄-alkyl, C₁-C₄ Alkoxy, C₁-C₄ Alkylthio, C₂-C₄ Alkenyl, C₂-C₄ Alkinyl, C₁-C₄ Alkylcarbonyl, eine Gruppe -N(R⁶)R⁷ oder Phenyl, das wahlweise substituiert ist mit einem oder mehreren Substituenten, ausgewählt aus Halogen oder C₁- C₄ Alkoxy, und
R⁶ und R⁷ jeweils unabhängig für Wasserstoff oder C₁-C₄ Alkyl stehen, das wahlweise mit Hydroxy oder Alkoxy substituiert ist oder eines von R⁶ und R⁷ für Wasserstoff steht und das andere für C₁-C₄ Alkylcarbonyl steht, oder R⁶ und R⁷ zusammen mit dem Stickstoffatom, an das sie gebunden sind, für eine fünf- oder sechsgliedrige Heterocyclylgruppe mit einem oder zwei Stickstoffatomen und wahlweise einem Sauerstoffatom im Ring stehen.

4. Verbindung nach Anspruch 2, worin
R¹ für Wasserstoff oder C₁-C₄ Alkyl steht,
R² steht für Wasserstoff, C₁-C₈ Alkyl, Hydroxy-C₁-C₈-alkyl oder C₁-C₄ Alkylcarbonyloxy-C₁-C₈-alkyl, C₂-C₄ Alkenyl, C₃-C₆ Cycloalkyl-C₁-C₄-alkyl, Heterocyclyl-C₁-C₄-alkyl, worin die Heterocyclylgruppe eine fünfgliedrige Heterocyclylgruppe mit einem Stickstoff- oder Sauerstoffatom im Ring ist, Phenyl-C₁-C₄-alkyl, worin der Phenylring wahlweise mit einem oder zwei Substituenten substituiert ist, ausgewählt aus C₁-C₄ Alkoxy, Amino, C₁- C₄ Alkylcarbonylamino, Chlor, Brom, C₁-C₄ Alkylsulfonylamino oder Di(C₁-C₄-alkyl)aminosulfonylamino, und wahlweise mit einem fünfgliedrigen heterocyclischen Ring mit zwei Sauerstoffatomen im Ring fusioniert ist,
R³ für Wasserstoff oder C₁-C₄ Alkyl steht,
R⁴ für Wasserstoff oder C₁-C₄ Alkyl steht,
R⁵ für eine Chinolinylgruppe der Formel II, eine Isochinolinylgruppe der Formel III oder eine Oxodihydroisochinolinylgruppe der Formel IIIA steht, worin R⁸, R⁹, R¹⁰, R¹¹, R¹² und R¹³ jeweils unabhängig ausgewählt sind aus Wasserstoff, Halogen, Cyano, Carboxy, Hydroxy, C₁-C₄ Alkyl, Hydroxy-C₁-C₄-alkyl, C₁-C₄ Alkoxy-C₁-C₄- alkyl, C₁-C₄ Alkylthio-C₁-C₄-alkyl, C₁-C₄ Alkoxy, C₁-C₄ Alkylthio, C₂-C₄ Alkenyl, C₂-C₄ Alkinyl, C₁-C₄ Alkylcarbonyl, eine Gruppe -N(R⁶)R⁷ oder Phenyl, das wahlweise substituiert ist mit einem oder zwei Substituenten, ausgewählt aus Halogen oder C₁-C₄ Alkoxy, oder R¹¹ und R¹² zusammen mit den Kohlenstoffatomen, an die sie gebunden sind, für eine fünfgliedrige, heterocyclische Gruppe mit zwei Sauerstoffatomen im Ring stehen, und
R⁶ und R⁷ jeweils unabhängig für Wasserstoff oder C₁-C₄ Alkyl stehen, das wahlweise mit Hydroxy oder Alkoxy substituiert ist oder eines von R⁶ und R⁷ für Wasserstoff steht und das andere für C₁-C₄ Alkylcarbonyl steht, oder R⁶ und R⁷ zusammen mit dem Stickstoffatom, an das sie gebunden sind, für eine sechsgliedrige heterocyclische Gruppe mit einem oder zwei Stickstoffatomen oder einem Stickstoffatom und einem Sauerstoffatom im Ring stehen.

5. Verbindung nach Anspruch 4, worin R⁵ für eine Isochinolinylgruppe der Formel III steht, worin R⁸ für Wasserstoff, C₁-C₄ Alkyl, Halogen, Cyano, -N(R⁶)R⁷ steht, worin R⁶ und R⁷ jeweils unabhängig für C₁-C₄ Alkyl stehen oder R⁶ und R⁷ zusammen mit dem Stickstoffatom, an das sie gebunden sind, für eine sechsgliedrige Heterocyclylgruppe mit einem oder zwei Stickstoffatomen oder einem Stickstoffatom und einem Sauerstoffatom im Ring stehen oder für Phenyl, das mit einer oder zwei C₁-C₄ Alkoxygruppen substituiert ist, R⁹ und R¹⁰ jeweils unabhängig voneinander für Wasserstoff, C₁-C₄ Alkyl oder Halogen stehen, R¹¹ und R¹² jeweils unabhängig für Wasserstoff, Halogen, Cyano, Carboxy, Hydroxy, C₁-C₄ Alkyl, C₁-C₄ Alkoxy oder C₂-C₄ Alkinyl stehen oder R¹¹ und R¹² zusammen mit den Kohlenstoffatomen, an die sie gebunden sind, für einen fünfgliedrigen Heterocyclus mit zwei Sauerstoffatomen im Ring stehen und R¹³ für Wasserstoff oder Halogen steht.

6. Verbindung der Formel XXXXVI 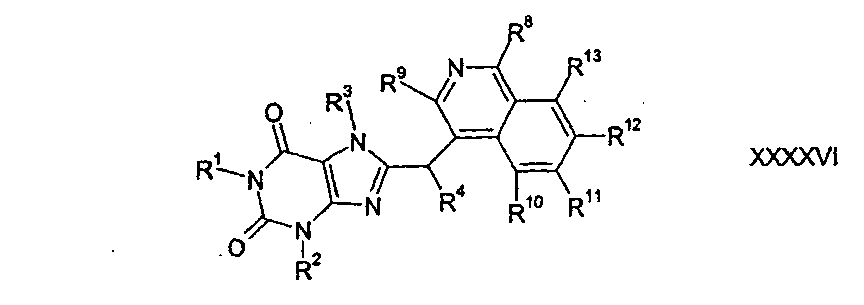 in freier Form, worin
(i) R¹ für CH₃ steht, R² für (CH₃)₂CHCH₂ steht, R³ und R⁴ jeweils für H stehen, R⁸ für CH₃ steht, R⁹ und R¹⁰ jeweils für H stehen und R¹¹ und R¹² jeweils für OCH₃ stehen, oder
(ii) R¹ für CH₃ steht, R² für (CH₃)₂CHCH₂ steht, R³, R⁴, R⁸, R⁹ und R¹⁰ jeweils für H stehen und R¹¹ und R¹² jeweils für OCH₃ stehen, oder
(iii) R¹ für CH₃ steht, R² für (CH₃)₃CCH₂ steht, R³, R⁴, R⁸, R⁹ und R¹⁰ jeweils für H stehen und R¹¹ und R¹² jeweils für OCH₃ stehen, oder
(iv) R¹ für CH₃ steht, R² für (CH₃)₂CHCH₂ steht, R³, R⁴, R⁹ und R¹⁰ jeweils für H stehen, R⁸ für Cl steht und R¹¹ und R¹² jeweils für OCH₃ stehen, oder
(v) R¹ für CH₃ steht, R² für (CH₃)₂CHCH₂ steht, R³, R⁴, R⁸, R⁹ und R¹⁰ jeweils für H stehen und R¹¹ für OCH₃ steht und R¹² für H steht, oder
(vi) R¹ für CH₃ steht, R² für Cyclopropylmethyl steht, R³, R⁴, R⁸, R⁹, R¹⁰ und R¹² jeweils für H stehen und R" für OCH₃ steht, oder
(vii) R¹ für CH₃ steht, R² für (CH₃)₂CHCH₂ steht, R³, R⁴, R⁸, R⁹, R¹⁰ und R¹² jeweils für H stehen und R¹¹ für CH≡C steht, oder
(viii) R¹ für CH₃ steht, R² für 4-(N-Dimethylaminosulfonylamino)benzyl steht, R³, R⁴, R⁸, R⁹ und R¹⁰ jeweils für H stehen und R¹¹ und R¹² jeweils für OCH₃ stehen, oder
(ix) R¹ für CH₃ steht, R² für HOCH₂CH(CH₃)CH₂ steht, R³, R⁴, R⁸, R⁹ und R¹⁰ jeweils für H stehen und R¹¹ und R¹² jeweils für OCH₃ stehen, oder
(x) R¹ filr CH₃ steht, R² für 1-Methylcyclopropylmethyl steht, R³, R⁴, R⁸, R⁹ und R¹⁰ jeweils für H stehen und R¹¹ und R¹² jeweils für OCH₃ stehen.

7. Verbindung nach einem der Ansprüche 1 bis 6 zur Verwendung als Pharmazeutikum.

8. Pharmazeutische Zusammensetzung, die als Wirkstoff eine Verbindung nach einem der Ansprüche 1 bis 6 wahlweise zusammen mit einem pharmazeutisch annehmbaren Verdünnungsmittel oder Träger enthält.

9. Verwendung einer Verbindung nach einem der Ansprüche 1 bis 6 zur Herstellung eines Arzneimittels zur Behandlung eines durch PDE5 bedingten Zustands.

10. Verwendung einer Verbindung nach einem der Ansprüche 1 bis 6 zur Herstellung eines Arzneimittels zur Behandlung von sexueller Dysfunktion, speziell männlicher Errektionsdysfunktion.

11. Verfahren zur Herstellung einer Verbindung der Formel I in freier Form oder Salzform, das umfaßt
1)
(a) Dehydratation einer Verbindung der Formel 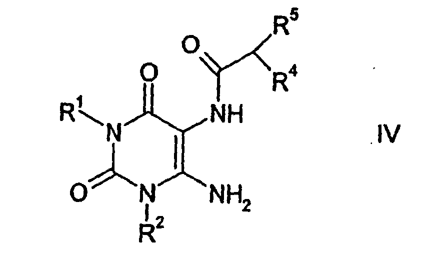 worin R¹, R², R⁴ und R⁵ wie in Anspruch 1 definiert sind, oder
(b) zur Herstellung einer Verbindung der Formel I in freier Form oder Salzform, worin R³ für Alkyl steht, das wahlweise substituiert ist durch Hydroxy, Alkoxy oder Alkylthio, Umsetzung einer Verbindung der Formel I in freier Form oder Salzform mit einem geeigneten Alkylierungsmittel, oder
(c) zur Herstellung einer Verbindung der Formel I in freier Form oder Salzform, worin R² filr Aralkyl steht, das im Arylring durch Alkylsulfonylamino oder Dialkylaminosulfonylamino substituiert ist, Umsetzung einer Verbindung der Formel I in Salzform, worin R² für Aralkyl steht, das mit Amino substituiert ist, mit jeweils einem Alkylsulfonylhalogenid oder Dialkylaminosulfonylhalogenid, oder
(d) zur Herstellung einer Verbindung der Formel I in freier Form oder Salzform, worin R²für hydroxysubstituiertes Alkyl steht, Hydratation einer Verbindung der Formel I, worin R² für Alkenyl steht, oder
(e) zur Herstellung einer Verbindung der Formel I in freier Form oder Salzform, worin R² für Alkyl steht, das mit Alkylcarbonyloxy substituiert ist, geeignete Veresterung einer Verbindung der Formel I, worin R² für hydroxysubstituiertes Alkyl steht, oder
(f) zur Herstellung einer Verbindung der Formel I in freier Form, worin R² für Aralkyl steht, das im Arylring mit Amino substituiert ist, Hydrolyse einer Verbindung der Formel I, worin R² für Aralkyl steht, das im Arylring durch Acylamino substituiert ist, oder
(g) zur Herstellung einer Verbindung der Formel I in freier Form oder in Salzform, worin R⁵ für Chinolinyl oder Isochinolinyl steht, das durch Hydroxy substituiert ist, Dealkylierung einer Verbindung der Formel I, worin R⁵ jeweils für Chinolinyl oder Isochinolinyl steht, das mit Alkoxy substituiert ist, oder
(h) zur Herstellung einer Verbindung der Formel I in freier Form oder in Salzform, worin R⁵ für Chinolinyl oder Isochinolinyl steht, das durch Halogen substituiert ist, Halogenierung einer Verbindung der Formel I, worin R⁵ jeweils für Chinolinyl oder Isochinolinyl steht, das ein unsubstituiertes Ringkohlenstoffatom aufweist, das für eine Halogenierung zugänglich ist, oder
(i) zur Herstellung einer Verbindung der Formel I in freier Form oder in Salzform, worin R² für eine Cyclopropylgruppe steht, die wahlweise mit Alkyl substituiert ist, Unterziehung einer Verbindung der Formel I, worin R² für Alkenyl steht, einer Simmons-Smith Cyclopropanierungsreaktion, und
2) Gewinnung des entstehenden Produkts der Formel I in freier Form oder Salzform.

12. Verbindung der Formel IV 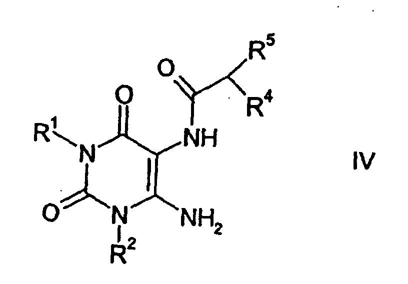 worin R¹, R², R⁴ und R⁵ wie in Anspruch 1 definiert sind.

## Revendications

1. Composé de formule : 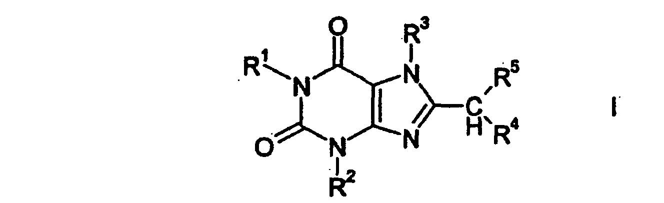 sous forme libre ou sous forme d'un sel, dans laquelle :
R¹ représente l'hydrogène ou un groupe alkyle facultativement substitué par un groupe hydroxy, alcoxy ou alkylthio,
R² représente l'hydrogène, un groupe alkyle, hydroxyalkyle, alkylcarbonyloxyalkyle, alcoxyalkyle, alkylthioalkyle, alcényle, cycloalkylalkyle, hétérocyclylalkyle, aralkyle, où le noyau aryle de celui-ci est facultativement condensé à un groupe hétérocyclique à 5 chaînons ou est facultativement substitué par un ou plusieurs substituants choisis parmi des groupes alcoxy, amino, alkylamino, dialkylamino, acylamino, halogène, hydroxy, aminosulfonyle, alkylaminosulfonyle, dialkylaminosulfonyle, alkylsulfonylamino ou dialkylaminosulfonylamino,
R³ représente l'hydrogène ou un groupe alkyle facultativement substitué par un groupe hydroxy, alcoxy ou alkylthio,
R⁴ représente l'hydrogène ou un groupe alkyle,
R⁵ est un groupe quinolinyle, isoquinolinyle ou oxodihydroisoquinolinyle facultativement condensé à un groupe hétérocyclique à 5 chaînons, et facultativement substitué par un ou plusieurs substituants choisis parmi un halogène, un groupe cyano, hydroxy, alkyle, hydroxyalkyle, alcoxyalkyle, alkylthioalkyle, alcoxy, alkylthio, alcényle, alcoxycarbonyle, alcynyle, carboxyle, acyle, un groupe de formule -N(R⁶)R⁷, aryle facultativement substitué par un ou plusieurs substituants choisis parmi un halogène ou un groupe alcoxy, ou hétéroaryle ayant 5 ou 6 atomes dans le noyau fixés par l'intermédiaire d'un atome de carbone du noyau à l'atome de carbone indiqué, et
R⁶ et R⁷ représentent chacun indépendamment l'hydrogène ou un groupe alkyle facultativement substitué par un groupe hydroxy ou alcoxy, ou l'un des radicaux R⁶ et R⁷ représente l'hydrogène et l'autre est un groupe acyle, ou bien R⁶ et R⁷, conjointement avec l'atome d'azote auquel ils sont fixés, représentent un groupe hétérocyclique à 5 ou 6 chaînons.

2. Composé suivant la revendication 1, dans lequel R⁵ est un groupe quinolinyle de formule : 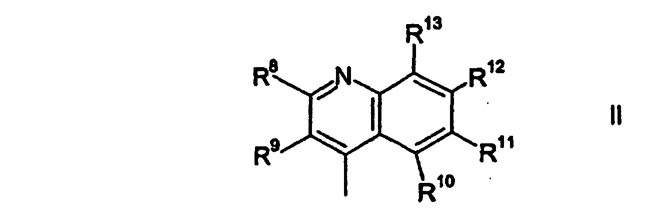 ou un groupe isoquinolinyle de formule : 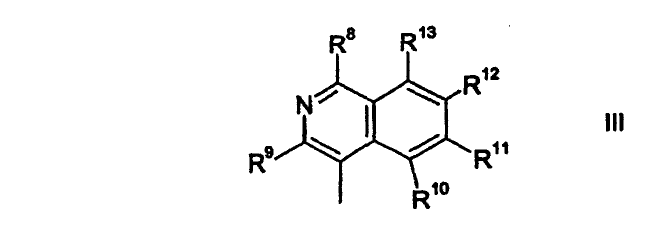 ou un groupe oxodihydroisoquinolinyle de formule : 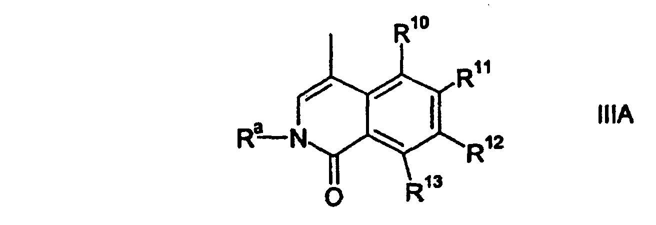 formules dans lesquelles R⁸, R⁹, R¹⁰, R¹¹, R¹² et R¹³ représentent chacun indépendamment l'hydrogène ou un substituant choisi parmi un halogène, un groupe cyano, hydroxy, alkyle, hydroxyalkyle, alcoxyalkyle, alkylthioalkyle, alcoxy, alkylthio, alcényle, alcoxycarbonyle, alcynyle, carboxyle, acyle, un groupe de formule -N(R⁶)R⁷, aryle facultativement substitué par un ou plusieurs substituants choisis parmi un halogène ou un groupe alcoxy, ou hétéroaryle ayant 5 ou 6 atomes dans le cycle, et R⁶ et R⁷ sont tels que définis dans la revendication 1, ou bien R¹¹ et R¹², conjointement avec les atomes de carbone auxquels ils sont fixés, représentent un groupe hétérocyclique à 5 chaînons ayant deux atomes d'oxygène ou d'azote dans le noyau, et R^{a} représente l'hydrogène ou un groupe alkyle en C₁ à C₄.

3. Composé suivant la revendication 1, dans lequel
R¹ représente l'hydrogène ou un groupe alkyle en C₁ à C₄ facultativement substitué par un groupe hydroxy, alcoxy en C₁ à C₄ ou alkylthio en C₁ à C₄,
R² représente l'hydrogène, un groupe alkyle en C₁ à C₈, hydroxy-alkyle en C₁ à C₈, alkyle en C₁ à C₄-carbonyloxy-alkyle en C₁ à C₈, alcoxy en C₁ à C₄-alkyle en C₁ à C₈ ou alkylthio en C₁ à C₄-alkyle en C₁ à C₈, alcényle en C₂ à C₄, cycloalkyle en C₃ à C₈-alkyle en C₁ à C₄, hétérocyclyl-alkyle en C₁ à C₄, où le groupe hétérocyclyle est un groupe hétérocyclyle à 5 ou 6 chaînons ayant un ou deux hétéroatomes choisis dans des atomes d'oxygène ou d'azote du noyau, phényl-alkyle en C₁ à C₄, dans lequel le noyau phényle est facultativement substitué par un ou plusieurs substituants choisis parmi un groupe alcoxy en C₁ à C₄, amino, alkyle en C₁ à C₄ amino, di-(alkyle en C₁ à C₄)-amino, alkyle en C₁ à C₄-carbonylamino, un halogène, un groupe alkyle en C₁ à C₄-sulfonylammo ou di-(alkyle en C₁ à C₄)aminosulfonylamino, et est facultativement condensé à un noyau hétérocyclique à 5 chaînons ayant deux atomes d'oxygène ou deux atomes d'azote dans le noyau,
R³ représente l'hydrogène ou un groupe alkyle en C₁ à C₄ facultativement substitué par un groupe hydroxy, alcoxy en C₁ à C₄ ou alkylthio en C₁ à C₄,
R⁴ représente l'hydrogène ou un groupe alkyle en C₁ à C₄,
R⁵ est un groupe quinolinyle, isoquinolinyle ou oxodihydroisoquinolinyle facultativement condensé à un groupe hétérocyclique à 5 chaînons ayant deux atomes d'oxygène ou deux atomes d'azote dans le noyau et facultativement substitué par un ou plusieurs substituants choisis parmi un halogène, un groupe cyano, carboxy, hydroxy, alkyle en C₁ à C₄, hydroxy-alkyle en C₁ à C₄, alcoxy en C₁ à C₄-alkyle en C₁ à C₄, alkylthio en C₁ à C₄-alkyle en C₁ à C₄, alcoxy en C₁ à C₄, alkylthio en C₁ à C₄, alcényle en C₂ à C₄, alcynyle en C₂ à C₄, alkyle en C₁ à C₄-carbonyle, un groupe -N(R⁶)R⁷ ou phényle facultativement substitué par un ou plusieurs substituants choisis entre un halogène et un groupe alcoxy en C₁ à C₄, et
R⁶ et R⁷ représentent chacun indépendamment l'hydrogène, ou un groupe alkyle en C₁ à C₄ facultativement substitué par un groupe hydroxy ou alcoxy, ou bien l'un des radicaux R⁶ et R⁷ représente l'hydrogène et l'autre est un groupe alkyle en C₁ à C₄-carbonyle, ou R⁶ et R⁷, conjointement avec l'atome d'azote auquel ils sont fixés, représentent un groupe hétérocyclyle à 5 ou 6 chaînons ayant un ou deux atomes d'azote et, facultativement, un atome d'oxygène dans le noyau.

4. Composé suivant la revendication 2, dans lequel
R¹ représente l'hydrogène ou un groupe alkyle en C₁ à C₄, R² représente l'hydrogène, un groupe alkyle en C₁ à C₈, hydroxy-alkyle en C₁ à C₈ ou alkyle en C₁ à C₄-carbonyloxy-alkyle en C₁ à C₈, alcényle en C₂ à C₄, cycloalkyle en C₃ à C₆-alkyle en C₁ à C₄, hétérocyclyle-alkyle en C₁ à C₄, dans lequel le groupe hétérocyclyle est un groupe hétérocyclyle à 5 chaînons ayant un atome d'azote ou un atome d'oxygène dans le noyau, phényl-alkyle en C₁ à C₄, dans lequel le noyau phényle est facultativement substitué par un ou deux substituants choisis parmi un groupe alcoxy en C₁ à C₄, amino, alkyle en C₁ à C₄-carbonylamino, chlore, brome, alkyle en C₁ à C₄-sulfonylamino ou di(alkyle en C₁ à C₄)aminosulfonylamino, et est facultativement condensé à un noyau hétérocyclique à 5 chaînons ayant deux atomes d'oxygène dans le noyau,
R³ représente l'hydrogène ou un groupe alkyle en C₁ à C₄,
R⁴ représente l'hydrogène ou un groupe alkyle en C₁ à C₄,
R⁵ est un groupe quinolinyle de formule II, un groupe isoquinolinyle de formule III ou un groupe oxodihydroisoquinolinyle de formule IIIA, où R⁸, R⁹, R¹⁰, R¹¹, R¹² et R¹³ sont choisis chacun indépendamment parmi l'hydrogène, un halogène, un groupe cyano, carboxy, hydroxy, alkyle en C₁ à C₄, hydroxy-alkyle en C₁ à C₄, alcoxy en C₁ à C₄-alkyle en C₁ à C₄, alkylthio en C₁ à C₄-alkyle en C₁ à C₄, alcoxy en C₁ à C₄, alkylthio en C₁ à C₄, alcényle en C₂ à C₄, alcynyle en C₂ à C₄, alkyle en C₁ à C₄-carbonyle, un groupe -N(R⁶)R⁷ ou phényle facultativement substitué par un ou deux substituants choisis entre un halogène ou un groupe alcoxy en C₁ à C₄, ou bien R¹¹ et R¹², conjointement avec les atomes de carbone auxquels ils sont fixés, représentent un groupe hétérocyclique à 5 chaînons ayant deux atomes d'oxygène dans le noyau, et
R⁶ et R⁷ représentent chacun indépendamment l'hydrogène ou un groupe alkyle en C₁ à C₄ facultativement substitué par un groupe hydroxy ou alcoxy, ou bien l'un des radicaux R⁶ et R⁷ représente l'hydrogène et l'autre est un groupe alkyle en C₁ à C₄-carbonyle, ou R⁶ et R⁷, conjointement avec l'atome d'azote auquel ils sont fixés, représentent un groupe hétérocyclyle à 6 chaînons ayant un ou deux atomes d'azote, ou un atome d'azote et un atome d'oxygène, dans le noyau.

5. Composé suivant la revendication 4, dans lequel R⁵ est un groupe isoquinolinyle de formule III où R⁸ représente l'hydrogène, un groupe alkyle en C₁ à C₄, un halogène, un groupe cyano, -N(R⁶)R⁷ dans lequel R⁶ et R⁷ représentent chacun indépendamment un groupe alkyle en C₁ à C₄, ou bien R⁶ et R⁷, conjointement avec l'atome d'azote auquel ils sont fixés, représentent un groupe hétérocyclyle à 6 chaînons ayant un ou deux atomes d'azote, ou un atome d'azote et un atome d'oxygène, dans le noyau, ou un groupe phényle substitué par un ou deux groupes alcoxy en C₁ à C₄ ; R⁹ et R¹⁰ représentent chacun indépendamment l'hydrogène, un groupe alkyle en C₁ à C₄ ou un halogène ; R¹¹ et R¹² représentent chacun indépendamment l'hydrogène, un halogène, un groupe cyano, carboxy, hydroxy, alkyle en C₁ à C₄, alcoxy en C₁ à C₄ ou alcynyle en C₂ à C₄, ou bien R¹¹ et R¹², conjointement avec les atomes de carbone auxquels ils sont fixés, représentent un hétérocycle à 5 chaînons ayant deux atomes d'oxygène dans le noyau ; et R¹³ représente l'hydrogène ou un halogène.

6. Composé de formule XXXXVI : 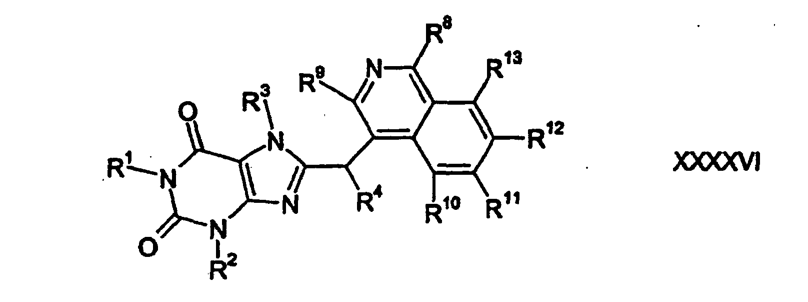 sous forme libre ou sous forme d'un sel, où
(i) R¹ est CH₃, R² est (CH₃)₂CHCH₂, R³ et R⁴ sont chacun H, R⁸ est CH₃, R⁹ et R¹⁰ sont chacun H et R¹¹ et R¹² sont chacun un groupe OCH₃ ; ou
(ii) R¹ est CH₃, R² est (CH₃)₂CHCH₂, R³, R⁴, R⁸, R⁹ et R¹⁰ sont chacun H, et R¹¹ et R¹² sont chacun OCH₃ ; ou
(iii) R¹ est CH₃, R² est (CH₃)₃CCH₂, R³, R⁴, R⁸, R⁹ et R¹⁰ sont chacun H, et R¹¹ et R¹² sont chacun OCH₃ ; ou
(iv) R¹ est CH₃, R² est (CH₃)₂CHCH₂, R³, R⁴, R⁹ et R¹⁰ sont chacun H, R⁸ est Cl et R¹¹ et R¹² sont chacun OCH₃ ; ou
(v) R' est CH₃, R² est (CH₃)₂CHCH₂, R³, R⁴, R⁸, R⁹ et R¹⁰ sont chacun H, R¹¹ est OCH₃, et R¹² est H; ou
(vi) R¹ est CH₃, R² est un groupe cyclopropylméthyle, R³, R⁴, R⁸, R⁹, R¹⁰ et R¹² sont chacun H, et R¹¹ est OCH₃ ; ou
(vii) R¹ est CH₃, R² est (CH₃)₂CHCH₂, R³, R⁴, R⁸, R⁹, R¹⁰ et R¹² sont chacun H, et R¹¹ est CH≡C ; ou
(viii) R¹ est CH₃, R² est un groupe 4-(N-diméthylaminosulfonylamino)benzyle, R³, R⁴, R⁸, R⁹ et R¹⁰ sont chacun H, et R¹¹ et R¹² sont chacun OCH₃ ; ou
(ix) R¹ est CH₃, R² est HOCH₂CH(CH₃)CH₂, R³, R⁴, R⁸, R⁹ et R¹⁰ sont chacun H, et R¹¹ et R¹² sont chacun OCH₃ ; ou
(x) R¹ est CH₃, R² est un groupe 1-méthylcyclopropylméthyle, R³, R⁴, R⁸, R⁹ et R¹⁰ sont chacun H, et R¹¹ et R¹² sont chacun OCH₃.

7. Composé suivant l'une quelconque des revendications 1 à 6 pour une utilisation comme médicament.

8. Composition pharmaceutique comprenant, comme ingrédient actif, un composé suivant l'une quelconque des revendications 1 à 6, facultativement conjointement avec un diluant ou support pharmaceutiquement acceptable.

9. Utilisation d'un composé suivant l'une quelconque des revendications 1 à 6 pour la fabrication d'un médicament pour le traitement d'une affection à médiation par PDE5.

10. Utilisation d'un composé suivant l'une quelconque des revendications 1 à 6 pour la fabrication d'un médicament pour le traitement d'un dysfonctionnement sexuel, en particulier un dysfonctionnement de l'érection chez l'homme.

11. Procédé de préparation d'un composé de formule I, sous forme libre ou sous forme d'un sel, qui comprend :
1)
(a) la déshydratation d'un composé de formule : 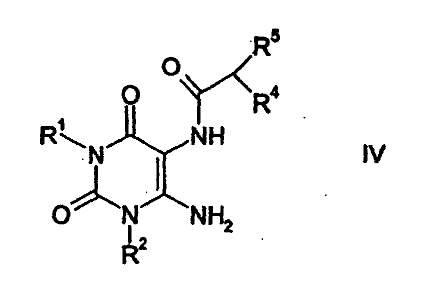 dans laquelle R¹, R², R⁴ et R⁵ sont tels que définis dans la revendication 1 ; ou
(b) pour la préparation d'un composé de formule I sous forme libre ou sous forme d'un sel, dans lequel R³ est un groupe alkyle facultativement substitué par un groupe hydroxy, alcoxy ou alkylthio, la réaction d'un composé de formule I, sous forme libre ou sous forme d'un sel, avec un agent d'alkylation approprié ; ou
(c) pour la préparation d'un composé de formule I, sous forme libre ou sous forme d'un sel, dans lequel R² est un groupe aralkyle substitué dans le noyau aryle par un groupe alkylsulfonylamino ou dialkylaminosulfonylamino, la réaction d'un composé de formule I, sous forme d'un sel, dans lequel R² est un groupe aralkyle substitué par un groupe amino avec un halogénure d'alkylsulfonyle ou un halogénure de dialkyloaminosulfonyle, respectivement ; ou
(d) pour la préparation d'un composé de formule I, sous forme libre ou sous forme d'un sel, dans lequel R² est un groupe alkyle à substitution hydroxy, l'hydratation d'un composé de formule I dans lequel R² est un groupe alcényle ; ou
(e) pour la préparation d'un composé de formule I, sous forme libre ou sous forme d'un sel, dans lequel R² est un groupe alkyle substitué par un groupe alkylcarbonyloxy, l'estérification appropriée d'un composé de formule I dans lequel R² est un groupe alkyle à substitution hydroxy ; ou
(f) pour la préparation d'un composé de formule I, sous forme libre ou sous forme d'un sel, dans lequel R² est un groupe aralkyle substitué dans le noyau aryle par un groupe amino, l'hydrolyse d'un composé de formule I dans lequel R² est un groupe aralkyle substitué dans le noyau aryle par un groupe acylamino ; ou
(g) pour la préparation d'un composé de formule I, sous forme libre ou sous forme d'un sel, dans lequel R⁵ est un groupe quinolinyle ou isoquinolinyle substitué par un groupe hydroxy, la désalkylation d'un composé de formule I dans lequel R⁵ représente, respectivement, un groupe quinolinyle ou isoquinolinyle substitué par un groupe alcoxy ; ou
(h) pour la préparation d'un composé de formule I, sous forme libre ou sous forme d'un sel, dans lequel R⁵ est un groupe quinolinyle ou isoquinolinyle substitué par un halogène, l'halogénation d'un composé de formule I dans lequel R⁵ représente, respectivement, un groupe quinolinyle ou isoquinolinyle ayant un atome de carbone de noyau non substitué disponible pour l'halogénation ; ou
(i) pour la préparation d'un composé de formule I, sous forme libre ou sous forme d'un sel, dans lequel R² est un groupe cyclopropyle, facultativement substitué par un groupe alkyle, l'action de soumettre un composé de formule I, dans lequel R² est un groupe alcényle, à une réaction de cyclopropanation de Simmons Smith ; et
2) la récupération du produit résultant de formule I sous forme libre ou sous forme d'un sel.

12. Composé de formule IV : 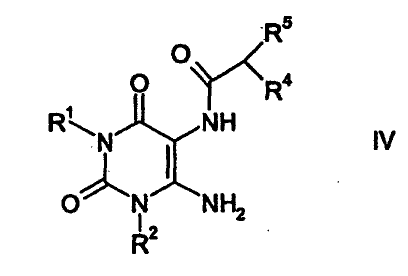 dans laquelle R¹, R², R⁴ et R⁵ sont tels que définis dans la revendication 1.
